# EUROPÄISCHE PATENTSCHRIFT

(11) **EP 1 102 761 B1**
(45) Veröffentlichungstag und Bekanntmachung des Hinweises auf die Patenterteilung: **29.01.2003**
(21) Anmeldenummer: 99939412.5
(22) Anmeldetag: 29.07.1999
(51) Int. Cl.: C07D 307/93, A61K 31/34, A61P 29/00

(54) **CYCLOPENTABENZOFURAN-DERIVATE UND IHRE VERWENDUNG**
CYCLOPENTABENZOFURAN DERIVATIVES AND THEIR USE
DERIVES DE CYCLOPENTABENZOFURANNE ET LEUR UTILISATION

(30) Priorität: 05.08.1998 DE 19835324
(43) Veröffentlichungstag der Anmeldung: 30.05.2001
(73) Patentinhaber: Bayer Aktiengesellschaft, 51368 Leverkusen (DE)
(72) Erfinder: GUARNIERI, Walter, D-51373 Leverkusen (DE); JAETSCH, Thomas, D-50668 Köln (DE); SCHOOP, Andreas, D-51491 Overath (DE); BAUMGARTEN, Jörg, D-42115 Wuppertal (DE); KRETSCHMER, Axel, D-42113 Wuppertal (DE); ANTONICEK, Horst-Peter, D-51467 Bergisch Gladbach (DE)
(86) Internationale Anmeldenummer: EP9905427
(87) Internationale Veröffentlichungsnummer: WO00008007

(56) Entgegenhaltungen:
- EP-A- 0 709 382
- WO-A-97/08161
- A.E. DAVEY ET AL.: "Synthesis of the Novel Anti-Leukaemic Tetrahydrocyclopenta[b]benzofuran, Rocaglamide and Related Synthetic Studies" JOURNAL OF THE CHEMICAL SOCIETY, PERKIN TRANSACTIONS 1,1992, Seiten 2657-2666, XP002129822 LETCHWORTH GB in der Anmeldung erwähnt
- A.E. DAVEY ET AL.: "Synthesis of the Novel Anti-leukaemic Tetrahydrocyclopenta[b]benzofuran, Rocaglamide" JOURNAL OF THE CHEMICAL SOCIETY, CHEMICAL COMMUNICATIONS,1991, Seiten 1137-1139, XP002129823 LETCHWORTH GB in der Anmeldung erwähnt
- DUMONTET V ET AL: "New Nitrogenous and Aromatic Derivatives from Aglaia argentea and A. forbesii" TETRAHEDRON,NL,ELSEVIER SCIENCE PUBLISHERS, AMSTERDAM, Bd. 52, Nr. 20, 13. Mai 1996 (1996-05-13), Seiten 6931-6942, XP004103936 ISSN: 0040-4020 in der Anmeldung erwähnt

## Beschreibung

Die Erfindung betrifft Cyclopentabenzofuran-Derivate, Verfahren zu ihrer Herstellung, die Verwendung von Cyclopentabenzofuran-Derivaten zur Herstellung eines Arzneimittels zur Therapie von NF-κB abhängigen Krankheiten und Arzneimittel, die die Cyclopentabenzofuran-Derivate enthalten.

Extrakte aus der Pflanze *Aglaia elliptifolia* zeigen antileukämische Eigenschaften. Als erste wirksame Verbindung wurde ein Rocaglamid genanntes Dihydrocyclopentabenzofuranol-Derivat identifiziert (J. Chem. Soc., Chem. Commun. 1982, 1150; US 4539414). Daraufhin erschienen mehrere Arbeiten über schließlich auch erfolgreiche Syntheseversuche. (J. Chem. Soc., Chem. Commun. 1991, 1137). Erst 10 Jahre nach der Isolierung von Rocaglamid wurden seine insektiziden Eigenschaften beschrieben (Pestic. Sci 36, 53 (1992) und Phytochemistry 32, 67 (1993)) und darauf in einer anderen Art, *Aglaia odorata,* noch drei, sich nur in einem Substituenten unterscheidende, Derivate gefunden (Phytochemistry 32, 307 (1993)). Später wurden z.B. aus der Art *Aglaia roxburghiana* erst vier anellierte Derivate des Rocaglamids isoliert (WO 96/04284), dann zahlreiche weitere neue Derivate sowie deren pharmakologische Eigenschaften beschrieben (vgl. z.B. J. Nat. Prod. 59, 650 (1996); Tetrahedron 52, 6931 (1996); Phytochemistry 44, 1455 (1997); Phytochemistry 45 1579 (1997); Z. Naturforsch., C: Biosci. 52, Tetrahedron 53, 17625 (1997); B.W. Nugroho, Dissertation, Bayer. Julius-Maximilian Univ. Würzburg, 1997); WO 97/08161 A1).

Ein bedeutender Schritt bei vielen endzündlichen Prozessen ist die Translocation des Proteins "Nuclear Factor kappa B", kurz NF-κB, in den Zellkern und die hierdurch verursachte Stimulierung der Expression der Gene, deren Produkt für enzündliche Reaktionen verantwortlich sind (Trends Pharmacol. Sci. 18, 46 (1997)). Beispielsweise bei Asthma ist die nicht nützliche, übermäßige (nicht selbstbegrenzende) Produktion dieser Proteine für die Verstärkung und Aufrechterhaltung des entzündlichen Prozesses und die damit verbundenen unangenehmen bis lebensbedrohlichen Symptome dieser Krankheit verantwortlich. Weil die dem heutigen Stand der Technik entsprechende Langzeitbehandlung mit Glucocorticoiden mit einigen Nachteilen behaftet ist, wird NF-κB als ein zwingendes Target gesehen für die Entwicklung von neuen entzündungshemmenden Wirkstoffen gegen Asthma.

Es wurde nun gefunden, daß die Cyclopentabenzofuran-Derivate der Formel (I) 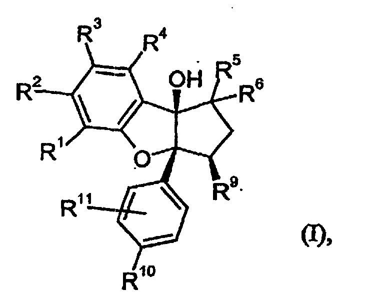 in welcher
- R¹ und R³: jeweils für Wasserstoff, Fluor, Chlor, Brom, Methyl oder Ethyl stehen,
- R² und R⁴: jeweils für Methoxy, Ethoxy, Trifluormethoxy, Difluormethoxy, Chlordifluormethoxy, 2-Chlor-1,1,2 trißuorethoxy, 1,1,2-Trifluorethoxy, 1,1,2,2-Tetrafluorethoxy, 2,2,2-Trichlor-1,1-difluormethoxy oder 1,1-Difluorethoxy stehen,
- R⁵: für Hydroxy, Methylamino, Ethylamino, n-Propylamino, Isopropylamino, n-Butylamino, Isobutylamio, sec.-Butylamino, tert.-Butylamino oder für den Rest-NR¹²-CHR¹³-COOR¹⁴ steht,
- R⁶: für Wasserstoff steht, oder
- R⁵ und R⁶: gemeinsam für Sauerstoff (Oxo) stehen,
- R⁹: für gegebenenfalls einfach oder zweifach durch Fluor, Chlor, Brom, Iod, Methyl, Ethyl, n-Propyl, Isopropyl, n-Butyl, Isobutyl, sec.-Butyl, tert.-Butyl, Trifluormethyl, Difluormethyl, Chlordifluormethyl, 2-Chlor-1,1,2-trifluorethyl, 1,1,2-Trifluorethyl, 1,1,2,2-Tetrafluorethyl, 1,1-Difluor-2,2,2-trichlorethyl, 2,2,2-Trifluorethyl, 1,1,2,3,3,3-Hexafluorpropyl, 2,2,3,3-Tetrafluorpropyl, 2,2,3,3,3-Pentafluorpropyl, Hydroxy, Methoxy, Ethoxy, n-Propoxy, Isopropoxy, n-Butoxy, Isobutoxy, sec.-Butoxy, tert.-Butoxy, Trifluormethoxy, Difluormethoxy, Chlordifluormethoxy, 2-Chlor-1,1,2-trifluorethoxy, 1,1,2-Trifluorethoxy, 1,1,2,2-Tetrafluorethoxy, 2,2,2-Trichlor-1,1-difluormethoxy, 1,1-Difluorethoxy, Methylthio, Ethylthio, Trifluormethylthio, 1,1-Difluorethylthio, 2,2,2-Trifluorethylthio, Phenyl, Phenoxy, Furyl, Thienyl, Pyrrolyl, Thiazolyl, Pyridyl, Furyloxy, Thienyloxy, Pyrrolyloxy, Thiazolyloxy, Pyridyloxy, Acetyl, Propionyl, Propylcarbonyl, Butylcarbonyl oder 2-Methylpropylcarbonyl substituiertes Phenyl, Methylendioxyphenyl, Furyl, Thienyl, Pyrrolyl, Oxazolyl, Isoxazolyl, Thiazolyl oder Pyridyl steht,
- R¹⁰: für Wasserstoff, Fluor, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy oder tert.-Butoxy steht,
- R¹¹: für Wasserstoff, Fluor, Chlor, Brom, Methyl, Ethyl, n-Propyl, Isopropyl, n-Butyl, Isobutyl, sec.-Butyl oder tert.-Butyl steht,
- R¹²: für Wasserstoff steht,
- R¹³: für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl, 3-Indolylmethyl oder 4-Imidazolylmethyl steht, und
- R¹⁴: für Methyl, Ethyl, n-Propyl, Isopropyl, n-Butyl, Isobutyl, sec.-Butyl, tert.-Butyl oder Benzyl steht, geeignet sidn zur Herstellung eines Arzneimittels zur Behandlung von Entzündungskrankheiten, immunologischen Erkrankungen, septischem Schock, Transplantatabstoßung, Strahlungsschäden, Reperfusionsverletzungen nach Ischämie, Schlaganfall und Hirntrauma, neurodegenerativen Erkrankungen, Leberzirrhose, Asthma oder bei komplexen, chronisch-entzündlichen Erkrankungen wie Arteriosklerose und Multiple sklerose.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfach heit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ist in einer Strukturformel die Zahl der Diastereomeren durch Festlegung der Konfiguration an mindestens zwei Chiralitätszentren eingeschränkt, so sind, wenn nicht anders vermerkt, grundsätzlich auch die anderen Enantiomere (Spiegelbilder) gemeint.

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Hetaryl steht im Rahmen der Erfindung im allgemeinen für einen aromatischen gegebenenfalls benzokondensierten 5- bis 7-gliedrigen, vorzugsweise 5- bis 6-gliedrigen Heterocyclus, der bis zu 3 Heteroatome aus der Reihe S, N und/oder O enthalten kann. Beispielsweise seien genannt: Indolyl, Isochinolyl, Chinolyl, Benzo[b]thiophen, Benzo[b]furanyl, Pyridyl, Thienyl, Furyl, Pyrrolyl, Thiazolyl, Oxazolyl, Isoxazolyl, Imidazolyl, Morpholinyl oder Piperidyl. Bevorzugt sind Furyl, Pyrrolyl, Oxazolyl, Isoxazolyl, Thiazolyl, Pyridyl und Thienyl.

Die aus der Peptidchemie bekannten Schutzgruppen sind z.B. aufgeführt in T. W. Greene, P. G. M. Wuts, Protective Groups in Organic Synthesis, 2^{nd} Ed., John Wiley & Sons, New York 1991. Beispiele für geeignete Schutzgruppen sind C₁-C₄-Alkyl und Benzyl insbesondere für Hydroxy- bzw. Carboxygruppen (C-terminale Schutzgruppen); Acetyl, trifluoracetyl, Trichloracetyl, Benzoyl, Phenylacetyl, tert.-Butoxycarbonyl (Boc), Benzyloxycarbonyl, (Cbz), (9-Fluorenyl)methoxycarbonyl (Fmoc) und Benzyl insbesondere für Hydroxy- und Aminogruppen (N-terminale Schutzgruppen).

Davon besonders bevorzugt sind tert.-Butyl, Benzyl, Acetyl, Boc und Cbz.

Die o.a. erfindungsgemäß verwendbaren Stoffe der Formel (I) sind neu mit Ausnahme von 3,3a-Dihydro-6,8-dimethoxy-3a-(4-methoxyphenyl)-3-phenyl-1Hcyclopenta[b]benzofuran-1,8b(2H)-diol und 2,3,3a,8b-Tetrahydro-8b-hydroxy-6,8-dimethoxy-3a-(4-methoxyphenyl)-3-phenyl-1H-cyclopenta[b]benzofuran-1-on (vgl. J. Chem. Soc., chem. Commun. 1991, 1137; J. Chem. soc. Perk. Trans. 1, 1992, 2657).

Weiterhin wurde gefunden, daß man die neuen Verbindungen der Formel (I) nach einem der im folgenden beschriebenen Verfahren erhält.
A) cis-Dihydrocyclopentabenzofurandiole der Formel (I-a) 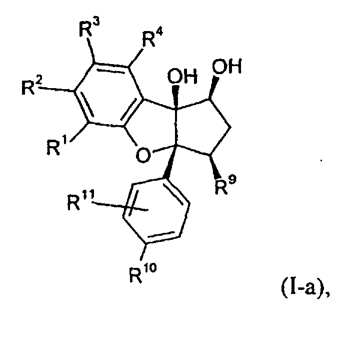 in welcher
   - R¹, R², R³, R⁴, R⁹, R¹⁰ und R¹¹: die oben angegebenen Bedeutungen haben,
   lassen sich herstellen, indem man Ketoaldehyde der Formel (II-a) 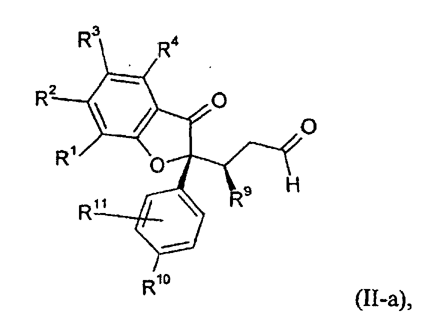 in welcher
   - R¹, R², R³, R⁴, R⁹, R¹⁰ und R¹¹: die oben angegebenen Bedeutungen haben,
   reduktiv cyclisiert.
B) Tetrahydrocyclopentabenzofuranone der Formel (I-b) 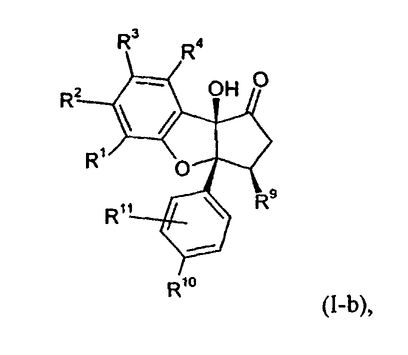 in welcher
   - R¹, R², R³, R⁴, R⁹, R¹⁰ und R¹¹: die oben angegebenen Bedeutungen haben,
   lassen sich herstellen, indem man
   Dihydrocyclopentabenzofurandiole der Formel (I-a) 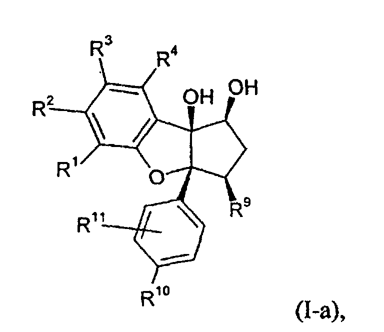 in welcher
   - R¹, R², R³, R⁴, R⁹, R¹⁰ und R¹¹: die oben angegebenen Bedeutungen haben,
   oxidiert.
C) trans-Dihydrocyclopentabenzofurandiole der Formel (I-c) 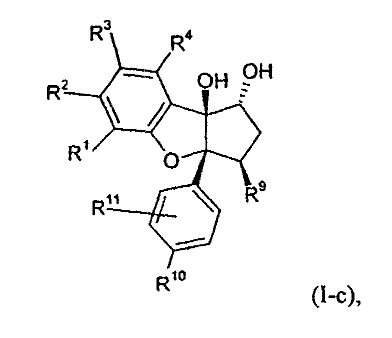 in welcher
   - R¹, R², R³, R⁴, R⁹, R¹⁰ und R¹¹: die oben angegebenen Bedeutungen haben,
   lassen sich herstellen, indem man
   Tetrahydrocyclopentabenzofuranone der Formel (I-b) 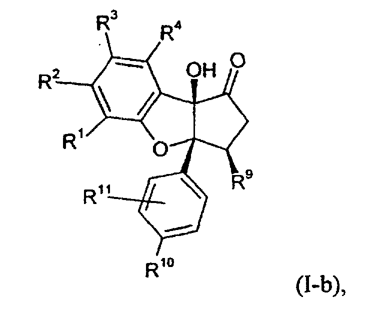 in welcher
   - R¹, R², R³, R⁴, R⁹, R¹⁰ und R¹¹: die oben angegebenen Bedeutungen haben,
   mit Alkali- oder Tetraalkylammonium-acyloxyboranaten reduziert.
D) Cyclopentabenzofuran-Derivate der Formel (I-d) 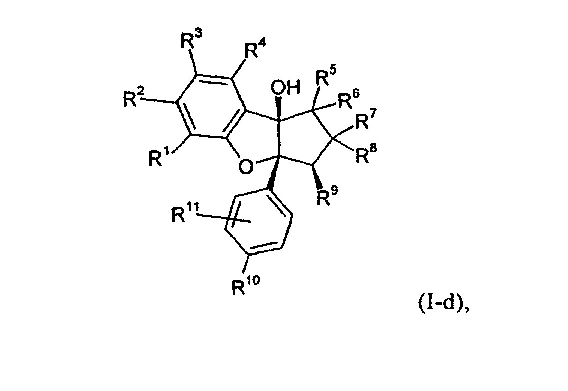 in welcher
   - R¹ bis R¹¹: die oben angegebenen Bedeutungen haben, mit der Einschränkung, daß mindestens einer der Reste R¹, R³ und R" für Halogen oder Alkyl steht,
   lassen sich herstellen, indem man diesen oder diese durch elektrophile aromatische Substitution von Verbindungenen der oben angegebenen Formel (I), in welchen der oder die zu substituierenden Reste für Wasserstoff steht, einführt.
E) Cyclopentabenzofuran-Derivate der Formel (I-e) 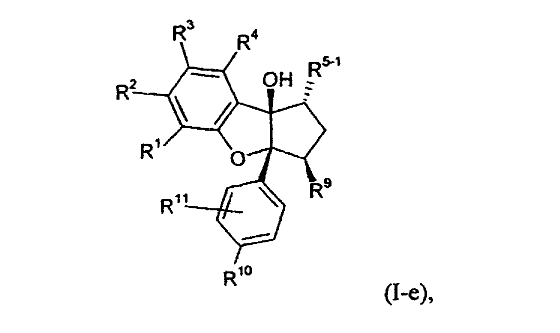 in welcher
   - R¹ bis R⁴ und R⁹ bis R¹¹: die oben angegebenen Bedeutungen haben und
   - R⁵⁻¹: für Alkylamino oder für den Rest -NR¹²-CHR¹³-COOR¹⁴ steht, worin
   R¹², R¹³ und R¹⁴ die oben angegebenen Bedeutungen haben,
   lassen sich herstellen, indem man
   Tetrahydrocyclopentabenzofuranone der Formel (I-b) 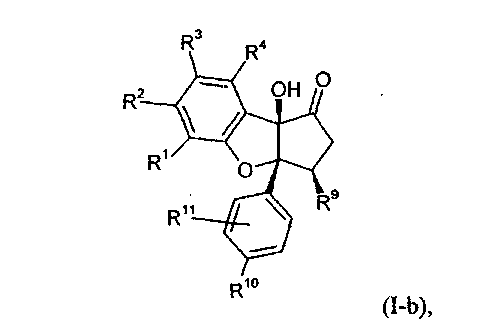 in welcher
   - R¹ bis R⁴ und R⁹ bis R¹⁰: die oben angegebenen Bedeutungen haben,
   mit primären Aminen oder Aminosäurederivaten der Formel (III)

   H-R⁵⁻¹ (III),

   in welcher
   - R⁵⁻¹: für Alkylamino oder für den Rest -NR¹²-CHR¹³-COOR¹⁴ steht, worin
   R¹², R¹³ und R¹⁴ die oben angegebenen Bedeutungen haben,
   in Gegenwart eines Reduktionsmittels umsetzt.

Verwendet man beispielsweise 2,3-Dihydro-4,6-diethoxy-2-(4-methoxyphenyl)-3-oxo-β-phenyl-2-benzofuranpropanal als Ausgangsstoff, so können die hier aufeinander folgenden Reaktionsabläufe der erfindungsgemäßen Verfahren (A), (B) und (C) durch das folgende Formelschema wiedergegeben werden:

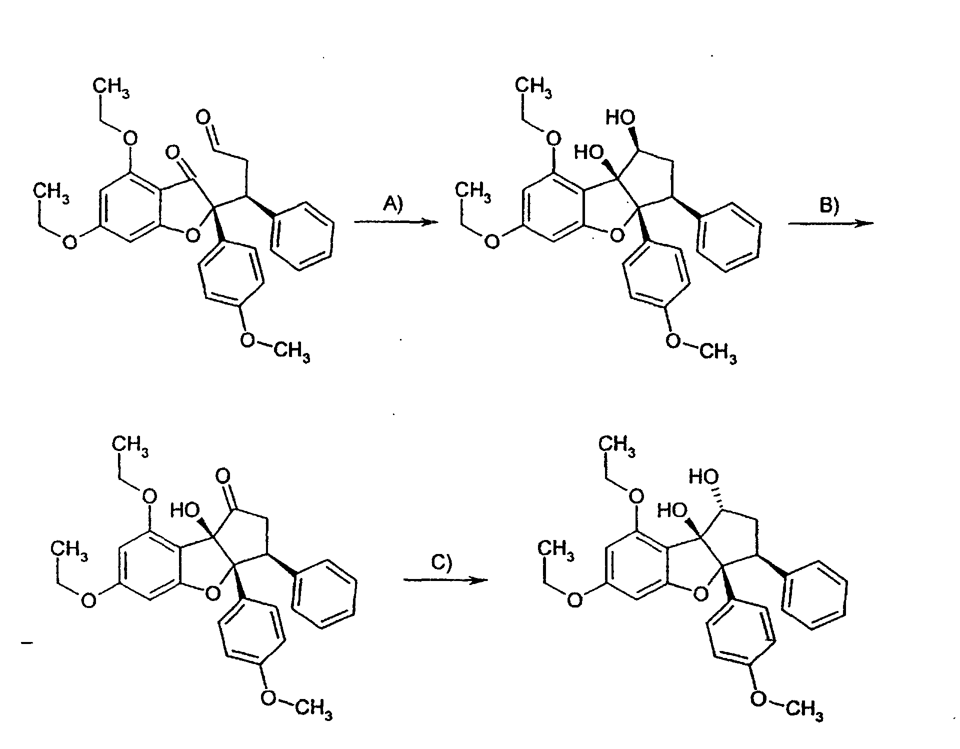

Verwendet man beispielsweise 3,3a-Dihydro-6,8-dimethoxy-3a-(4-methoxyphenyl)-3-phenyl-1H-cyclopenta[b]benzofuran-1,8b(2H)-diol als Ausgangsstoff und Brom-Pyridin-Komplex als Reagenz so kann der Reaktionsablauf des erfindungsgemäßen Verfahrens (D) durch das folgende Formelschema wiedergegeben werden:

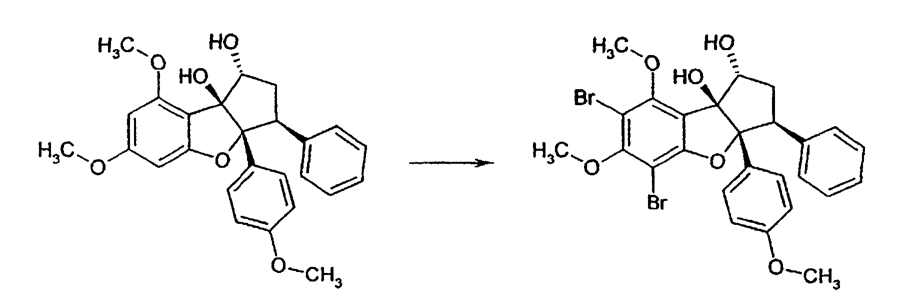

Verwendet man beispielsweise 2,3,3a,8b-Tetrahydro-8b-hydroxy-6,8-dimethoxy-3a-(4-methoxyphenyl)-3-phenyl-1H-cyclopenta[b]benzofuran-1-on und Glycinmethylester-hydrochlorid als Ausgangsstoffe und Tetramethylammoniumtrisacetoxyborhydrid in Gegenwart von Molekularsieb als Reagenz so kann der Reaktionsablauf des erfindungsgemäßen Verfahrens (E) durch das folgende Formelschema wiedergegeben werden:

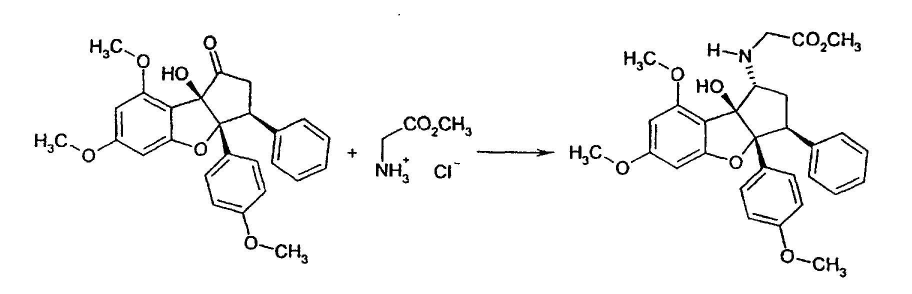

Die zur Durchführung des erfindungsgemäßen Verfahrens (A) benötigten Ketoaldehyde sind durch die Formel (II-a) allgemein definiert. In dieser Formel haben R¹ bis R¹¹ vorzugsweise diejenigen Bedeutungen, die bereits im Zusammenhang mit der Beschreibung der Cyclopentabenzofuran-Derivate der Formel (I) als bevorzugt genannt wurden. Die Ketoaldehyde der Formel (II-a) und der weiter unten angegebenen anderen Diastereomeren der Formel (II-b) sind neu mit Ausnahme von 2,3-Dihydro-4,6-dimethoxy-2-(4-methoxyphenyl)-3-oxo-β-phenyl-2-benzofuranpropanal.

Ketoaldehyde der Formeln (II-a) und (II-b) lassen sich z.B. herstellen, indem man Benzofuranone der Formel (IV) an Zimtaldehyde bzw. deren heterocyclischen Analoga der Formel (V) addiert in Gegenwart eines Säurebindemittels, wie beispielsweise Benzyltrimethylammoniumhydroxid-Lösung oder Natriummethylat und in Gegenwart eines Verdünnungsmittels, wie beispielsweise Methanol oder tert.-Butanol gemäß folgendem Reaktionsschema:

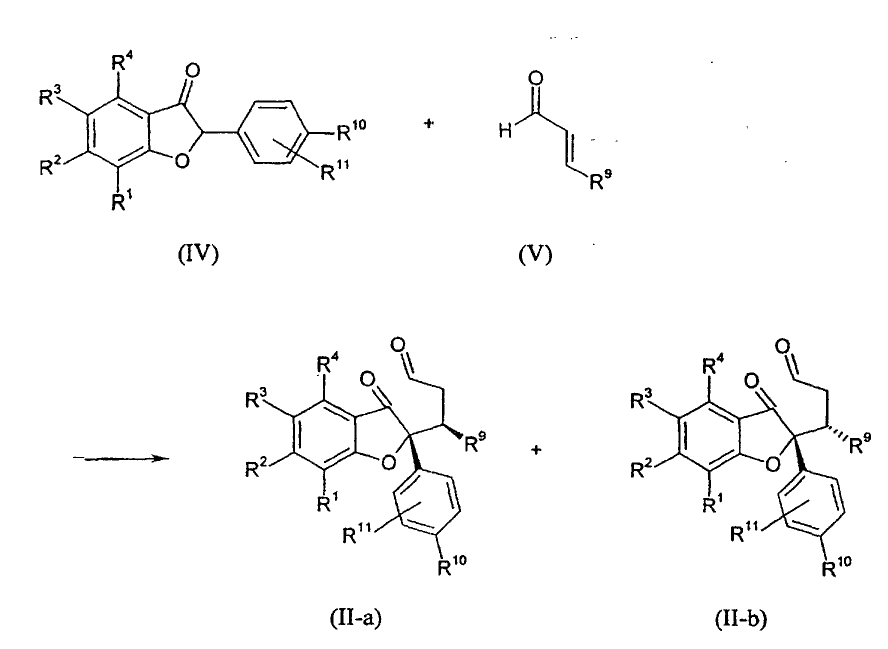

Die Diastereomeren (II-a) und (II-b) lassen sich z.B. nach üblichen Methoden säulenchromatographisch trennen.

Die vinylogen Arylaldehyde der Formel (V) sind z.T. kommerziell erhältlich, bekannt oder lassen sich nach bekannten Methoden herstellen.

Benzofuranone der Formel (IV) lassen sich z.B. durch Hoesch-Reaktion aus Cyanhydrinen (vgl. Chem. Soc. Perkin Trans. I, 1992, 2657) oder bevorzugt Trimethylsilylcyanhydrinen (vgl. Herstellbeispiele) der Formel (VI) 4-substituierter Benzaldehyde, in welcher R¹⁵ für Wasserstoff oder Trimethylsilyl (TMS) steht, mit Phenolen der Formel (VII) gemäß dem folgenden Reaktionsschema herstellen:

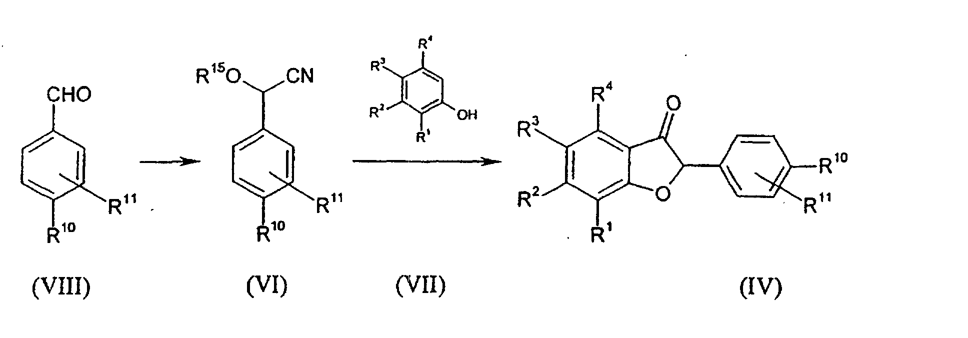

Die hierfür benötigten Benzaldehyde der Formel (VIII) und Phenole der Formel (VII) sind allgemein bekannte Verbindungen der organischen Chemie. Die Umsetzung der Benzaldehyde zu den Cyanhydrinen der Formel (VI) erfolgt z.B. mit Natriumcyanid bzw. Trimethylsilylcyanid nach bekannten Methoden.

Ein Beispiel zur Derivatisierung der Reste R² und R⁴ auf der Stufe des Benzofuranons der Formel (IV) ist die Alkylierung (z.B. mit Diethylsulfat/Kaliumcarbonat) mit anschließender Spaltung des entstehenden Enolethers (z.B. mit Salzsäure) gemäß dem folgendem Reaktionsschema:

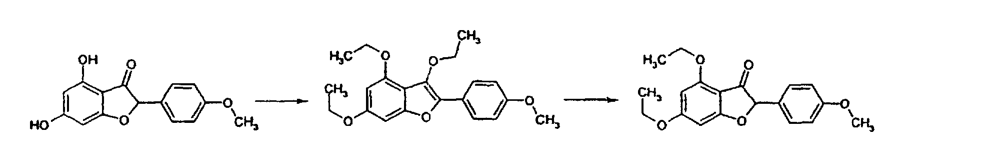

Die zur Durchführung des erfindungsgemäßen Verfahrens (B) benötigten Dihydrocyclopentabenzofurandiole der Formel (I-a) sind Teilmengen der erfindungsgemäßen Verbindungen der allgemeinen Formel (I) und lassen sich beispielsweise nach den Verfahren (A) oder (D) herstellen.

Die zur Durchführung der erfindungsgemäßen Verfahren (C) und (E) benötigten Tetrahydrocyclopentabenzofuranone der Formel (I-b) sind Teilmengen der erfindungsgemäßen Verbindungen der allgemeinen Formel (I) und lassen sich beispielsweise nach dem Verfahren (B) oder (D) herstellen.

Die zur Durchführung des erfindungsgemäßen Verfahrens (D) benötigten Cyclopentabenzofurane sind Teilmengen der erfindungsgemäßen Verbindungen der allgemeinen Formel (I) und lassen sich beispielsweise nach dem Verfahren (A), (C) oder (E) herstellen.

Die zur Durchführung des erfindungsgemäßen Verfahrens (E) benötigten Amine oder Aminosäurederivate sind durch Formel (III) allgemein definiert. In dieser Formel hat R⁵⁻¹, soweit zutreffend, sowie R¹² bis R¹⁴ vorzugsweise diejenigen Bedeutungen, die bereits im Zusammenhang mit der Beschreibung der Cyclopentabenzofuran-Derivate der Formel (I) für R⁵ sowie R¹² bis R¹⁴ als bevorzugt genannt wurden. Die Verbindungen der Formel (III) sind überwiegend kommerziell erhältlich oder lassen sich nach bekannten Methoden der Aminosäurechemie herstellen.

Das erfindungsgemäße Verfahren (A) wird in Gegenwart eines Reduktionsmittel durchgeführt. Hierzu wird vorzugsweise Samariumdiiodid eingesetzt. Samariumdiiodid kann als Lösung (0,1 M) in THF eingesetzt werden oder durch Reaktion von Samarium mit 1,2-Diiodethan in Lösung hergestellt werden.

Das erfindungsgemäße Verfahren (A) wird vorzugsweise in Gegenwart eines Verdünnungsmittel durchgeführt. Als solches kommen organische Lösungsmittel wie beispielsweise aromatische Kohlenwasserstoffe wie Benzol oder Toluol oder Ether wie Tetrahydrofuran oder Dioxan in Betracht.

Das erfindungsgemäße Verfahren (B) wird als Swern- oder Parikh-Doering-Oxidation durchgeführt. Als Reagenzien setzt man beispielsweise Dimethylsulfoxid und Oxalylchlorid/Triethylamin oder Schwefeltrioxid-Pyridin-Komplex und Triethylamin ein.

Das erfindungsgemäße Verfahren (B) wird vorzugsweise in Gegenwart eines Verdünnungsmittel durchgeführt. Als solches kommen organische Lösungsmittel wie beispielsweise Ether wie Diethylether, Tetrahydrofuran oder Dioxan oder Sulfoxide wie Dimethylsulfoxid in Betracht.

Die zur Durchführung des erfindungsgemäßen Verfahrens (C) benötigten Alkalioder Tetraalkylammonium-acyloxyboranate sind beispielsweise Lithium-, Natrium-, Kalium- oder C₁-C₄-Tetraalkylammoniumsalze von Tris-C₁-C₅-(halogen)alkylcarbonyloxyboranaten wie Natriumtrisacetoxyborhydrid oder Tetramethylammoniumtrisacetoxyborhydrid. Man kann das Reduktionsmittel auch in situ herstellen, indem man beispielsweise Lithium- oder Natriumborhydrid und die dem gewünschten Acylrest entsprechende Carbonsäure, wie z.B. Essigsäure, Trifluoressigsäure oder Propionsäure einsetzt.

Das Verfahren (C) wird vorzugsweise in Gegenwart eines Verdünnungsmittels durchgeführt. Hierzu kommen organische Lösungsmittel in Betracht. Beispielhaft seien genannt: Nitrile, wie Acetonitril, Propionitril, n- oder i-Butyronitril oder Benzonitril; Carbonsäuren wie Essigsäure oder Propionsäure.

Das erfindungsgemäße Verfahren (D) umfaßt Halogenierungen und Friedel-Crafts-Alkylierungen. Als Reagenzien sind beispielsweise geeignet: Chlor, Brom, Brom-Pyridin-Komplex, *N*-Bromsuccinimid, *I,I-*Bis*(trifluoracetoxy)-iodbenzol,* Alkylchloride und Alkylbromide.

Das Verfahren (D) wird vorzugsweise in Gegenwart eines Verdünnungsmittels durchgeführt. Hierzu kommen organische Lösungsmittel in Betracht. Beispielhaft seien genannt: aliphatische oder alicyclische Kohlenwasserstoffe, wie beispielsweise Petrolether, Hexan, Heptan, Cyclohexan, Methylcyclohexan oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Nitrile, wie Acetonitril, Propionitril, n- oder i-Butyronitril oder Benzonitril.

Das Verfahren (D) wird gegebenenfalls in Gegenwart einer Lewis-Säure als Katalysator durchgeführt. Beispielsweise seien genannt: Eisen (bei Bromierungen) Eisenchloride und -bromide, Aluminiumchlorid und -bromid, Zinkchlorid oder Bortrifluorid.

Die Umsetzungen der erfindungsgemäßen Verfahren können bei Normaldruck oder unter erhöhtem Druck durchgeführt werden. Vorzugsweise wird bei Normaldruck gearbeitet. Die Reaktionsdurchführung, Aufarbeitung und Isolierung der Reaktionsprodukte erfolgt nach allgemein üblichen, bekannten Methoden. Die Endprodukte werden vorzugsweise durch Kristallisation, chromatographische Trennung oder durch Entfernung der flüchtigen Bestandteile, gegebenenfalls im Vakuum, gereinigt (vergl. auch die Herstellungsbeispiele).

Die erfindungsgemäß verwendbaren Stoffe sind niedermolekulare Inhibitoren, die selektiv Nuclear Factor kappa B (NF-κB)-vermittelte pathophysiologische Prozesse hemmen. NF-κB-vermittelte Prozesse treten bei Entzündungskrankheiten, immunologischen Erkrankungen, septischem Schock, Transplantatabstoßung, Strahlungsschäden, Reperfusionsverletzungen nach Ischämie, Schlaganfall und Hirntrauma, Thrombosen, Leberzirrhose, Asthma oder bei komplexen, chronisch-entzündlichen Erkrankungen wie Arteriosklerose und Mutible sklerose auf.

### Zur pharmakologischen Wirkung von Inhibitoren des Nuclear Factor kappa B

Nuclear Factor kappa B (NF-κB) ist ein in vielen Gewebezellen und insbesondere in Blutzellen vorkommender dimerer Proteinkomplex. NF-κB nimmt eine besondere Rolle zur Steuerung der Expression von Genen ein, die in ihrer Promotorsequenz eine NF-κB Bindesequenz aufweisen (5'-GGGPuNNPyPyCC-3'). Insofern ist NF-κB ein Transkriptionsfaktor. Die physiologische Aktivität von NF-κB zur Kontrolle der Genexpression unterliegt jedoch einem Regulationsprinzip, bei dem NF-κB aus einem Komplex mit dem Protein IκB freigesetzt wird, um als Transkriptionsfaktor in den Zellkern zur Genaktivierung zu translozieren. Das Regulationsprinzip zur Freisetzung von aktivem NF-κB aus einem Komplex mit dem Protein IκB ist noch nicht in Einzelheiten bekannt.

Ebenso ist nicht bekannt, wie die Bildung von homodimeren und heterodimeren NF-κB Proteinkomplexen erfolgt. NF-κB wirkt als dimerer Transkriptionsfaktor auf die Genaktivierung ein. Die Dimerisierung kann unter den strukturell verwandten Transkriptionsfaktoren Rel A, Rel B, c-Rel, p50 oder p52 erfolgen, die eine Familie von Transkriptionsfaktorproteinen bilden. Auch kann in der Dimerisierung der Untereinheiten zum NF-κB bereits ein Regulationsprinzip zur Steuerung der später näher beschriebenen Gene liegen, das noch nicht bekannt ist.

Ein entscheidendes Merkmal von NF-κB gegenüber anderen Transkriptionsfaktoren ist, daß NF-κB ein primärer Transkriptionsfaktor ist. Primäre Transkriptionsfaktoren sind in inaktiver (meist komplexgebundener) Form bereits in der Zelle vorhanden und werden nach einem entsprechenden Stimulus freigesetzt, um ihre Wirkung sehr schnell entfalten zu können. Primäre Transkriptionsfaktoren werden nicht erst durch die Aktivierung des zugehörigen Gens und anschließende Transkription und Translation gebildet.

Diese Eigenschaft des NF-κB, die Ausbildung homodimerer oder heterodimerer Rel-Proteine sowie die Ausbildung eines inaktiven Proteinkomplexes mit einem IκB Protein, bieten ganz andere Angriffspunkte für pharmakologisch wirksame Substanzen, als die Angriffspunkte der de novo Biosynthese von Transkriptionsfaktoren. Der Vollständigkeit halber sei erwähnt, daß die Gene zur Bildung von NF-κB (Gene der Rel Familie) und die Gene zur Bildung der IκB Proteine (Genfamilie umfassend die Gene für IκB-α, IκB-β, p105/IκB-γ, p100/IκB-δ, IκB-ε u.a.) ihrerseits natürlich auch einer Regulation unterliegen, die Angriffspunkte für pharmazeutisch wirksame Substanzen darstellen können. So ist bekannt, daß die Expression der konstitutiv gebildeten IκB Proteine p105 und p100 durch Stimuli erhöht wird, die auch NF-κB aktivieren, wie z.B. Tumor Necrosis Factor-α (TNF-α) oder Phorbolmyristatacetat (PMA).

Ein Regulationsmechanismus ist in der Literatur beschrieben, in dem gezeigt wird, daß die Überexpression von IκB aktives NF-κB bindet und damit inaktiviert. Dies gilt auch, wenn das NF-κB bereits eine Komplexbindung mit der DNA eingegangen ist (P.A. Baeuerle, T. Henkel, Annu. Rev. Immunol. 12, 141-179, 1994). Daraus kann geschlossen werden, daß es mehrere spezifische Angriffspunkte in der biochemischen Funktion von NF-κB und IκB Proteinen gibt, die es ermöglichen sollten, eine ungewünschte, pathophysiologische, NF-κB-abhängige Genaktivierung selektiv zu hemmen.

Eine chemische Verbindung, die selektiv die Funktion von NF-κB hemmt oder die Funktion von IκB Proteinen oder IκB Genen verstärkt, sollte als Pharmazeutikum zur Unterdrückung von NF-κB-vermittelten Krankheitsprozessen Verwendung finden können.

Primär kann NF-κB alle pathophysiologischen Prozesse fordern, an denen Gene beteiligt sind, die in ihrem Promotor die NF-κB Bindesequenz aufweisen. Namentlich sind dies Gene, die bei immunologischen Komplikationen, bei Entzündungskrankheiten, Autoimmunerkrankungen, septischem Schock, Transplantatabstoßung, Thrombosen oder aber auch bei chronisch-entzündlichen Krankheiten wie Arteriosklerose, Arthritis und Rheuma Psoriasis eine entscheidende ursächliche Rolle spielen.

NF-κB Bindesequenzen enthalten z.B. die Promotoren von Rezeptoren lymphoider Zellen (T-Zellrezeptoren), von MHCI- und MHCII-Genen, von Zelladhäsionsmolekülen (ELAM-1, VCAM-1, ICAM-1), von Zytokinen und Wachstumsfaktoren (siehe auch nachfolgende Tabelle). Weiterhin finden sich NF-κB Bindesequenzen in den Promotoren von Akutphasenproteinen (Angiotensinogen, Komplementfaktoren u.a.).

Eine chronisch erhöhte oder akut überschießende Aktivierung der genannten Gene führt zu vielfältigen pathophysiologischen Prozessen und Syndromen.

So ist die schnelle und überschießende Produktion von Zytokinen der Entzündungsreaktion (TNFα, Interleukin-2, Inteerleukin-4, Interleukin-6, Interleukin-8 u.a.) und der Adhäsionsmoleküle (ELAM-1, VCAM-1) in Leukozyten, insbesondere in Makrophagen und auch in Endothelzellen, ein ursächliches Merkmal für oft tödlich verlaufende Prozesse bei septischem Schock; oder führt bei Strahlungsschäden und bei Transplantatabstoßung zu erheblichen Komplikationen. Hemmstoffe, die die NF-KB-vermittelte Genexpression verhindern, greifen bei einigen Krankheiten sehr früh in die Ausprägung pathophysiologischer Veränderungen ein und können daher ein sehr wirkungsvolles therapeutisches Prinzip darstellen. Ein Beispiel sind auch NF-κB Inhibitoren für Krankheiten, die auf eine Überexpression von Akutphasenproteinen zurückzuführen sind. Eine ungewünschte Überexpression von Akutphasenproteinen kann eine komplexe Allgemeinreaktion hervorrufen, bei der Gewebeschäden verschiedenster Art, Fieber und lokale Symptome wie Entzündungen und Nekrosen auftreten können. Meist ist das Blutbild verändert. NF-κB induziert zum Beispiel stark das Serum Amyloid A Vorläuferprotein in der Leber im Zuge einer Induktion von Akutphasenproteinen.

Beispielsweise kann die NF-κB-vermittelte Genexpression des Interleukin-2-(Il-2)-Gens gehemmt werden.

Interleukin-2 ist ein Cytokin das u.a. als hämatopoetischer Wachstumsfaktor eine zentrale Rolle bei diversen entzündlichen Prozessen spielt (Annu. Rev. Immunol. 12 141 (1994)). Der Promotor des Interleukin-2 Gens ist NF-κB abhängig. Eine Inhibition der NF-κB Stimulierung eröffnet somit die Möglichkeit, ein Überschießen der 11-2 Produktion zu verhindern und somit entzündliche Prozesse zu therapieren.

Bei anderen Krankheitsbildern wie Gewebeschädigung nach Reperfusion oder Leberzirrhose können Hemmstoffe der NF-κB-vermittelten Genexpression ebenfalls einen bedeutenden Therapiefortschritt darstellen. Es gibt Evidenzen, daß durch Oxidationsreaktionen, die zu oxidativem Streß nach Reperfusion von ischämischem Gewebe führen, NF-κB-gesteuerte Gene induziert werden. Auf diese Weise wird eine Überexpression von Zytokinen und Zelladhäsionsmolekülen im ischämischen Gewebe eine übermäßige Rekrutierung von infiltrierenden Lymphozyten ausgelöst. Die rekrutierten Lymphozyten tragen ursächlich zur Gewebeschädigung bei.

Ebenso ist die Beteiligung der NF-κB-gesteuerten Genexpression bei mehreren neurodegenerativen Erkrankungen evident. Insbesondere bei Nervenkrankheiten, bei denen der Redoxzustand von Zellen des neuronalen Gewebes gestört ist, wird der selektiven Hemmung von Genen mit NF-κB Bindesequenz ein therapeutischer Nutzen beigemessen. Ein gestörter Redoxzustand neuronaler Zellen wird bei amytropher Lateralsklerose und bei Down-Syndrom angenommen.

Es ist bekannt, daß NF-κB ein häufig anzutreffender Transkriptionsfaktor in neuronalem Gewebe ist und daß NF-κB im Gehirn ein Redoxpotential-gesteuerter Transkriptionsfaktor ist (P.A. Bauerle, T. Henkel, Annu. Rev. Immunol. 12, 141-179, 1994). Ein Aufstellung der Gene, die durch NF-κB induziert werden, ist in Tabelle 1 wiedergegeben.

**Tabelle 1**

| Gene, die durch NF-κB induziert werden (P.A. Bauerle, T. Henkel, Annu. Rev. Immunol. 12, 141-179, 1994) | |
|---|---|
| Immunorezeptoren | Immunoglobulin κ light chain T cell receptor β T cell receptor α chain (human) Major histocompatibility complex class I (H-2K) β₂-Microglobulin Invariant chain I Tissue factor-1 |
| Zelladhäsionsmoleküle | Endothelial leukocyte adhesion molecule 1 (ELAM-1) Vascular cell adhesion molecule 1 (VCAM-1) Intercellular cell adhesion molecule 1 (ICAM-1)* |
| Zytokine und Wachstumsfaktoren | β-Interferon Granulocyte/macrophage colony-stimulating factor (GM-CSF) Granulocyte colony-stimulating factor (G-CSF) Macrophage colony-stimulating actor (M-CSF) Melanoma growth stimulating activity (groα-γ/MGSA) Interleukin-2 Interleukin-6 Interleukin-8 TNFα Lymphotoxin (TNF-β) Proenkephalin MPC-1/JE* |
| Akutphasenproteine | Angiotensinogen Serum amyloid A precursor Complement factor B Complement factor c4 Urokinase-type plasminogen activator* |

| | |
|---|---|
| * Die Bindung von NF-κB an den Promotor des genannten Gens ist noch nicht schlüssig experimentell nachgewiesen. | |

Neben den bereits genannten Genen, deren Aktivität mit der Freisetzung des NF-κB gesteuert wird und die besonders bei Entzündungsprozessen, septischem Schock und Transplantatabstoßung eine Rolle spielen, seien auch noch NF-κB-gesteuerte Gene in Viren erwähnt und solche, die oncogene zelluläre Veränderungen hervorrufen (Oncogene wie c-myc, c-rel, Melanoma Growth Stimulating Activity MGSA). Auch bei diesen Genen stellt eine selektive Hemmung der NF-κB Bindung ein vielversprechendes, therapeutisch nutzbares Konzept dar. Die Genexpression lymphotropher Viren wie HIV, HTLV und Epstein-Barr Virus wird entweder direkt oder durch NF-κB aktiviert oder in der infizierten Wirtszelle wird NF-κB induziert, was der Virusreplikation förderlich ist. Neben HIV wirkt NF-κB positiv auf die Genexpression im Zytomegalievirus (CMV) und Adenovirus. Auch hier sind antivirale Effekte mit NF-κB Inhibitoren denkbar.

Die Herstellung und die Verwendung der erfindungsgemäßen Stoffe gehen aus den nachfolgendenen Beispielen hervor.

### Herstellbeispiele

### Beispiel I-1

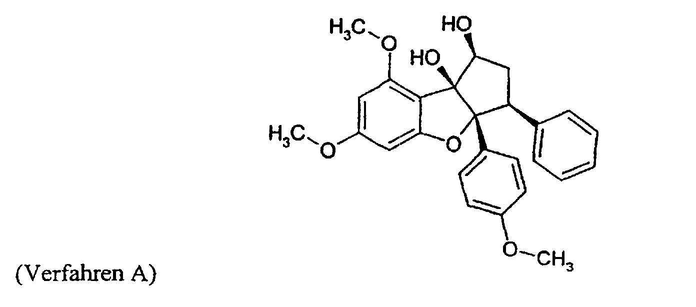

Zu 1,92 g (12,8 mmol) Samarium wird eine Lösung von 1,83 g (6,5 mmol) Diiodethan in 7 ml THF gegeben und man läßt für 30 min. rühren. Nach weiteren 2 h im Ultraschallbad werden 25 ml Benzol zugegeben und man läßt für weitere 2 h rühren. Dann wird eine Lösung von 1,40 g (3,24 mmol) (S*,R*)-2,3-Dihydro-4,6-diethoxy-2-(4-methoxyphenyl)-3-oxo-β-phenyl-2-benzofuranpropanal (rac.) (z.B. aus Beispiel II-1) in 45 ml Benzol hinzugegeben, und man läßt nochmals für 3 h im Ultraschallbad und dann für weitere 12 h bei Raumtemperatur rühren. Nach Zugabe von 30 ml 1 N HCl wird mit Ether extrahiert, über MgSO₄ getrocknet und eingeengt. Chromatographie mit t-Butylmethylether : Toluol : Cyclohexan = 2 : 1 : liefert 533 mg (1,23 mmol, 38%) racemisches (1α,3α,3aα,8bα)-3,3a-Dihydro-6,8-dimethoxy-3a-(4-methoxyphenyl)-3-phenyl- 1H-cyclopenta[b]benzofuran-1,8b(2H)-diol.
¹H-NMR (CDCl₃): δ (ppm) 2,38 (ddd, 1 H), 2,51 (s, 1 H), 2,63 (m, 1 H), 3,08 (d, 1 H), 3,46 (dd, 1 H), 3,72 (s, 3 H), 3,84 (s, 3 H), 3,86 (s, 3 H), 4,80 (dt, 1 H), 6,10 (d, 1 H), 6,27 (d, 1 H), 6,69 (d, 2 H), 6,98-7,12 (m, 5 H), 7,20 (d, 2 H).

### Beispiel I-2

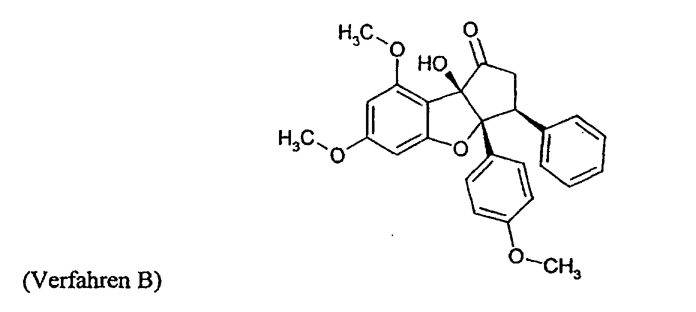

Bei -78°C werden zu einer Lösung von 74 µl (0,86 mmol) Oxalylchlorid in 2,5 ml THF 66 µl (0,92 mmol) DMSO addiert. Nach etwa 5 min wird eine Lösung des Diols aus Beispiel I-1 (250 mg, 0,576 mmol) in 8 ml THF hinzugegeben und man läßt für weitere 30 min bei -78°C rühren. Dann werden 1,19 ml (8,6 mmol) Triethylamin hinzugegeben und man läßt 1 h bei -78°C und dann über Nacht bei Raumtemperatur rühren. Es wird mit 15 ml IN HCI hydrolysiert und mit Dichlormethan extrahiert. Nach dem Trocknen über MgSO₄ wird mit t-Butylmethylether : Cyclohexan : Toluol = 2 : 1 : 1 chromatographiert. Ausbeute 193 mg (0,446 mmol, 77%) racemisches (3α,3aα,8bα)-2,3,3a,8b-Tetrahydro-8b-hydroxy-6,8-dimethoxy-3a-(4-methoxyphenyl)-3-phenyl-cyclopenta[b]benzofuran-1-on.
¹H-NMR (CDCl₃): δ (ppm) 2,94-3,10 (m, 2 H), 3,10 (s, 1 H), 3,70 (s, 3 H), 3,83 (s, 3 H), 3,85 (s, 3 H), 3,89 (dd, 1 H), 6,10 (d, 1 H), 6,34 (d, 1 H), 6,68 (d, 2 H), 6,92-6,98 (m, 4 H), 7,08-7,13 (m, 3 H).

### Beispiel I-3

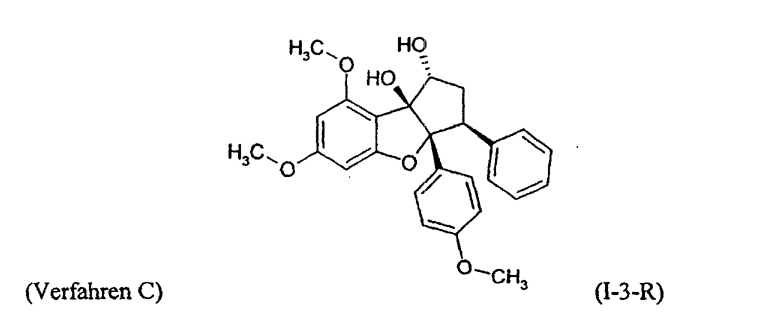

304 mg (1,16 mmol) Tetramethylammoniumtrisacetoxyborhydrid werden in 1,2 ml Acetonitril mit 1,2 ml Essigsäure versetzt. Nach 30 min wird bei Raumtemperatur 100 mg (0,231 mmol) des Ketons aus Beispiel I-2 in 2,0 ml Acetonitril hinzugegeben. Man läßt über Nacht rühren, hydrolysiert vorsichtig mit 25 ml ges. NaHCO₃ und extrahiert mit Dichlormethan Nach dem Trocknen über MgSO₄ wird eingeengt und mit t-Butylmethylether : Cyclohexan : Toluol = 2 : 1 : 1 chromatographiert. Ausbeute: 100 mg (0,23 mmol, 100%) racemisches (1α,3β,3aβ,8bβ)-3,3a-Dihydro-6,8-dimethoxy-3a-(4-methoxyphenyl)-3-phenyl-1H-cyclopenta[b]benzofuran-1,8b(2H)-diol.
¹H-NMR (CDCl₃): δ (ppm) 2,20 (dd, 1 H, J = 13,6, 6,6), 2,36 (s, 1 H), 2,75 (td, 1 H, J = 13,8, 6,4), 3,29 (s, 1 H), 3,71 (s, 3 H), 3,84 (s, 3 H), 3,91 (s, 3 H), 4,01 (dd, 1 H, J = 14,0, 6,5), 4,82 (d, 1 H, J = 6,3), 6,15 (d, 1 H, J = 1,9), 6,29 (s, 1 H, J = 1,9), 6,68 (d, 2 H, J = 8,9), 6,98-7,19 (m, 7 H).

Die direkte flüssigchromatographische Trennung dieses Racemates erfolgte unter Verwendung einer chiralen stationären Polyamid-Kieselgelphase mit 10 mm Korngröße basierend auf dem Selektor Poly-(N-Methacryloyl-L-leucin-d-menthylamid) und der mobilen Phase aus n-Heptan und THF (2/1; vol/vol). Beschrieben wurden solche Phasen in EP-A 0 379 917 und Angew. Chem. 103, 1685-1687 (1991). Auf einer Säule 500* 30 mm konnten bei einem Fluß von 25 ml/min pro Lauf 200 mg nahezu quantitativ getrennt werden. Das oben abgebildete [lR-(1α,3β,3aβ,8bβ)]-konfigurierte Isomer, hier als Verbindung (I-3-R) bezeichnet, wurde als zweite, das spiegelbildliche (S,R,S,R)-konfigurierte Isomer (I-3-S) als erste Substanz eluiert. Der gefundene optische Drehwert [α]_{D} von Enantiomer (I-3-R) stimmt gut mit dem des bereits bekannten des Naturstoffes überein: [α]_{D} = -131,4 (c = 0,4 CHCl₃)

### Beispiel I-4

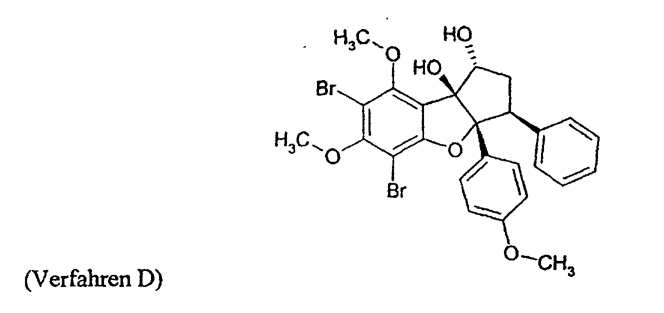

100 mg (0,23 mmol) des Diols aus Beispiel 1-3 werden in 5 ml Dichlormethan vorgelegt und portionsweise mit 112 mg (0,47 mmol) Brom-Pyridin-Komplex versetzt. Nach 1 h wird Ether addiert, mit Wasser gewaschen, über MgSO₄ getrocknet und eingeengt. Ausbeute 97mg (0,15 mmol, 63%) racemisches (1α,3β,3aβ,8bβ)-5,7-Dibrom-3,3a-dihydro-6,8-dimethoxy-3a-(4-methoxyphenyl)-3-phenyl-1H-cyclopenta[b]benzofuran-1,8b(2H)-diol.
¹H-NMR (CDCl₃): δ (ppm) 2,23 (dd, 1 H), 2,81 (td, 1 H), 3,46 (s, 1 H), 3,72 (s, 3 H), 3,95 (s, 3 H), 4,02 (s, 3 H), 4,84 (d, 1 H), 6,71 (d, 2 H), 7,01-7,19 (m, 7 H).

### Beispiel I-5

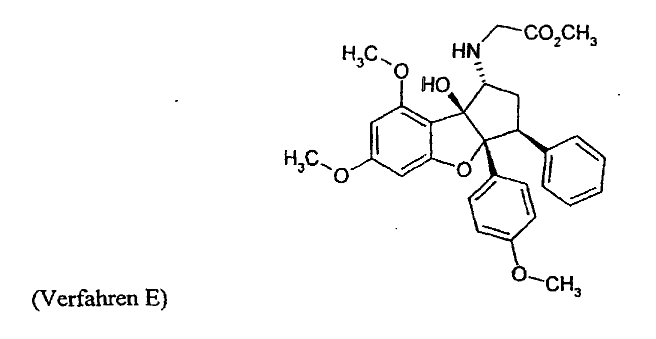

100 mg (0,23 mmol) des Ketons aus Beispiel 1-2 werden in 6 ml Dichlormethan gelöst und mit 29 mg (0,23 mmol) Glycinmethylesterhydrochlorid und 250 mg Molekularsieb 4 Å für 3 h bei Raumtemperatur gerührt. Dann werden 85 mg (0,32 mmol) Tetramethylammoniumtrisacetoxyborhydrid hinzugegeben und man läßt über Nacht rühren. Nach Filtration wird NaH₂PO₄/Na₂HPO₄-Puffer hinzugegeben und mit Dichlormethan extrahiert. Nach dem Trocknen über MgSO₄ wird chromatographisch gereinigt (t-Butylmethylether : Toluol : Cyclohexan = 2 : 1 : 1). Ausbeute: 37 mg (0,07 mmol, 32%) (1α,3β,3aβ,8bβ)-*N*-(3,3a-Dihydro-6,8-dimethoxy-3a-(4-methoxyphenyl)-3-phenyl-cyclopenta[b]benzofuran-8b(2H)-ol-1-yl)-2-aminoessigsäuremethylester.
¹H-NMR (CDCl₃): δ (ppm) 2,19 (ddd, 1 H), 2,62 (td, 1 H), 3,50 (AB, 2 H), 3,69 (s, 3 H), 3,73 (s, 3 H), 3,77 (dd, 1 H), 3,83 (s, 3 H), 3,88 (s, 3 H), 4,11 (dd, 1 H), 6,11 (d, 1 H), 6,24 (d, 1 H), 6,64 (d, 2 H), 7,04-7,27 (m, 9 H).

### Beispiel I-6

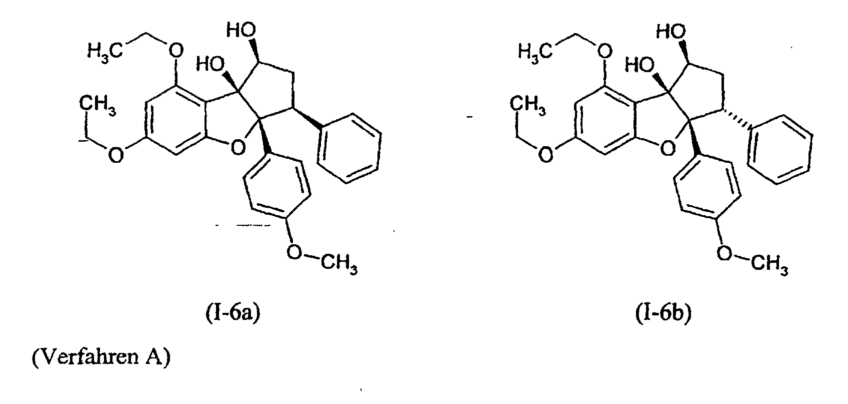

Analog Beispiel I-1 werden 1,31 g (2,85 mmol) 2,3-Dihydro-4,6-diethoxy-2-(4-methoxyphenyl)-3-oxo-β-phenyl-2-benzofuranpropanal (rac.) (z.B. aus Beispiel II-2), 1,50 g (9,96 mmol) Samarium und 2,81 g (9,96 mmol) Diiodethan eingesetzt. Nach RP-HPLC werden zwei Isomere erhalten: 149 mg racemisches trans-Isomer (1α,3β,3aα,8bα)-3,3a-Dihydro-6,8-diethoxy-3a-(4-methoxyphenyl)-3-phenyl-1H-cyclopenta[b]benzofuran-1,8b(2H)-diol (I-6b):
¹H-NMR (CDCl₃): δ (ppm) 1,40 (m, 6 H), 2,12 (dt 1 H), 2,23 (dd, 1 H), 2,39 (s, 1 H), 3,81 (s, 3 H), 3,99 (q, 2 H), 4,04 (q, 2 H), 4,26 (dd, 1 H), 4,56 (d, 1 H), 6,01 (d, 1 H), 6,07 (d, 1 H), 6,90 (d, 2 H), 7,02 (m, 2 H), 7,18 (m, 3 H), 7,42 (d, 2 H),
und 37 mg racemisches cis-Isomer (I-6a) (1α,3α,3aα,8bα)-konfiguriert:
¹H-NMR (CDCl₃): δ (ppm) 1,44 (m, 6 H), 2,48 (ddd, 1 H), 2,62 (m, 1 H), 3,47 (m, 1 H), 3,71 (s, 3 H), 4,07 (m, 4 H), 4,80 (t, 1 H), 6,09 (d, 1 H), 6,23 (d, 1 H), 6,70 (d, 2 H), 6,99-7,12 (m, 5 H), 7,19 (d, 2 H).

### Beispiel I-7

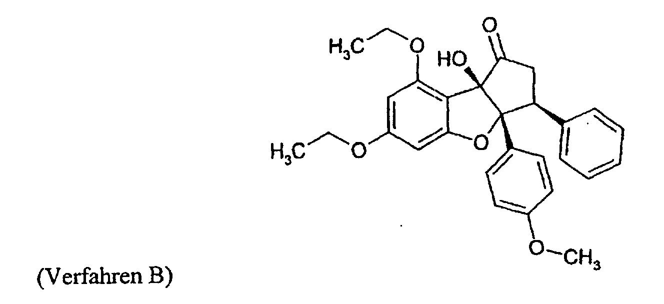

35 mg (0,076 mmol) Diol (I-6a) aus Beispiel I-6 werden in 0,2 ml DMSO gelöst und mit 84 mg (0,83 mmol) Triethylamin versetzt. 122 mg (0,77 mmol) Schwefeltrioxid-Pyridin Komplex werden in 0,6 ml DMSO hinzugegeben und es wird für 2 Tage gerührt. Nach Zugabe von NaH₂PO₄/NaH₂PO₄-Puffer wird mit Dichlormethan extrahiert, über MgSO₄ getrocknet und eingeengt. Man erhält 49 mg eines Öls. Das Massenspektrum und das Produkt der folgenden Umsetzung nach Beispiel I-8 korrelieren mit der Struktur des racemischen (3α,3aα,8bα)-8b-Hydroxy-6,8-diethoxy-3a-(4-methoxyphenyl)-3-phenyl-2,3,3a,8b-tetrahydrocyclopenta-[b]benzofuran-1-ons.

### Beispiel I-8

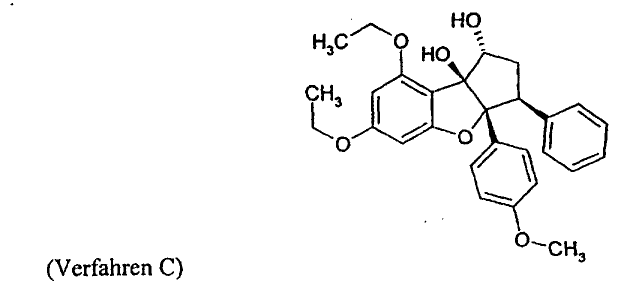

Analog zu Beispiel I-3 werden 49 mg Keton aus Beispiel 1-7 mit 302 mg (1,15 mmol) Tetramethylammoniumtrisacetoxyborhydrid umgesetzt. Ausbeute: 10 mg (0,02 mmol, 29%) racemisches (1α,3β,3aβ,8bβ)-3,3a-Dihydro-6,8-diethoxy-3a-(4-methoxyphenyl)-3-phenyl-1H-cyclopenta[b]benzofuran-1,8b(2H)-diol.
¹H-NMR (CDCl₃): δ (ppm) 1,48 (m, 6 H), 2,11 (dd, 1 H), 2,67 (dt, 1 H), 3,65 (s, 3 H), 3,92 (m, 1 H), 3,98 (m, 2 H), 4,08 (m, 1 H), 4,78 (d, 1 H), 6,06 (d, 1 H), 6,19 (d, 1 H), 6,61 (d, 2 H), 6,91-7,07 (m, 7 H).

### Beispiele I-9 bis I-79

Analog zu den Beispielen I-1 bis 1-8 und den allgemeinen Angaben zur Herstellung wurden die in der nachfolgenden Tabelle 2 aufgeführten Verbindungen der Formel (I-n) erhalten.

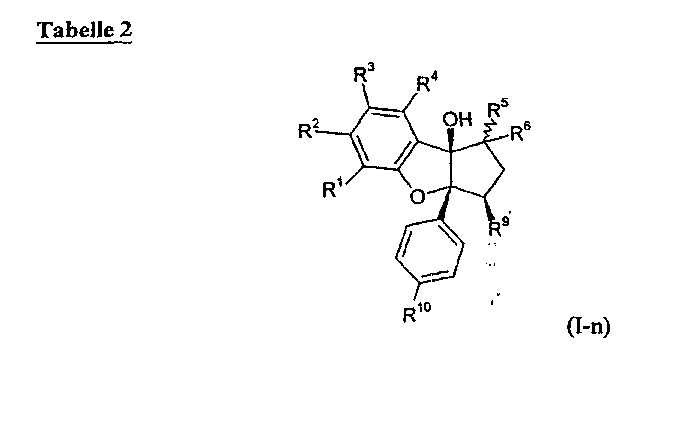

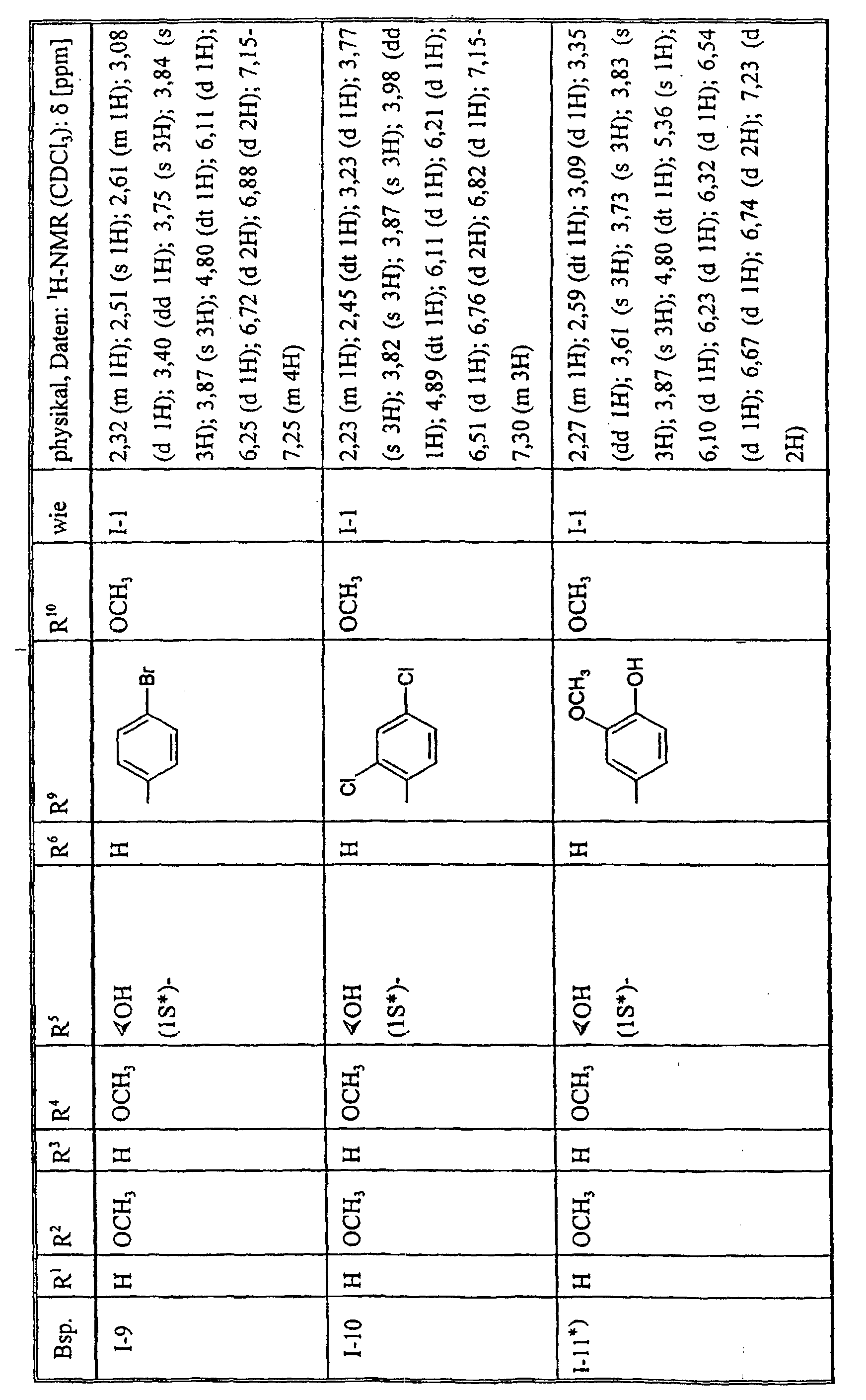

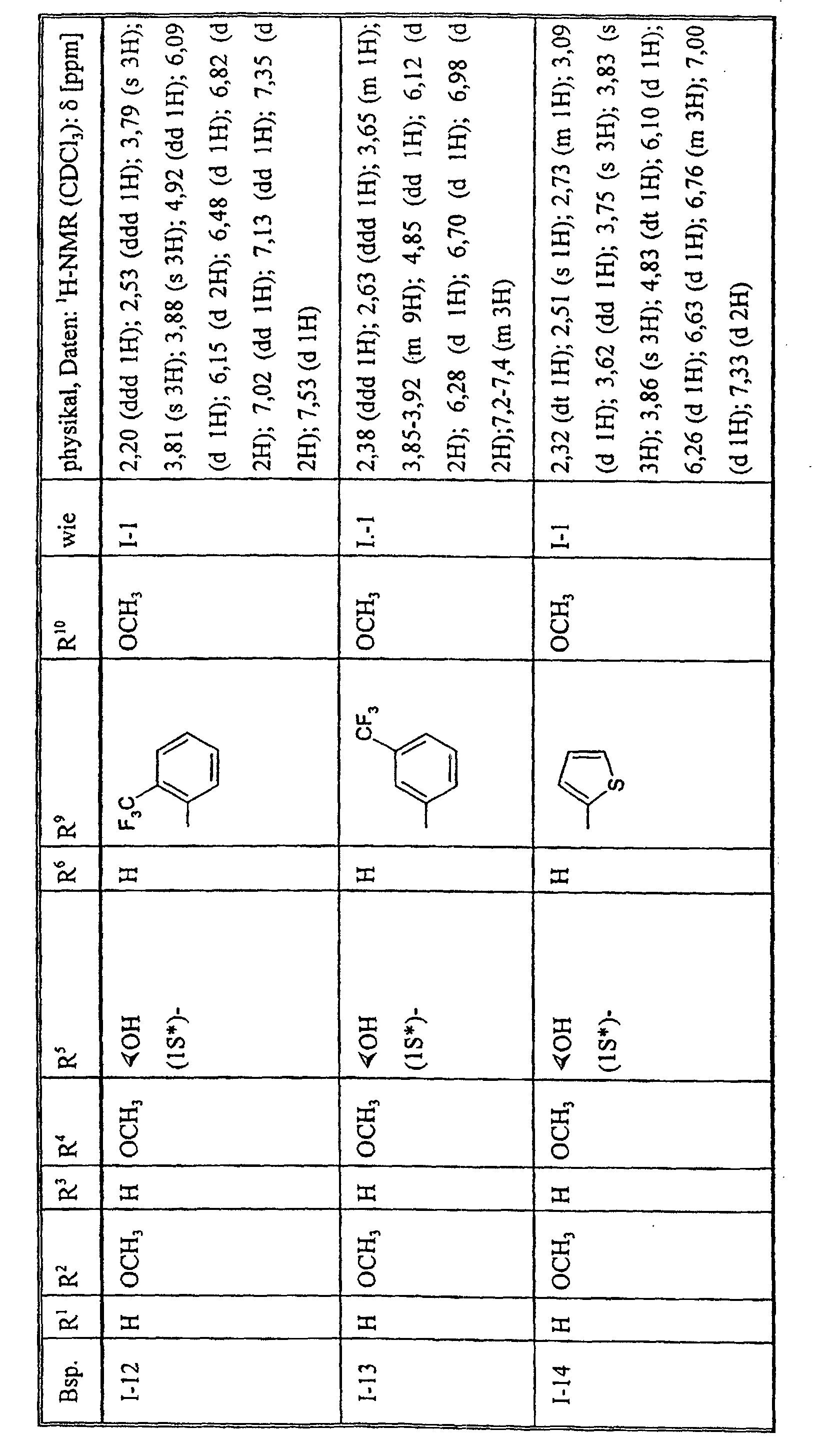

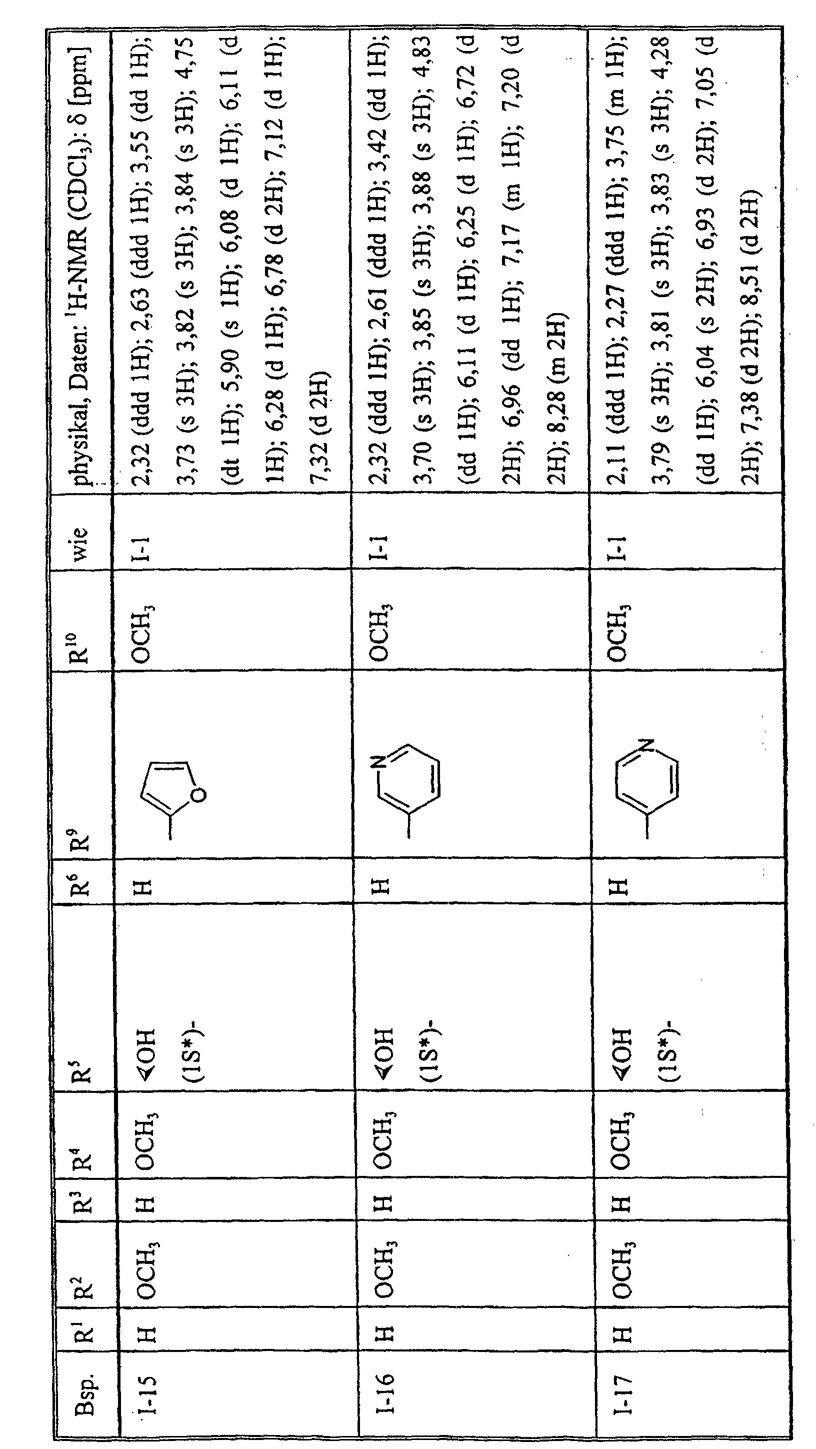

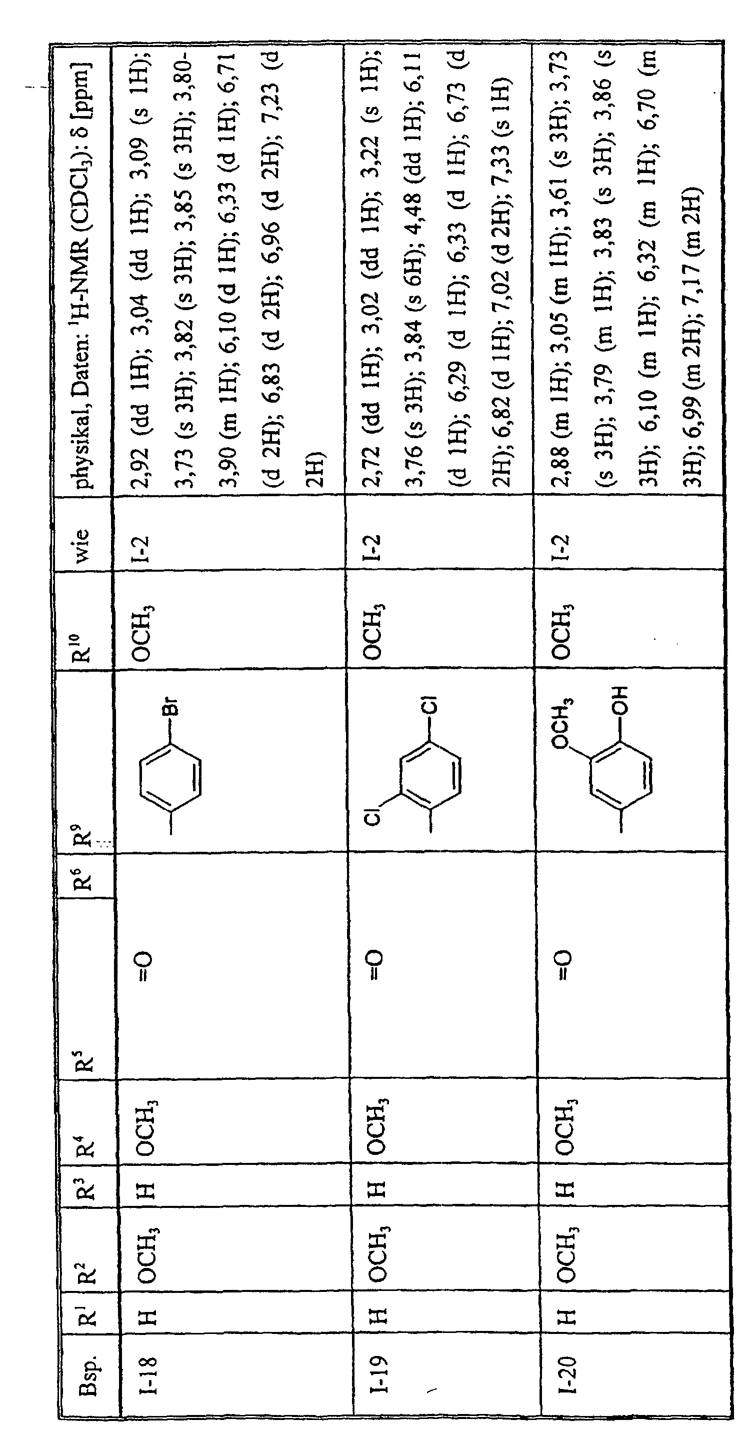

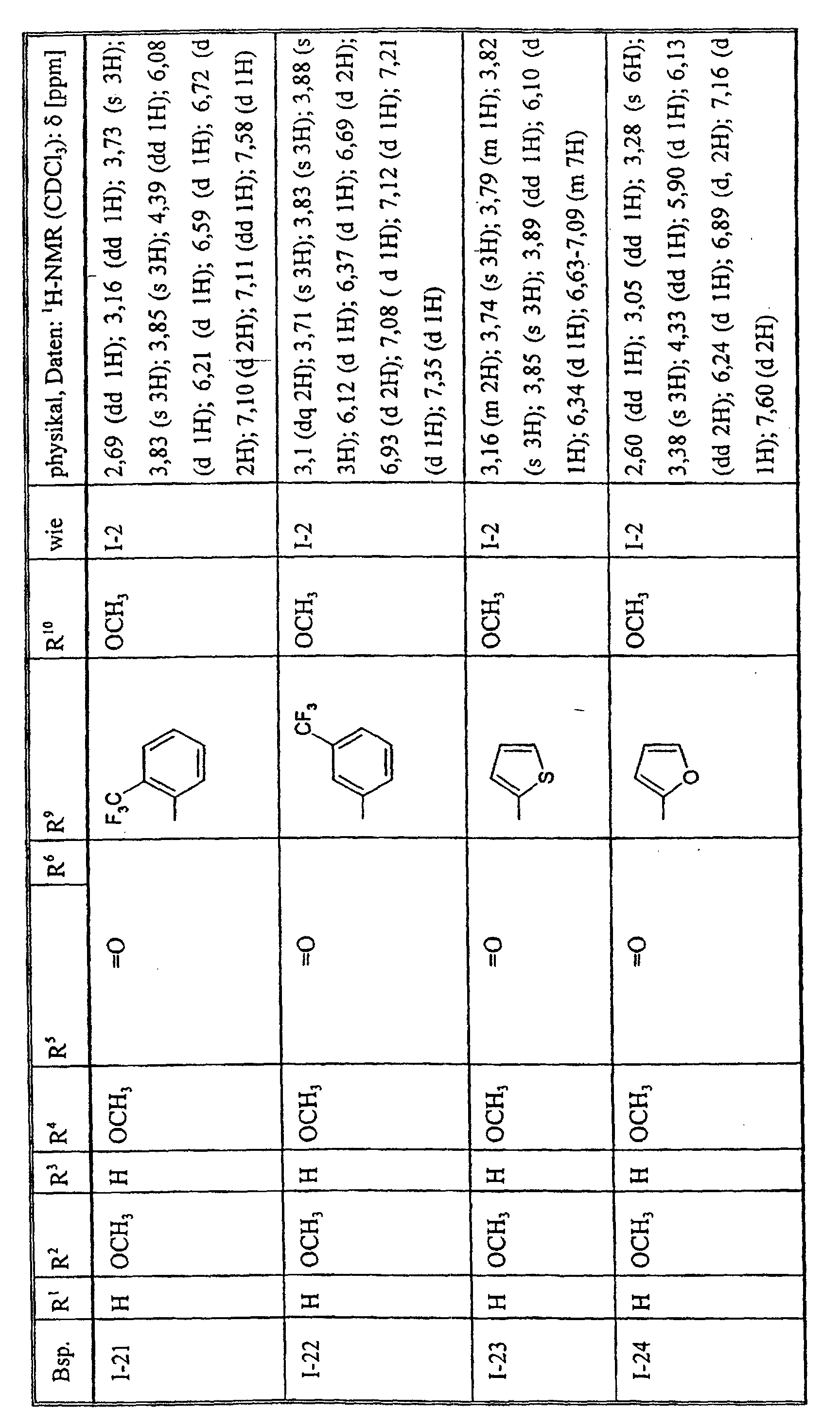

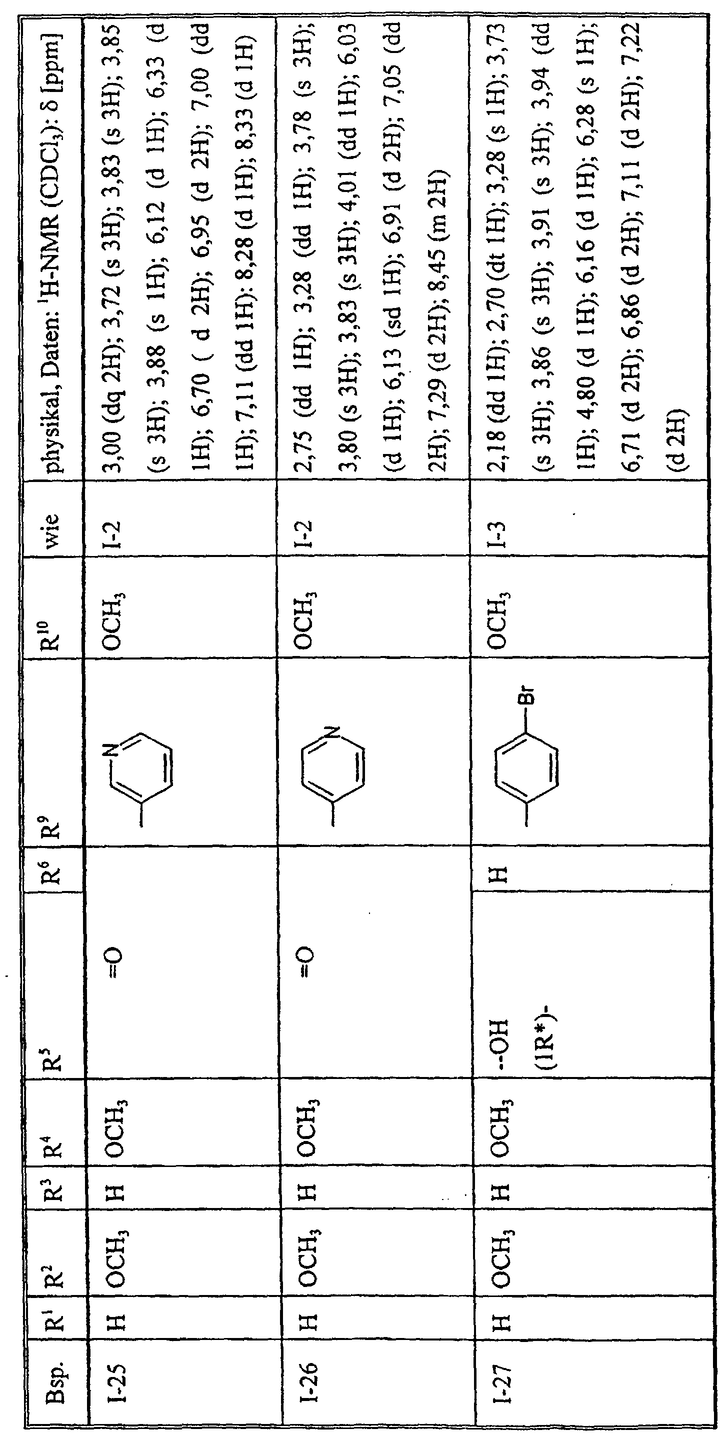

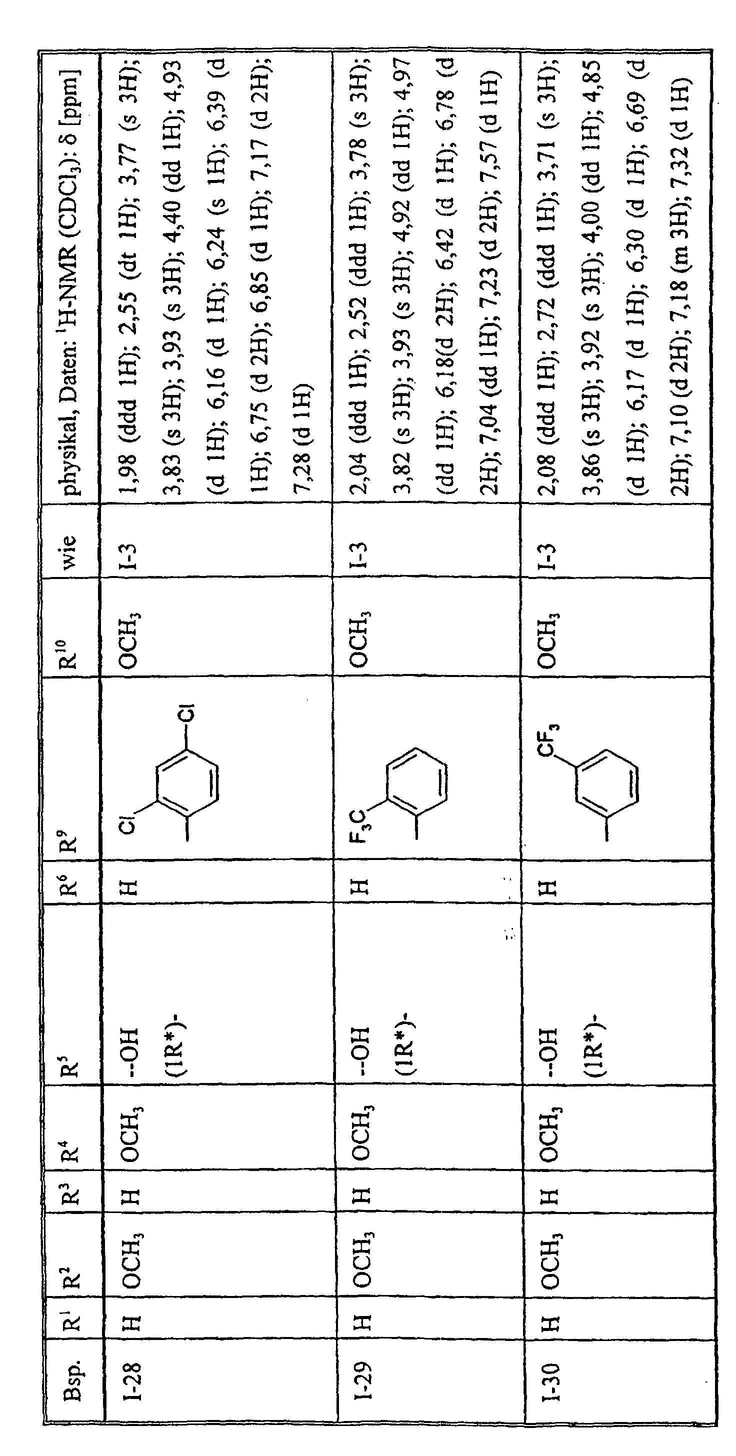

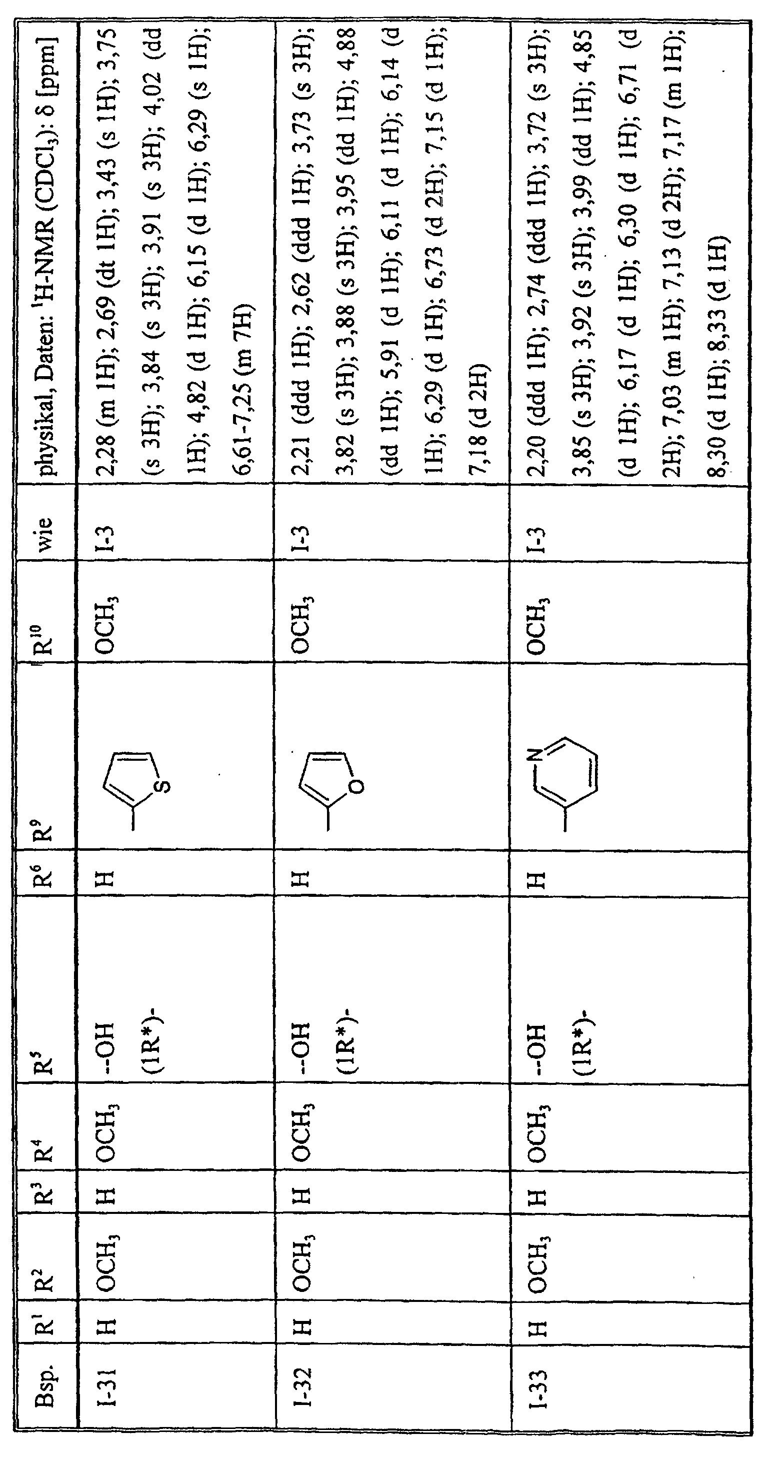

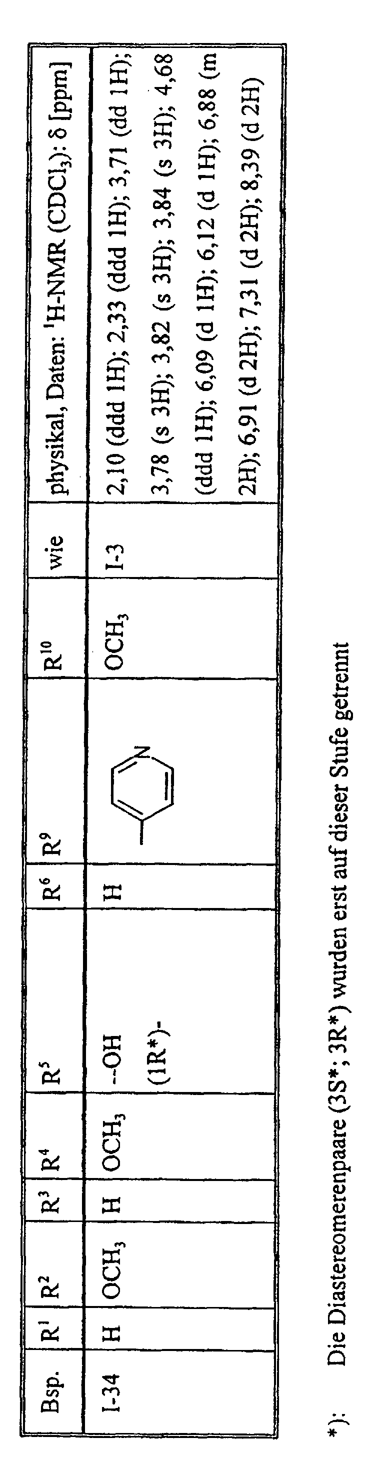

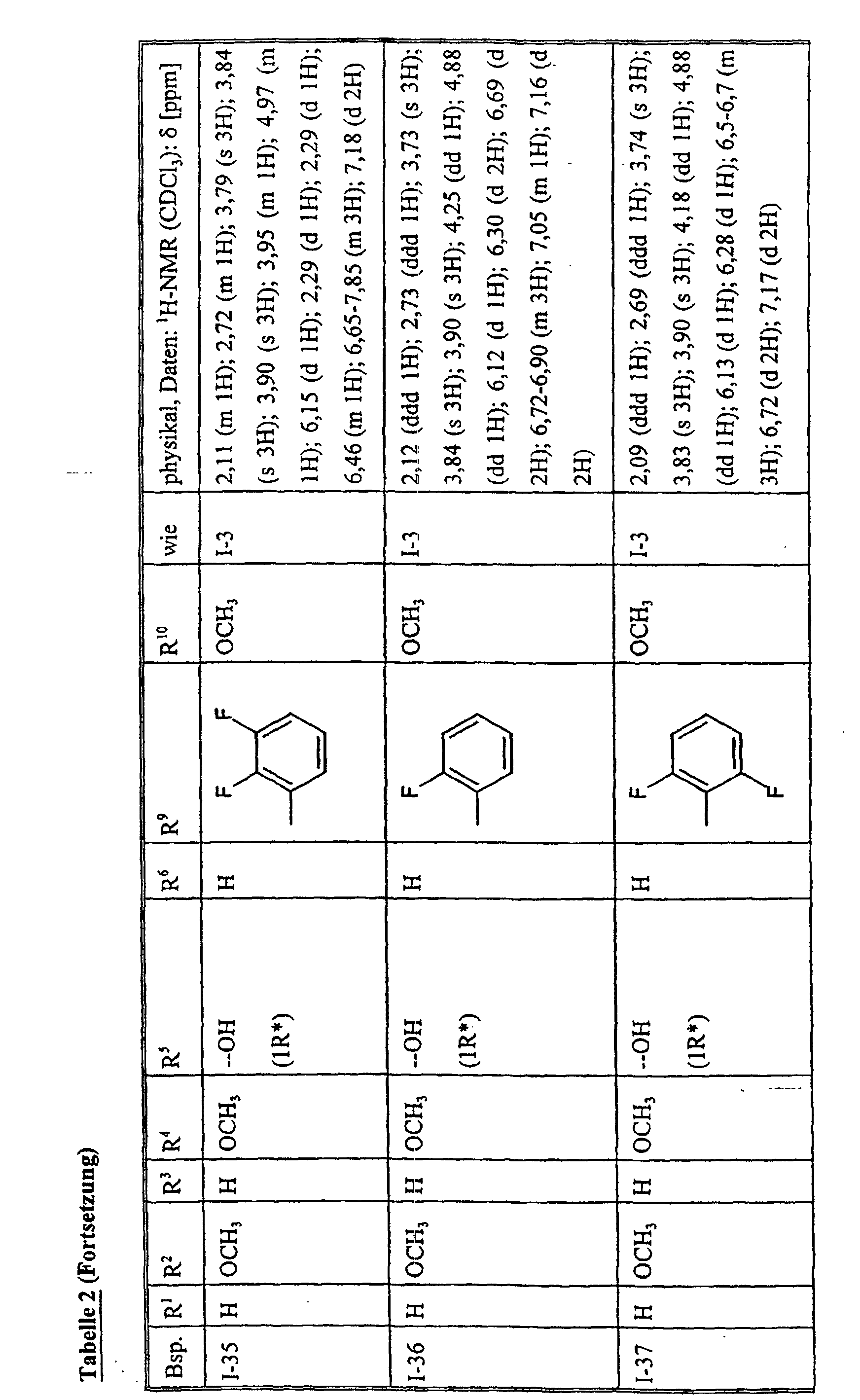

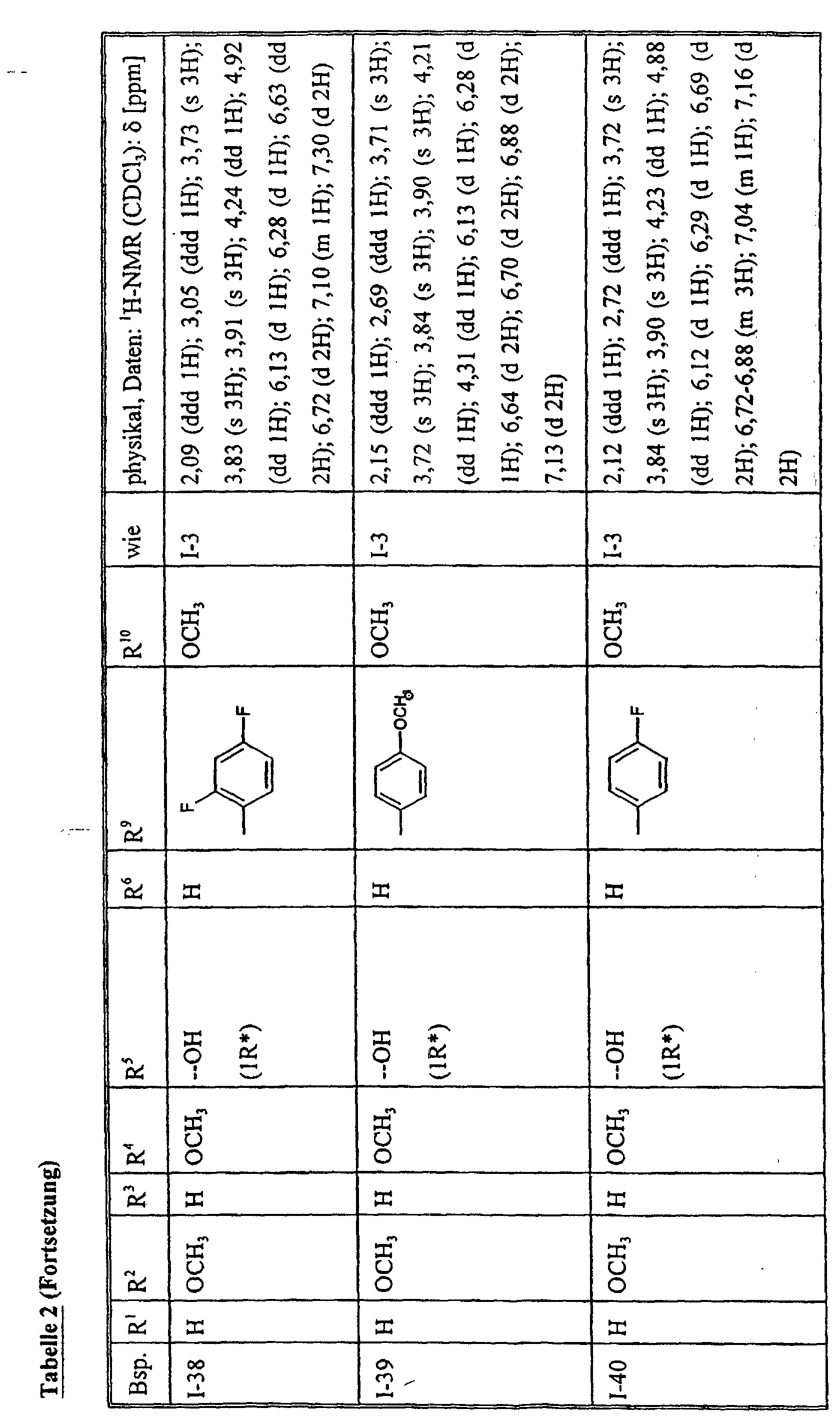

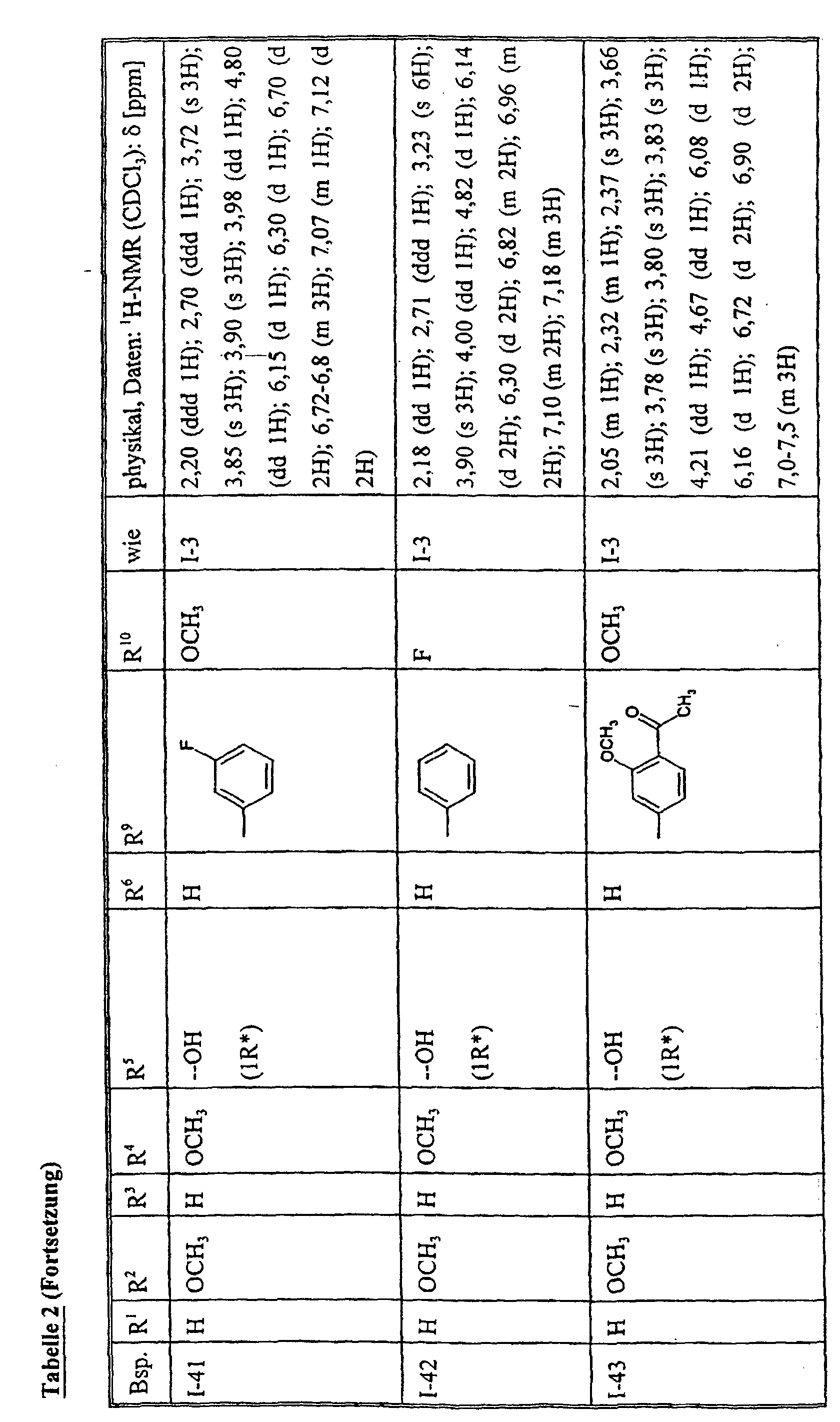

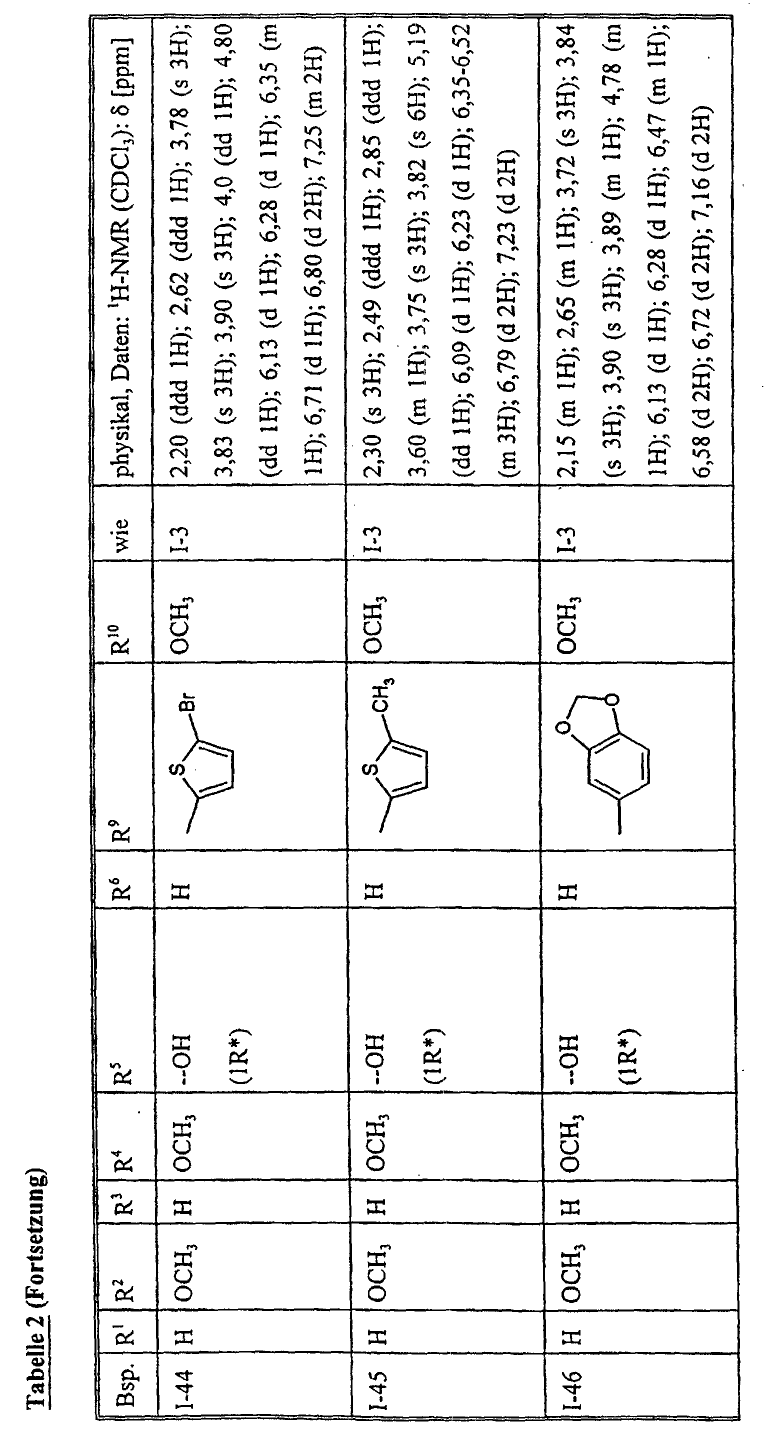

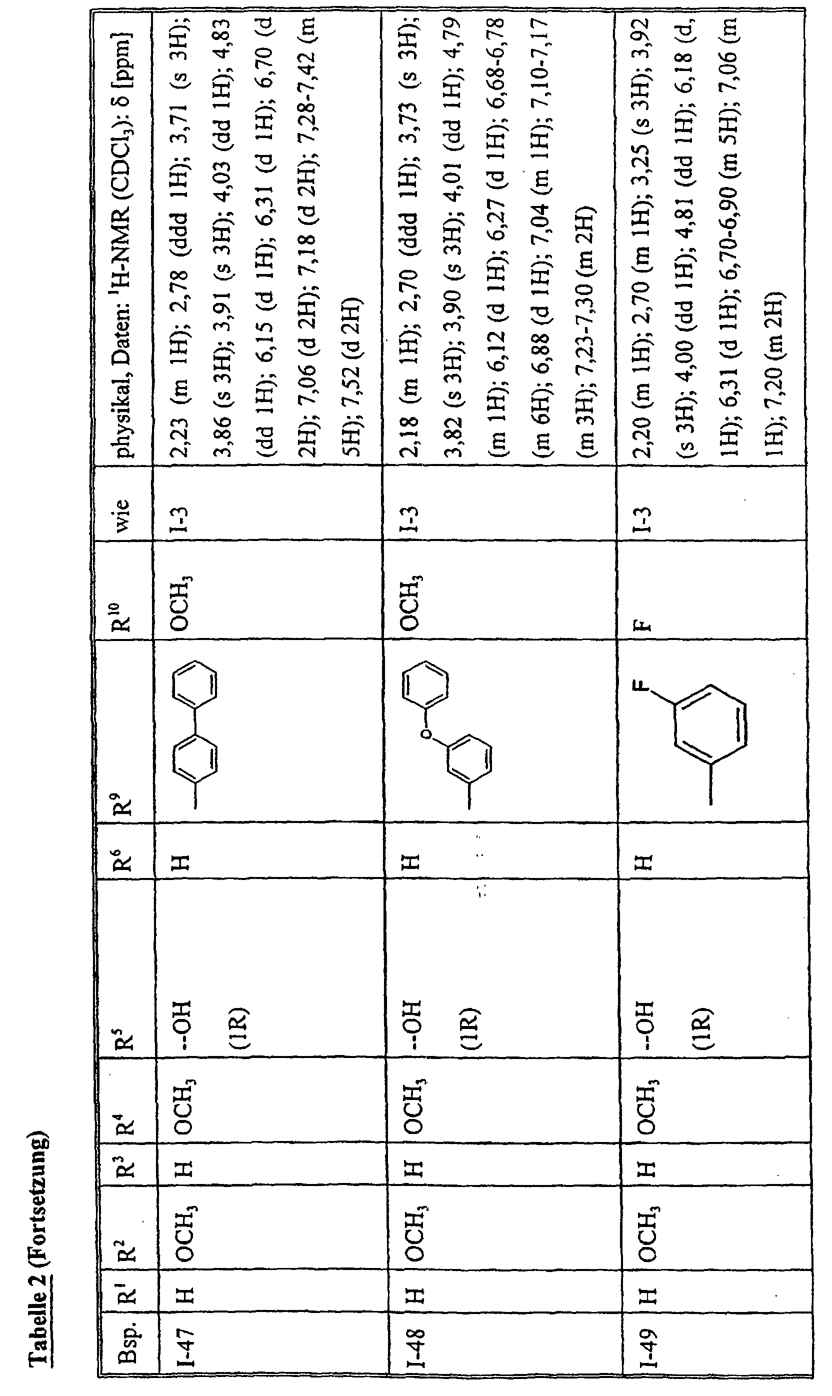

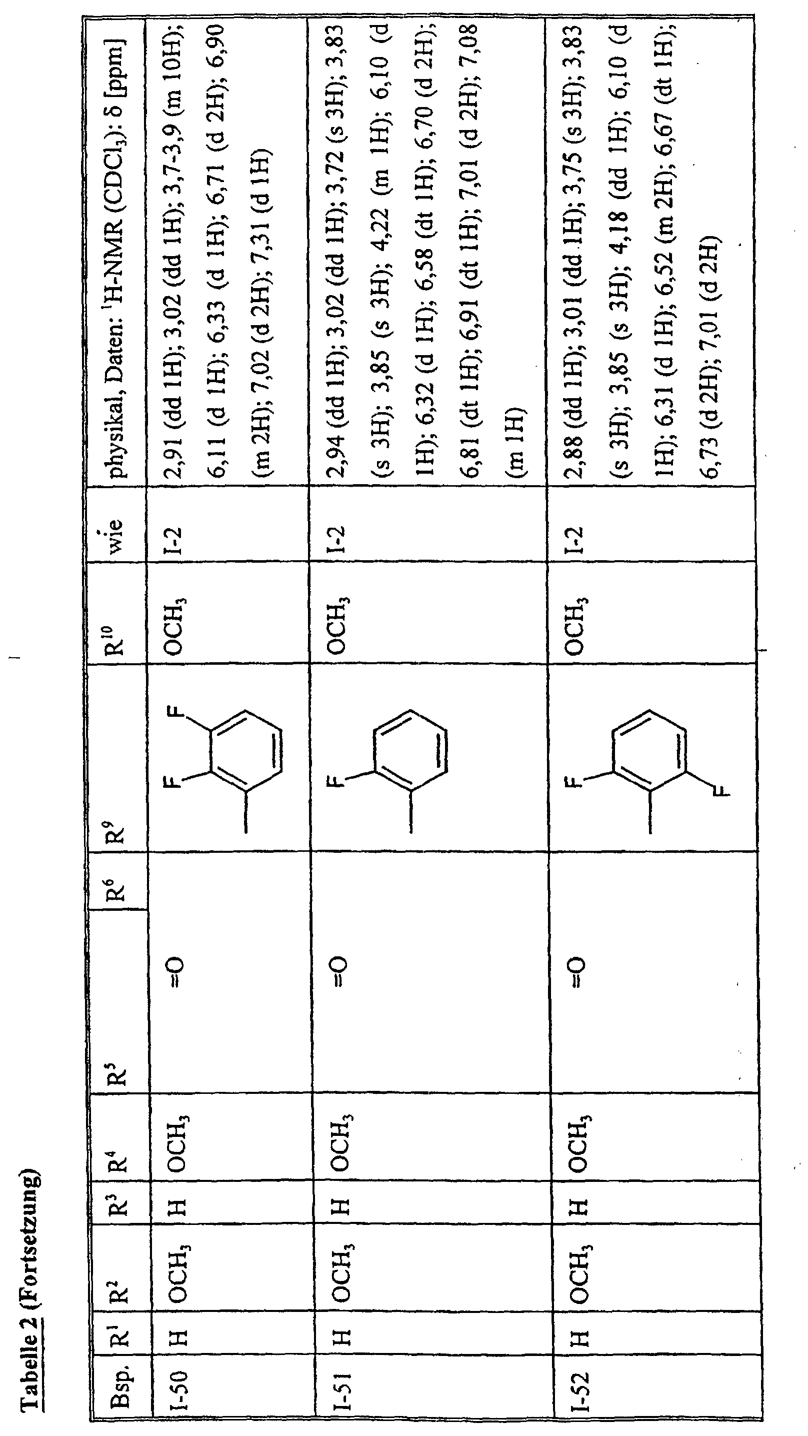

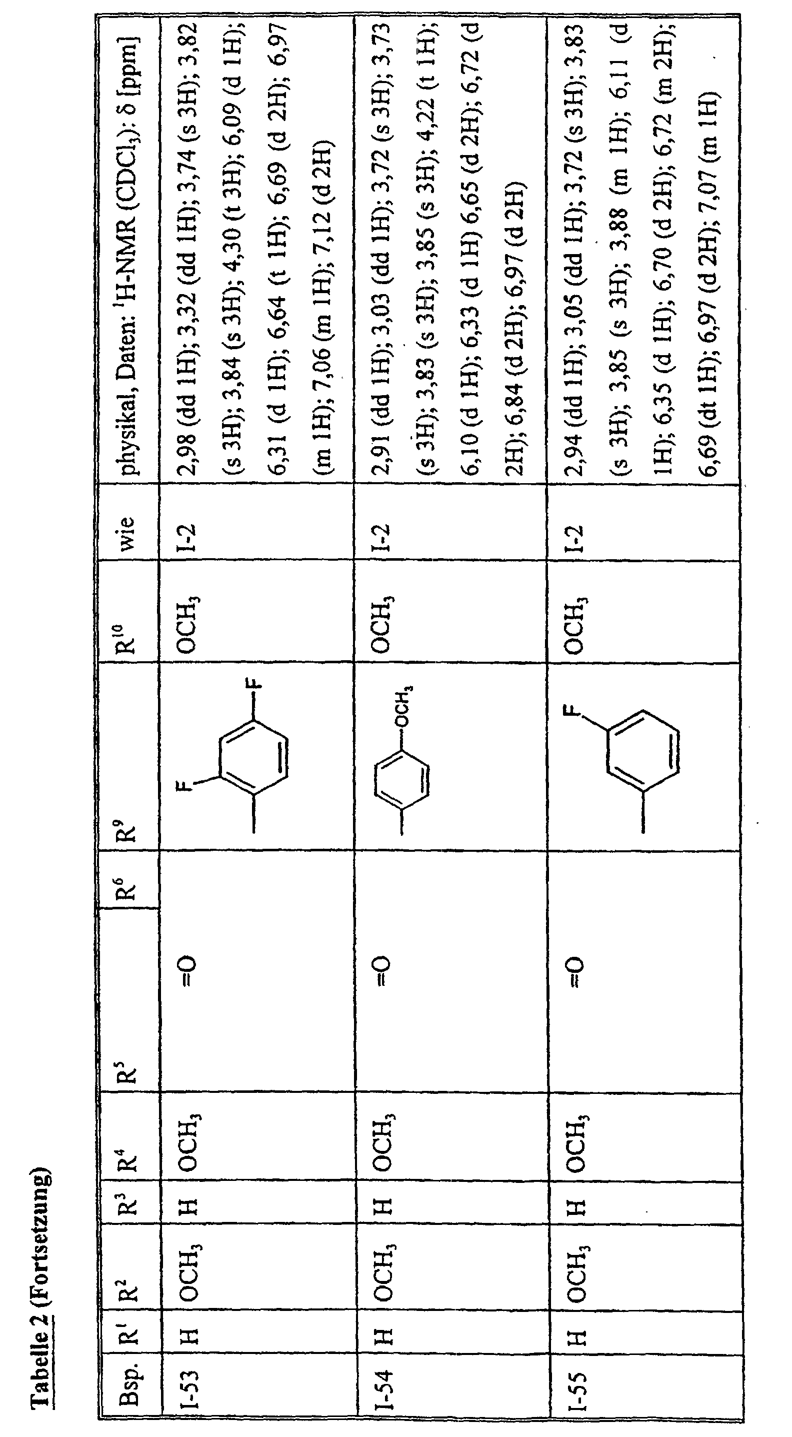

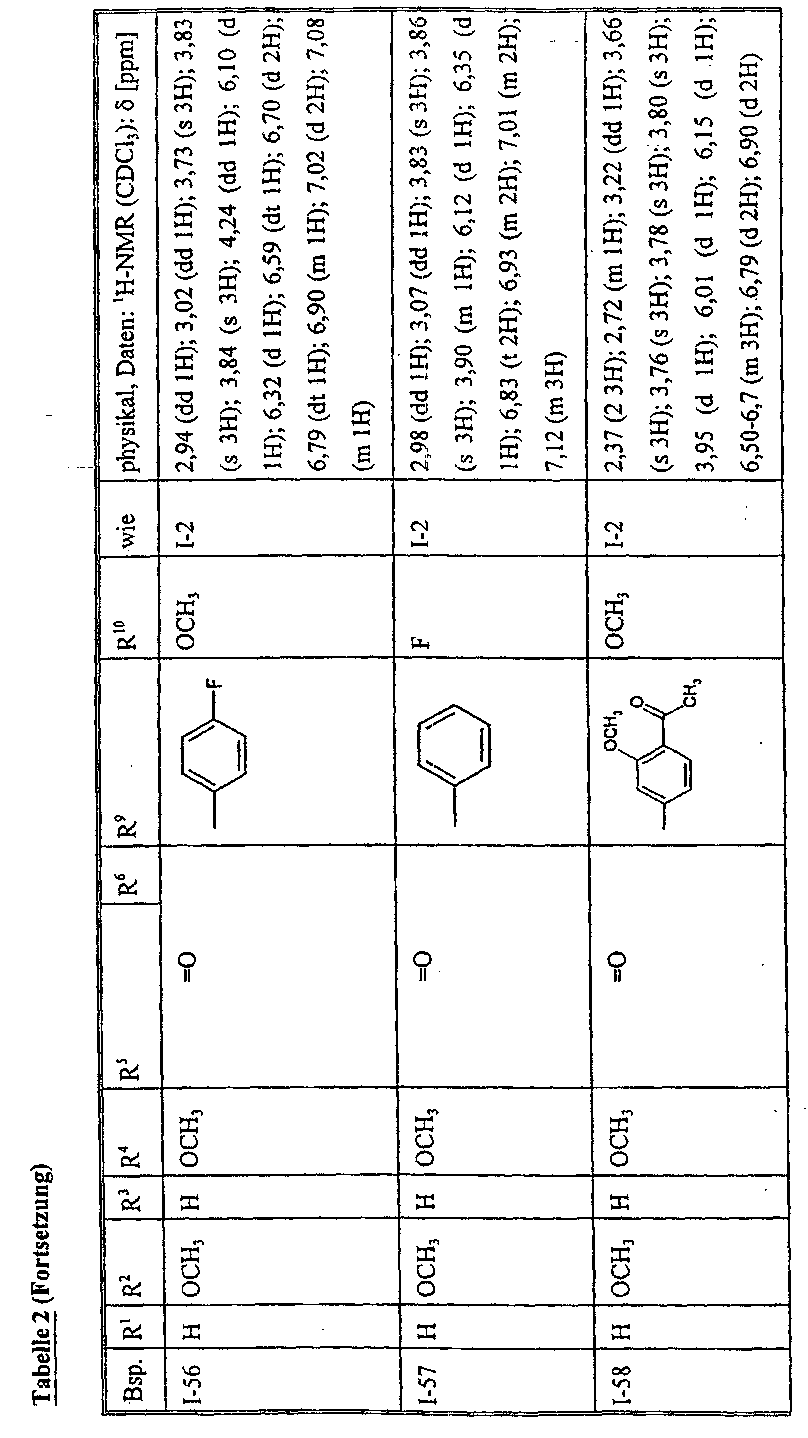

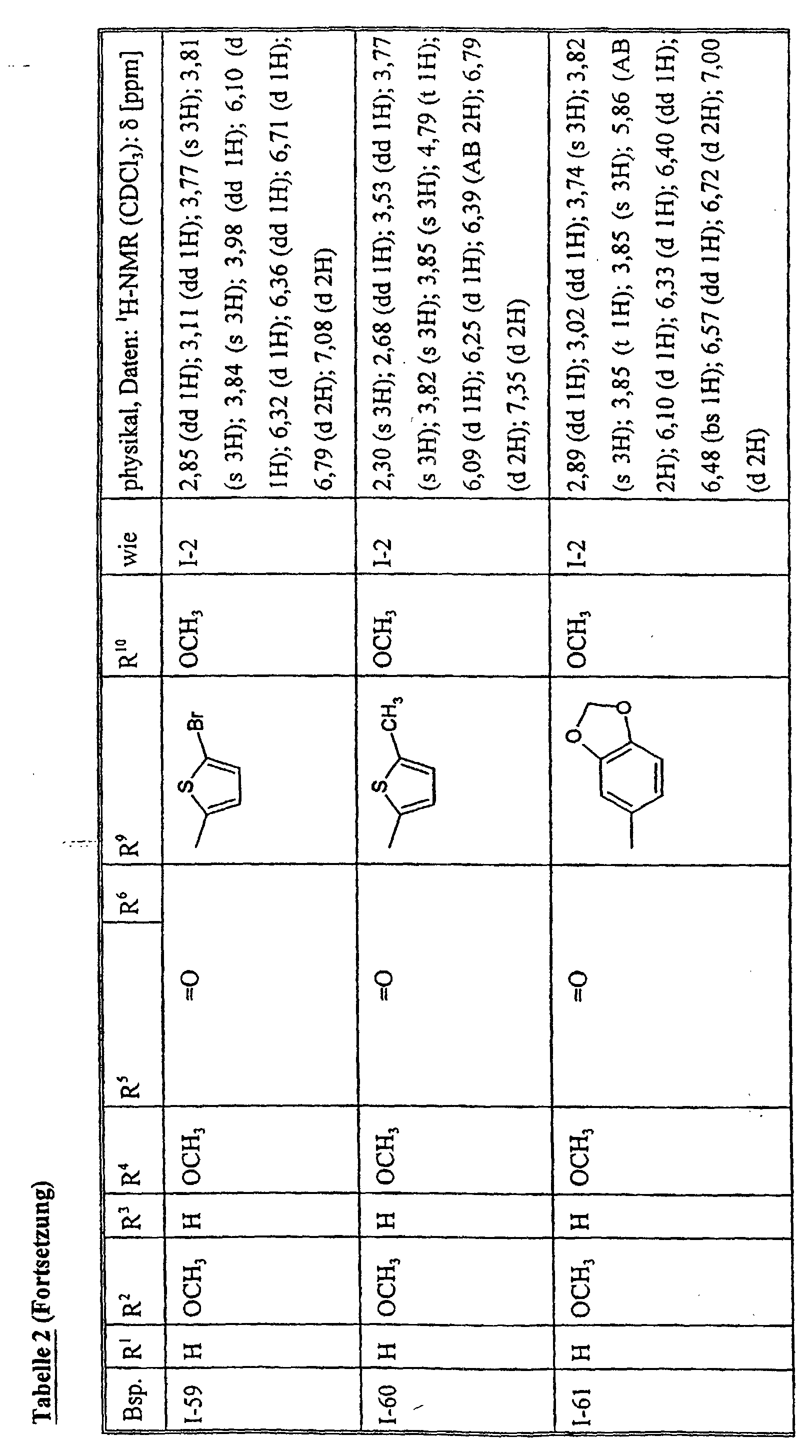

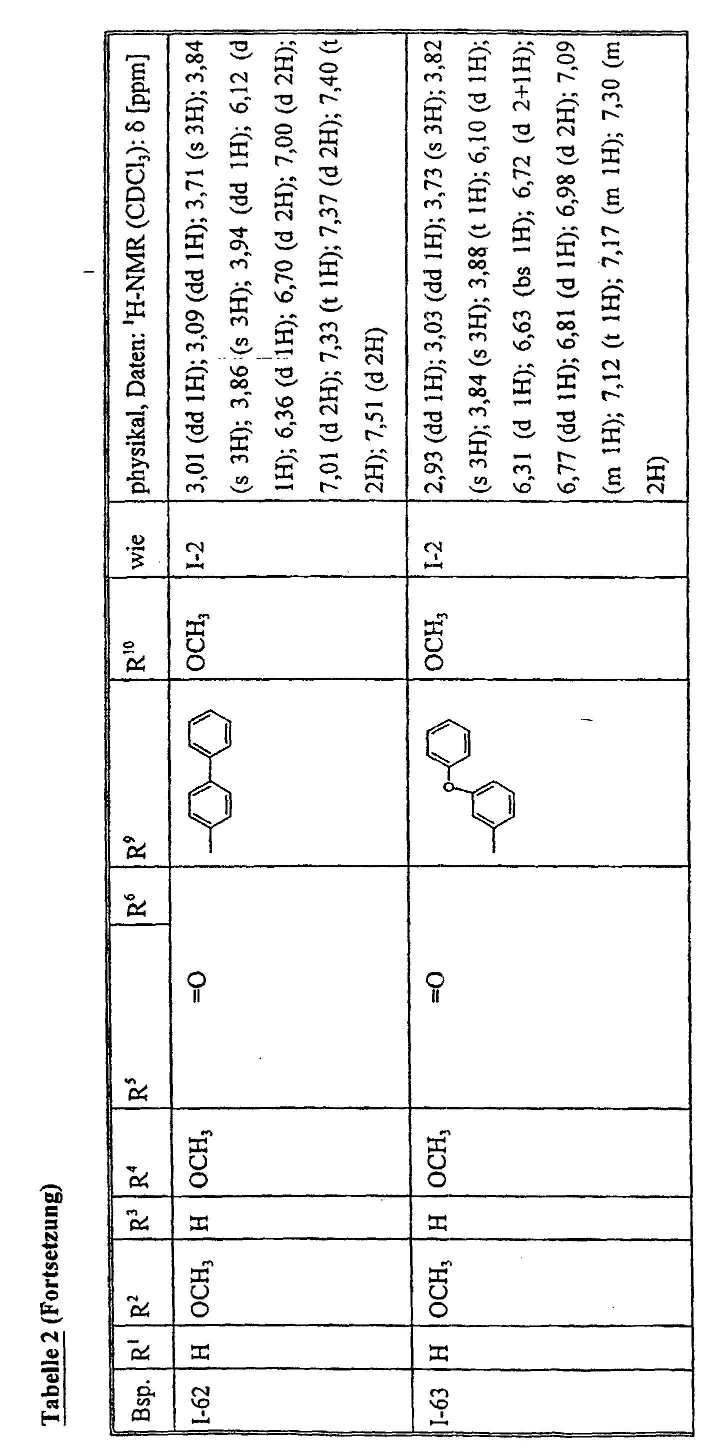

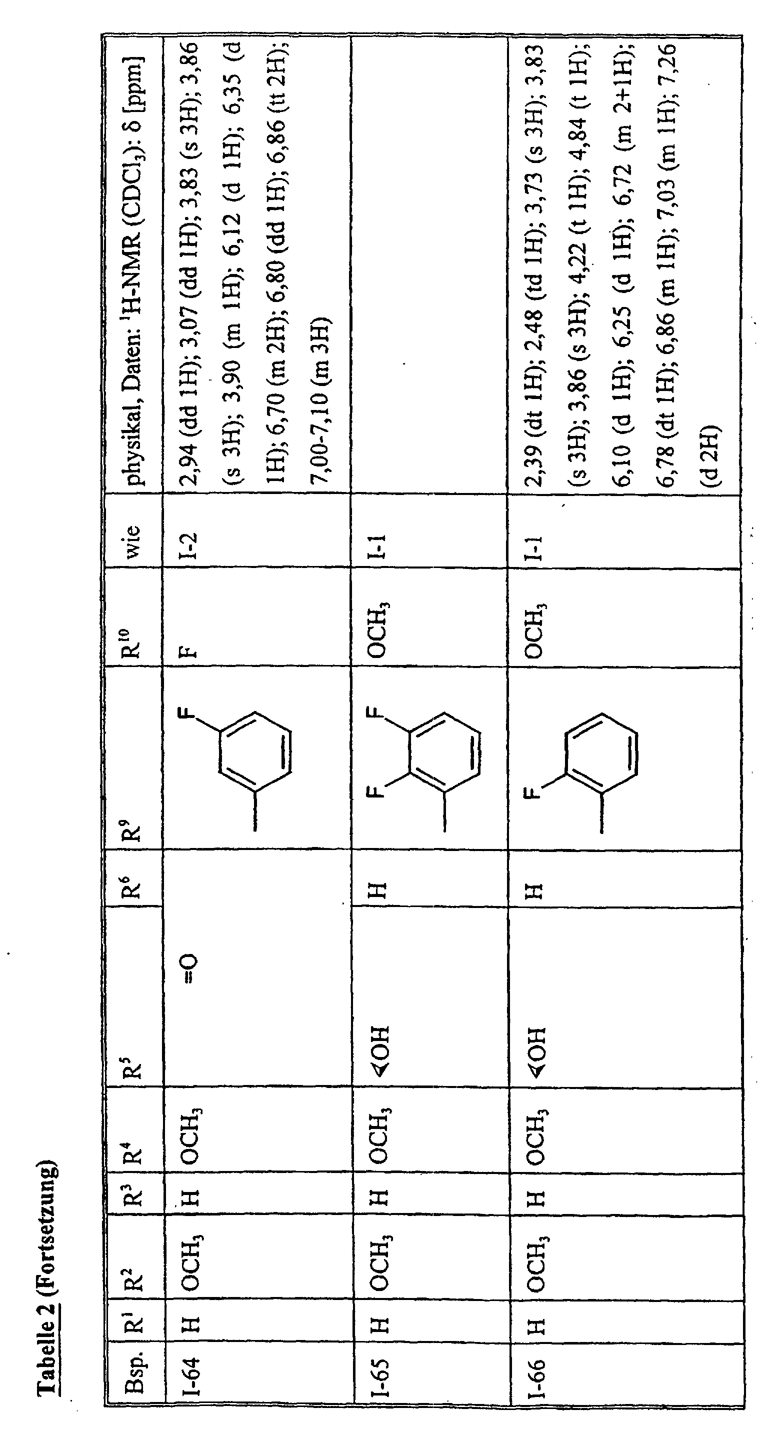

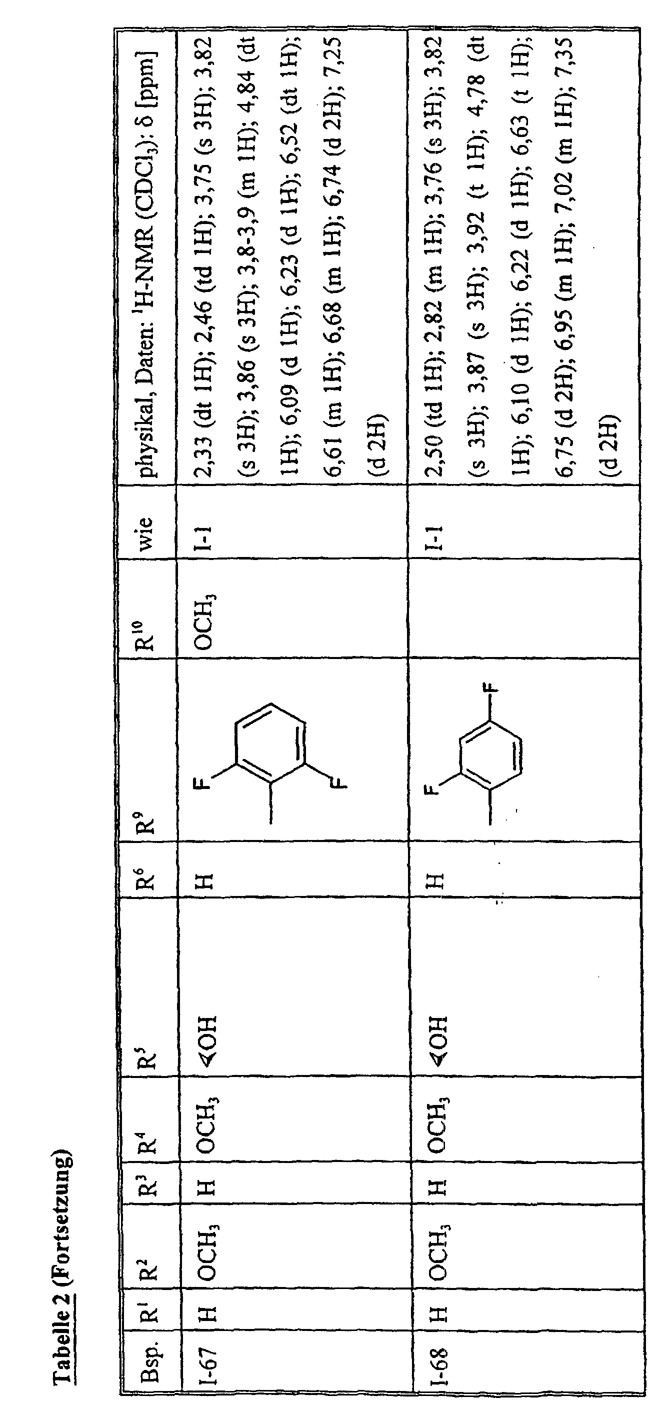

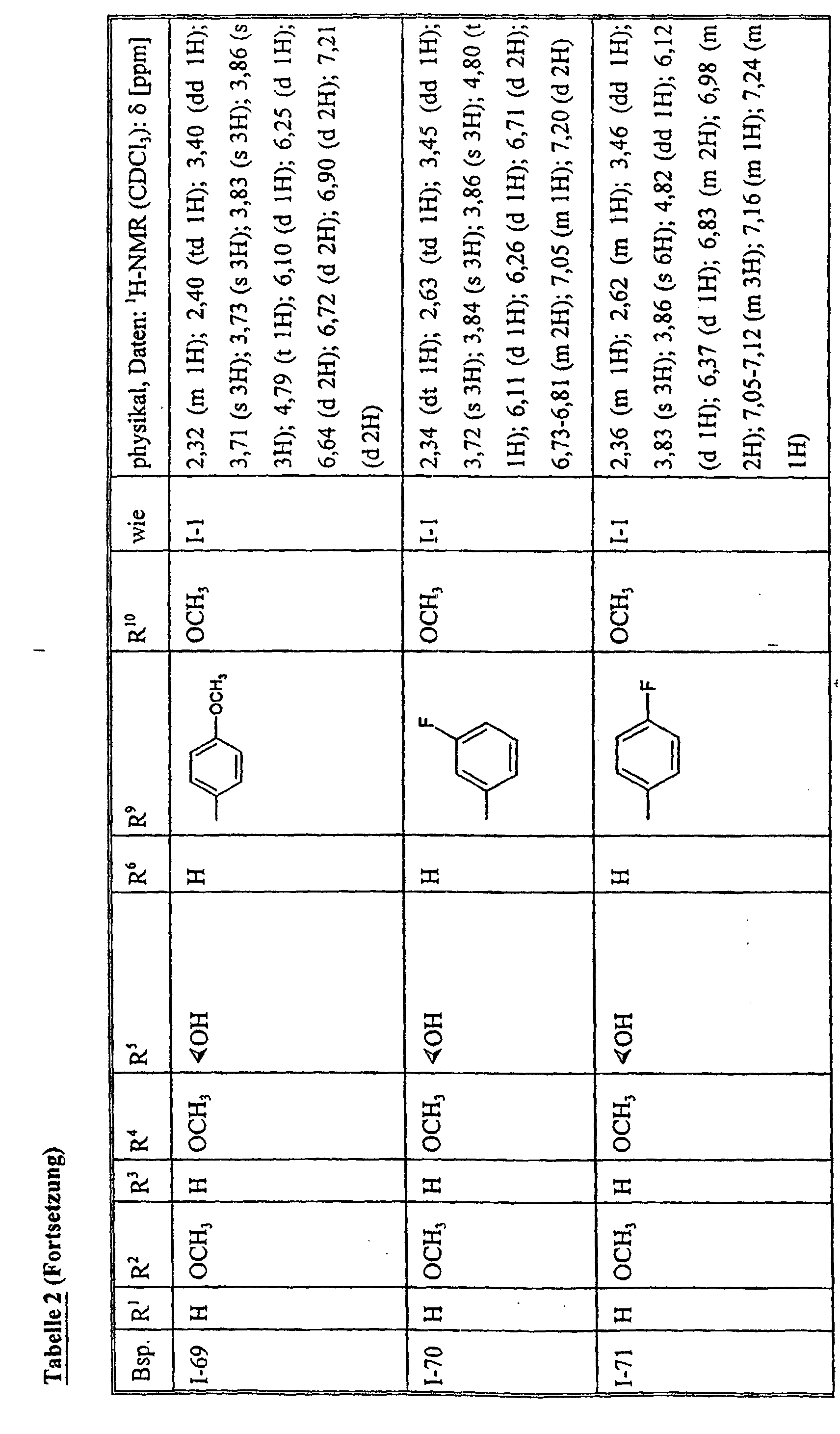

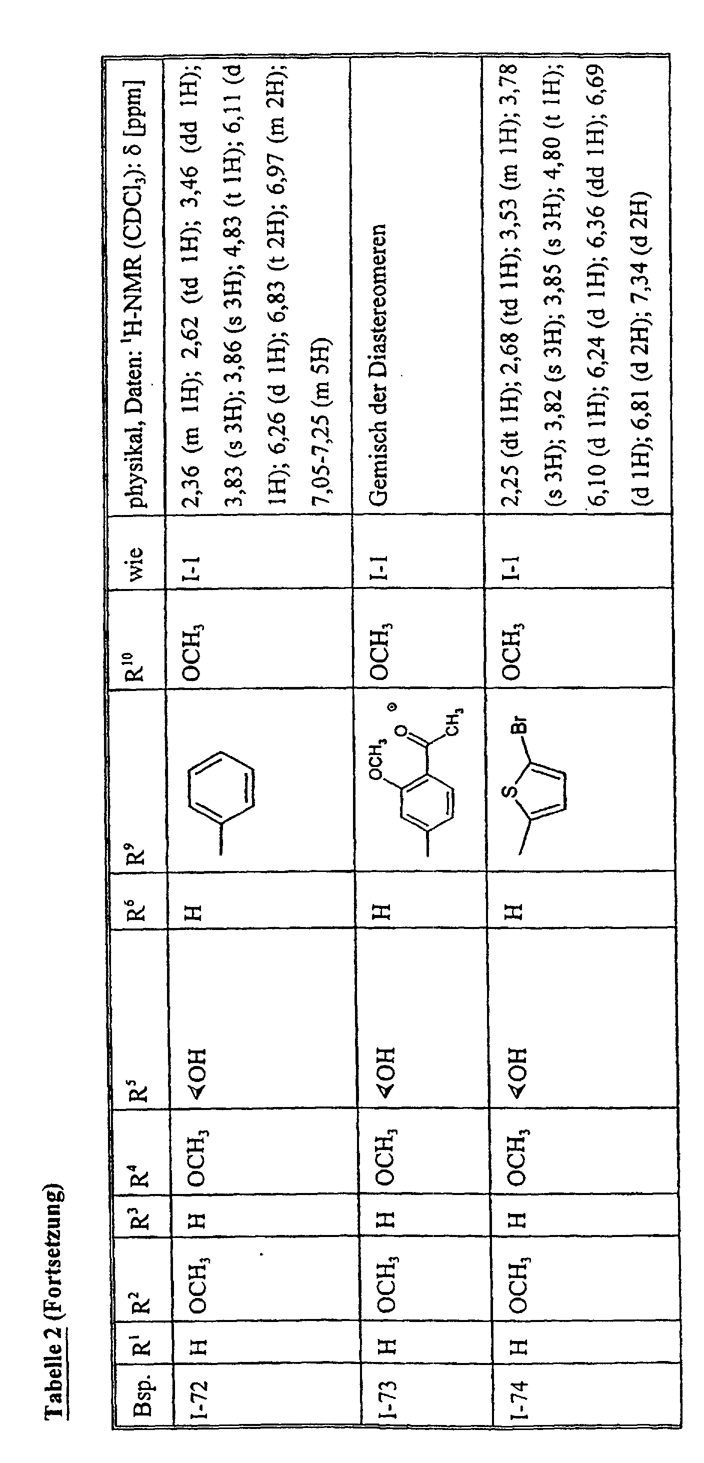

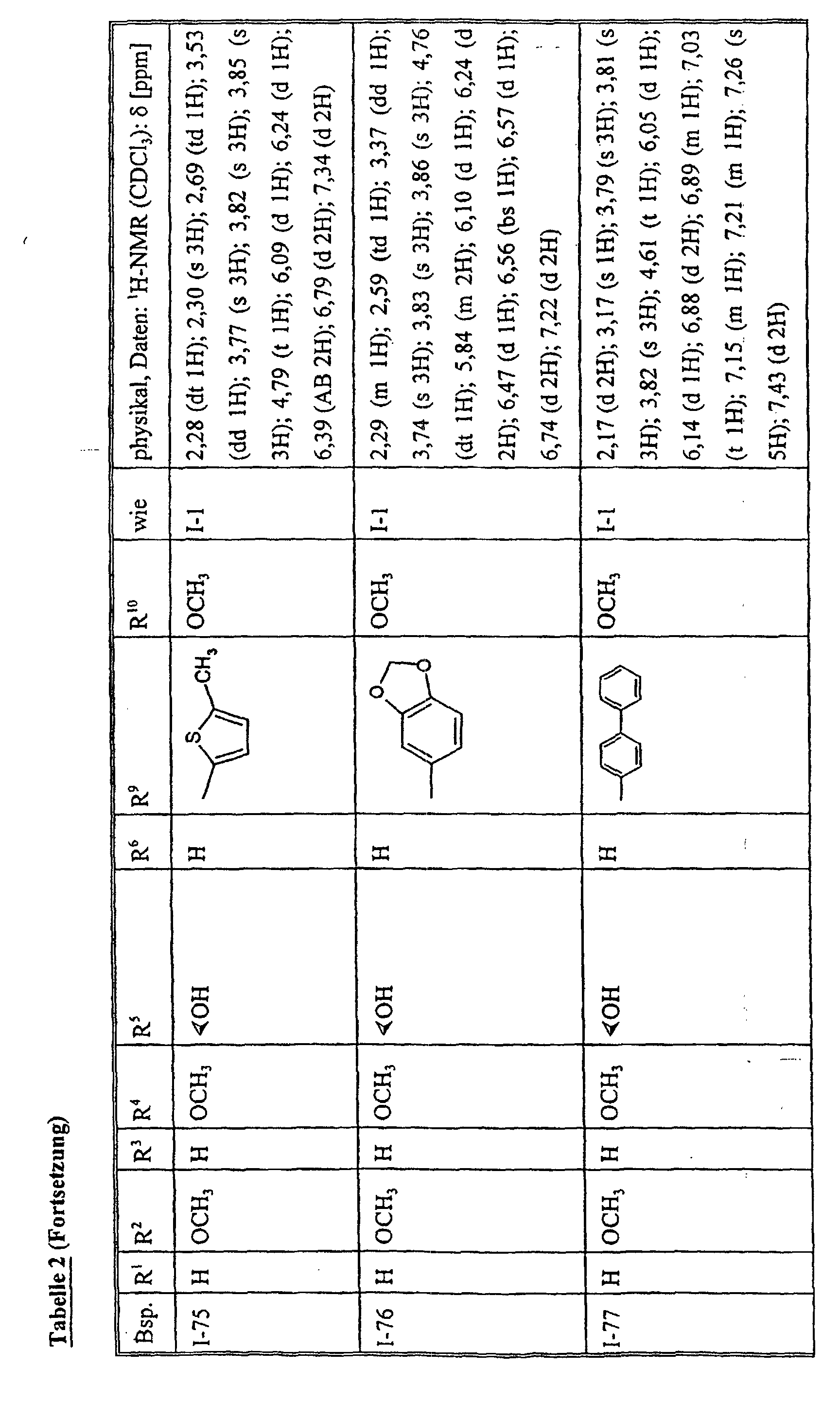

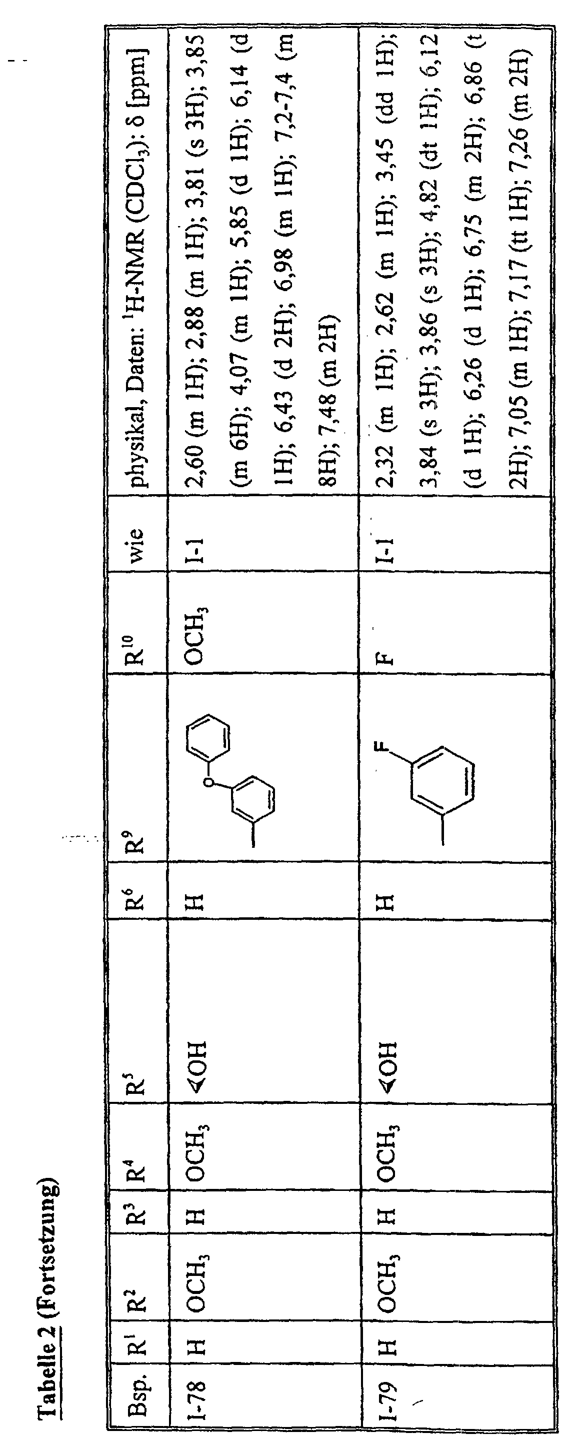

### Herstellung der Vorprodukte

### Beispiel II-1

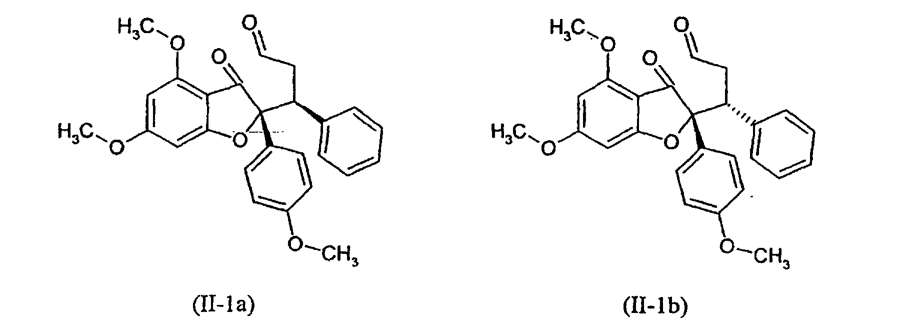

5,0 g (16,5 mmol) 4,6-Dimethoxy-2-(4-methoxyphenyl)-benzofuran-3-on werden in 50 ml tert.-Butanol vorgelegt und auf 60°C erwärmt. Dann werden 1,05 ml Benzyltrimethylammoniumhydroxid-Lösung (Triton ® B 40% in Methanol, 3,0 mmol) und 2,2 ml (17,5 mmol) frisch destillierter E-Zimtaldehyd hinzugeführt, und man läßt 3 h bei 60°C rühren. Anschließend werden 50 ml 1 N HCl addiert, und es wird mit CH₂Cl₂ (3x 50 ml) extrahiert. Nach dem Trocknen über MgSO₄ wird chromatographiert (t-Butylmethylether: Toluol : Cyclohexan = 2 : 1 : 1). Man erhält neben einer Mischfraktion von 1,03 g (2,39 mmol, 14%) 2,16g (5,0 mmol, 30%) racemisches (S*,R*)-3-[4,6-Dimethoxy-2-(4-methoxyphenyl)-3-oxo-2,3-dihydrobenzofuran-2-yl]-3-phenylpropanal (II-la) und 635 mg (1,47 mmol, 9%) racemisches (R*,R*)-Derivat (II-1b).
(II-1a): ¹H-NMR (CDCl₃): δ (ppm) 2,68 (ddd, 1 H), 3,03 (ddd, 1 H), 3,70 (s, 3 H), 3,79 (s, 3 H), 3,85 (s, 3 H), 4,20 (dd, 1 H), 5,80 (d, 1 H), 6,21 (d, 1 H), 6,89 (d, 2 H), 7,06-7,19 (m, 3 H), 7,32 (d, 2 H), 7,66 (d, 2 H), 9,42 (dd, 1 H).
(II-1b): ¹H-NMR (CDCl₃): δ (ppm) 2,66 (ddd, 1 H), 3,00 (ddd, 1 H), 3,70 (s, 3 H), 3,87 (s, 3 H), 3,90 (s, 3 H), 4,13 (dd, 1 H), 5,99 (d, 1 H), 6,31 (d, 1 H), 6,69 (d, 2 H), 7,05-7,19 (m, 5 H), 7,38 (d, 2 H), 9,48 (dd, 1 H).

### Beispiel II-2

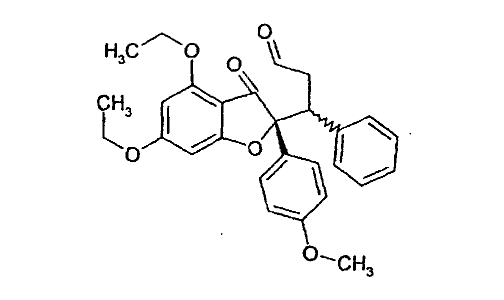

4,17 g (12,7 mmol) 4,6-Diethoxy-2-(4-methoxyphenyl)benzofuran-3-(2H)-on werden in 40ml Methanol vorgelegt und auf 60°C erwärmt. Dann werden 137 mg (2,54 mmol) Natriummethanolat in 4 ml Methanol, 2,1 ml (16,5 mmol) E-Zimtaldehyd und 20 ml Toluol hinzugegeben. Man läßt über Nacht bei 60°C rühren. Dann wird 1 N HCl addiert und es wird mit Dichlormethan extrahiert. Nach dem Trocknen über MgSO₄ wird chromatographiert (t-Butylmethylether : Toluol : Cyclohexan = 1 : 1 : 1). Man erhält 1,31 g einer Mischung von Diastereomeren des 3-[4,6-Diethoxy-2-(4-methoxyphenyl)-3-oxo-2,3-dihydrobenzofuran-2-yl)-3-phenylpropanals.
¹H-NMR (CDCl₃) ausgewählte Signale: δ (ppm) 5,78 (d, 1 H), 6,17 (d, 1 H), 6,89 (d, 2 H), 7,65 (d, 2 H), 9,39 (m, 1 H) und 5,98 (d, 1 H), 6,28 (d, 1 H), 6,68 (d, 2 H), 7,38 (d, 2 H), 9,48 (dd, 1 H).

### Beispiele II-3 bis II-26

Analog zu den Beispielen II-1 und II-2 und den allgemeinen Angaben zur Herstellung wurden die in der nachfolgenden Tabelle 3 aufgeführten Verbindungen der Formel (II-n) erhalten.

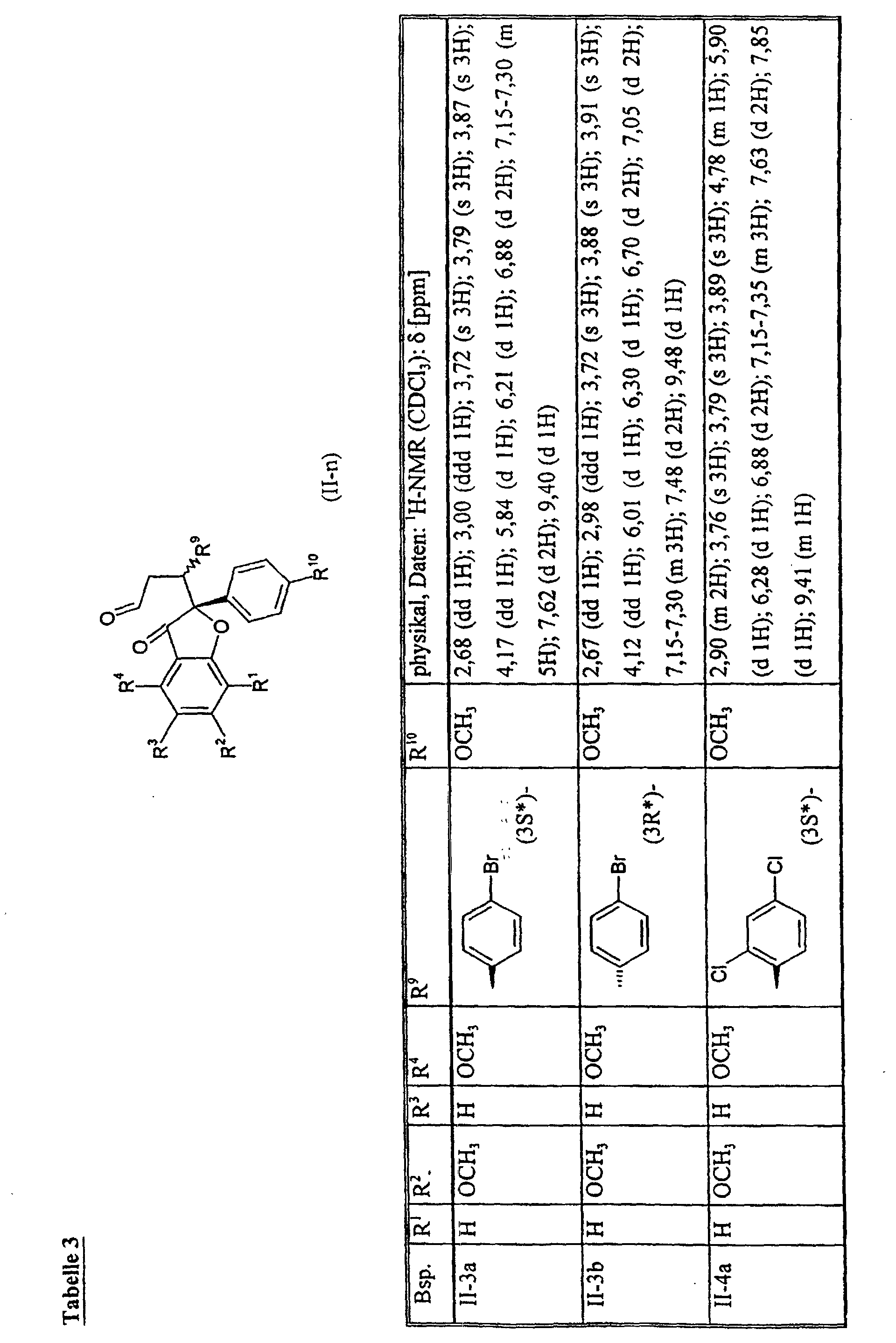

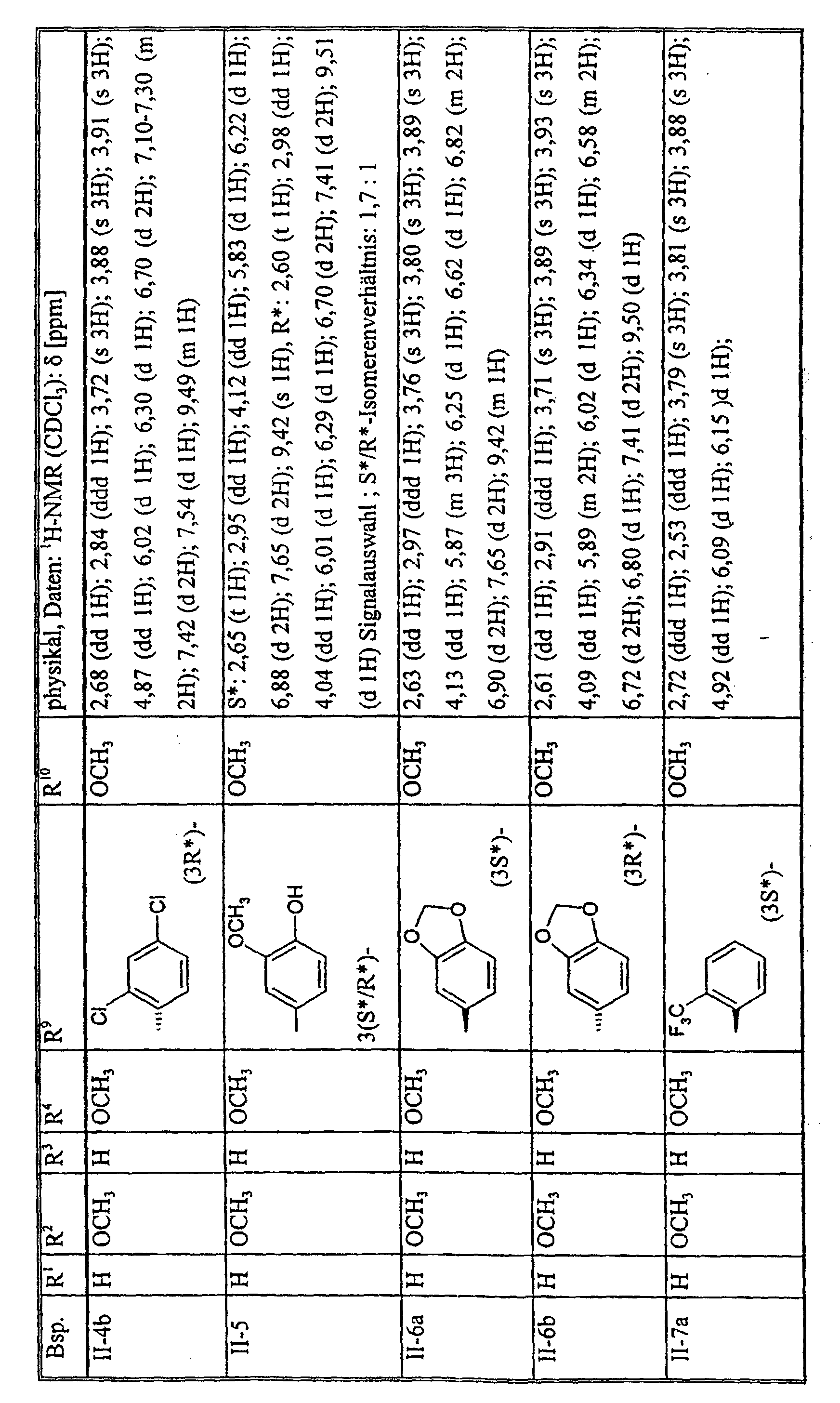

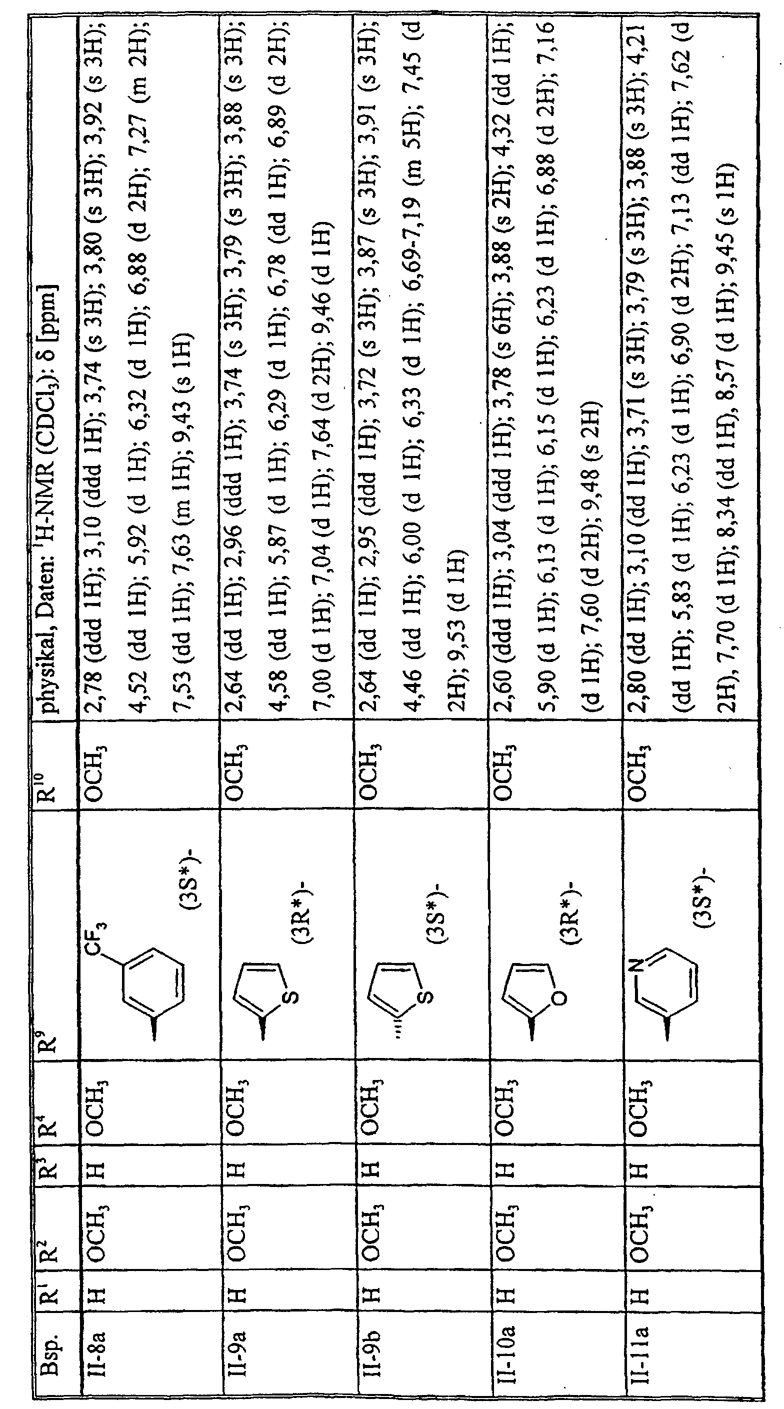

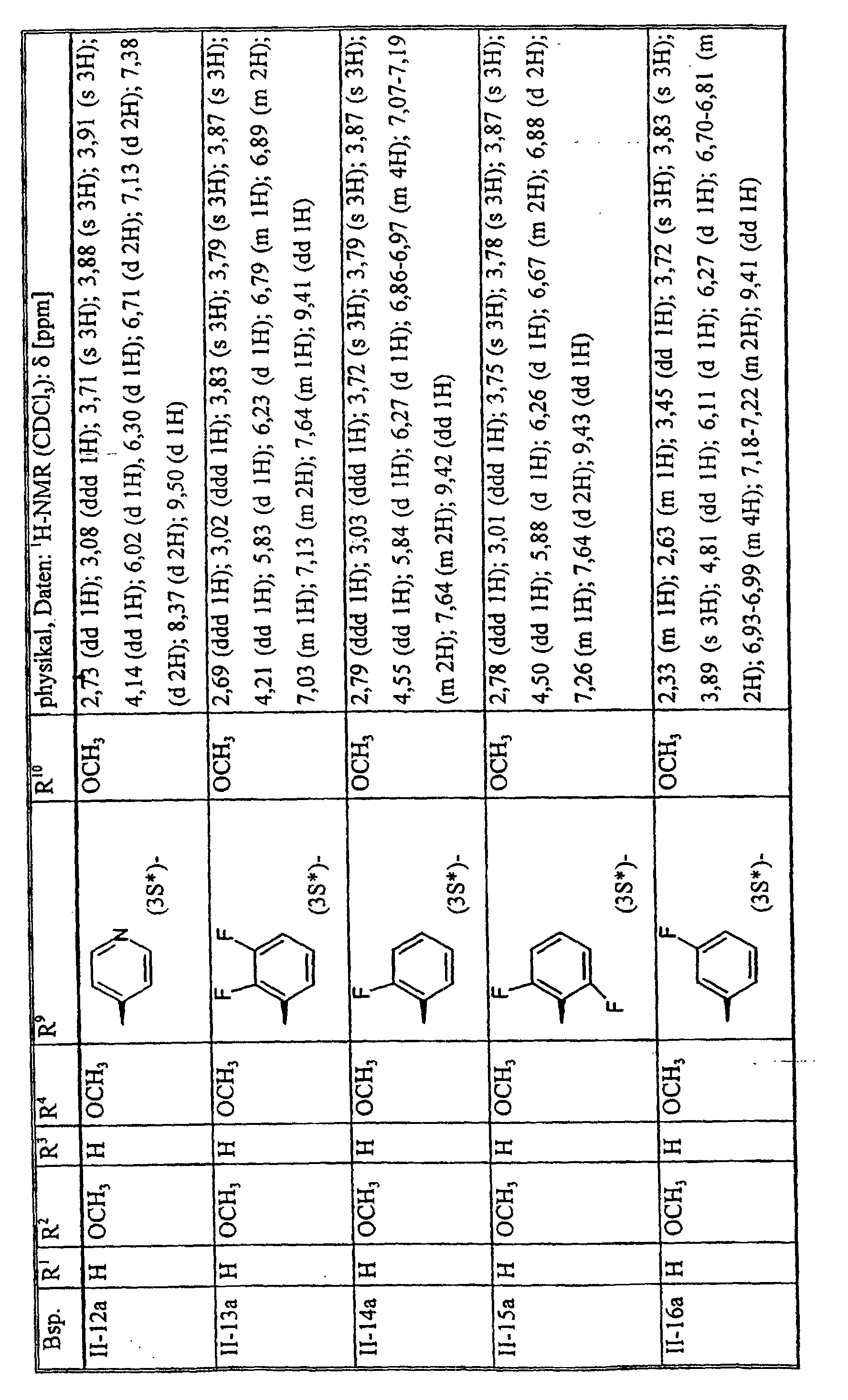

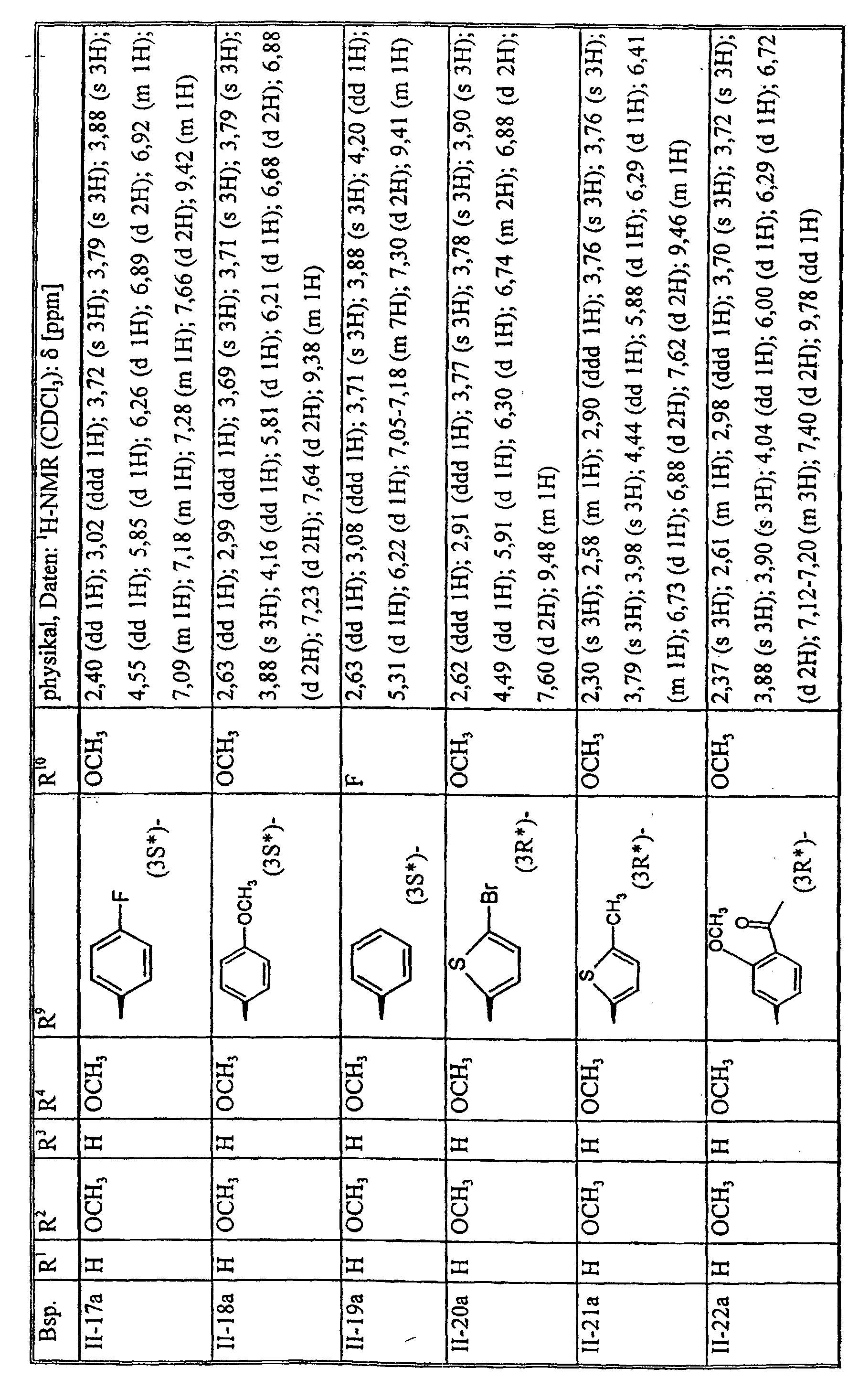

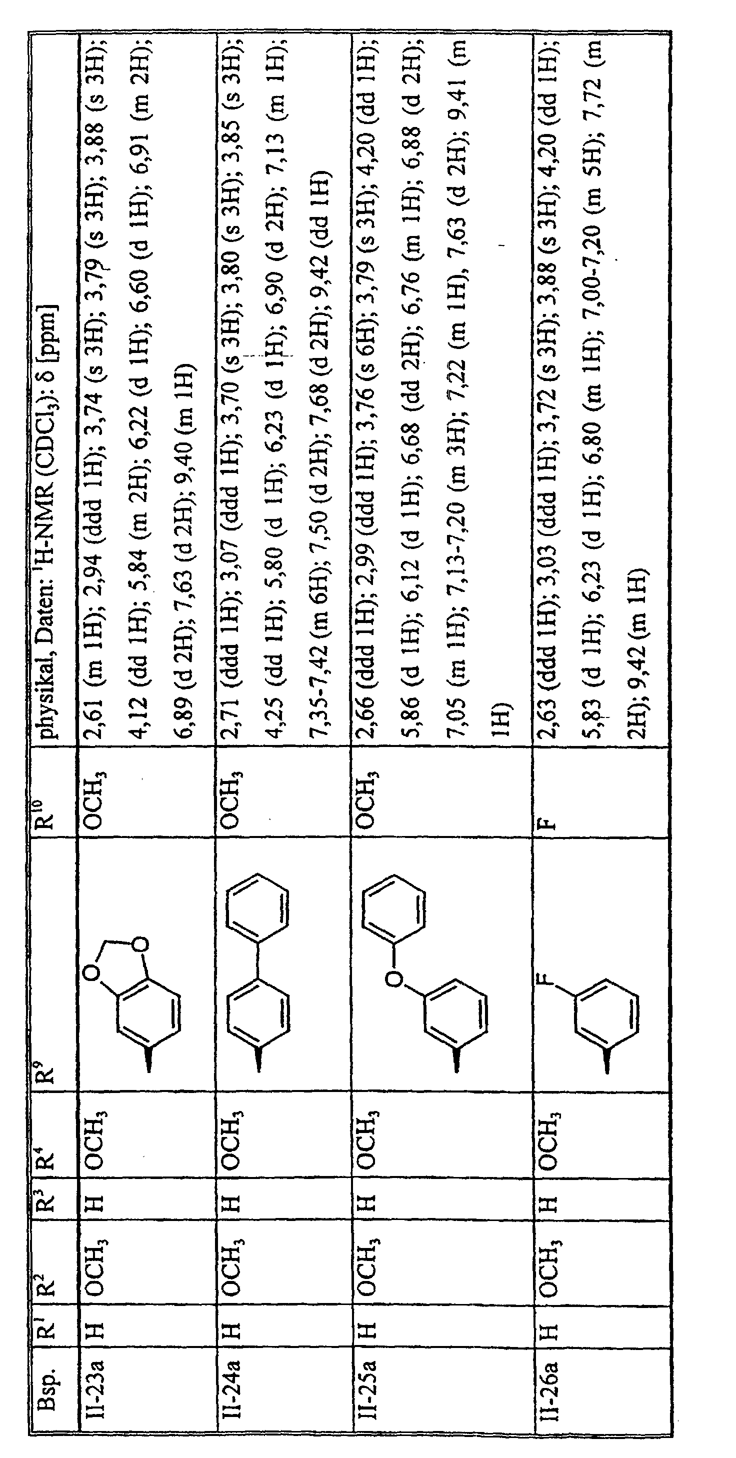

### Beispiel IV-1

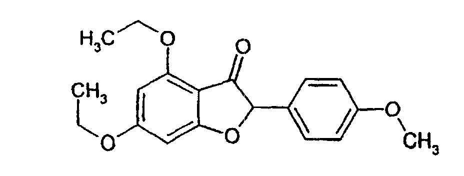

15,62g 3,4,6-Triethoxy-2-(4-methoxyphenyl)benzofuran (z.B. aus Beispiel IV-1') werden in 200 ml Methanol vorgelegt, mit 200 ml 0,5 N HCl versetzt und für 3 h unter Rückfluß erhitzt. Nach dem Abkühlen der Lösung wird der Niederschlag abgesaugt. Ausbeute: 12,69 g 4,6-Diethoxy-2-(4-methoxyphenyl)benzofuran-3-(2H)-on.
¹H-NMR (CDCl₃): δ (ppm) 1,43 (m, 6 H), 3,79 (s, 3 H), 4,13 (m, 4 H), 5,41 (s, 1 H), 6,02 (d, 1 H), 6,19 (d, 1 H), 6,89 (d, 2 H), 7,31 (d, 2 H).

### Beispiel IV-1'

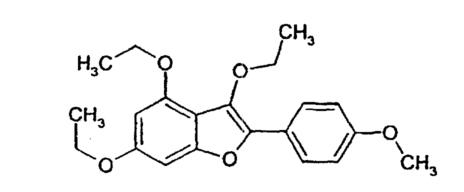

10 g (36,7 mmol) 4,6-Dihydroxy-2-(4-methoxyphenyl)benzofuran-3-(2H)-on werden in 400 ml Aceton gelöst und mit 32,5 g (6,4 mmol) Kaliumcarbonat versetzt. 36,3 g (6,4 mmol) Diethylsulfat werden hinzugegeben und es wird für 2,5 h unter Rückfluß erhitzt. Anschließend werden 50 ml Methanol addiert, es wird für eine weiter Stunde unter Rückfluß erhitzt, abfiltriert und eingeengt. Das Rohprodukt wird über Kieselgel chromatographiert (Essigester : Cyclohexan = 1 : 3). Ausbeute: 15,62 g 3,4,6-Triethoxy-2-(4-methoxyphenyl)benzofuran.
¹H-NMR (CDCl₃): δ (ppm) 1,43 (m, 9 H), 3,85 (s, 3 H), 4,06 (q, 2 H), 4,14 (q, 2 H), 4,21 (q, 2 H), 6,29 (d, 1 H), 6,58 (d, 1 H), 6,96 (d, 2 H), 7,92 (d, 2 H).

### Beispiel V-1

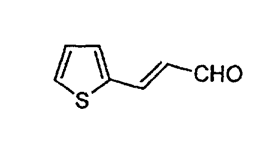

5 g NaOH werden in 60 ml Wasser gelöst und 30 ml Ethanol werden addiert. 22,5 g (239 mmol) Thiophen-2-carbaldehyd werden innerhalb von 15 min. zugetropft und bei 0°C läßt man langsam (1,5 h) 50 g (1,135 mol) einer Acetaldehydlösung (40% in Wasser) zutropfen. Es wird 1 h bei 0°C gerührt, mit eiskalter Essigsäure neutralisiert, mit Dichlormethan extrahiert, mit Wasser gewaschen, über MgSO₄ getrocknet, vorsichtig eingeengt und bei 1 mbar/80-95°C destilliert. Ausbeute: 8,74 g (26% d.Th.) E-3-(2-Thienyl)-propenal.
¹H-NMR (CDCl₃): δ (ppm) 6,52 (dd, 1 H), 7,11 (dd, 1 H), 7,37 (d, 1 H), 7,51 (d, 1 H), 7,59 (d, 1 H), 9,63 (d, 1 H).

### Beispiel V-2

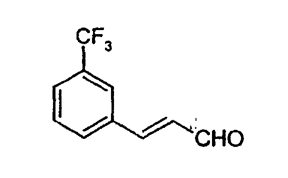

1) Unter Argon werden 12,29 ml IM Diisobutylaluminiumhydrid-Lösung in THF mit 20 ml THF verdünnt vorgelegt und eine Lösung von 1,00 g (4,095 mmol) E-3-Trifluormethylzimtsäureethylester in 20 ml THF zugetropft. Man rührt 30 min nach, hydrolysiert mit Wasser, extrahiert anschließend mit Dichlormethan, trocknet die vereinigten Extrakte und engt ein. Säulenchromatographie an Kieselgel (mobile Phase: Cyclohexan : Ethylacetat = 7 : 3) liefert in der ersten Fraktion 420 mg (50,7% d.Th.) E-3-Trifluormethylzimtalkohol, der weiter zur Oxidation eingesetzt wird.
   ¹H-NMR (CDCl₃): δ (ppm) 1,68 (s, 1H); 4,38 (d, 2H); 6,43 (dt, 1H); 6.66 (d, 1H); 7,42 (dd, 1H); 7,49 (d, 1H); 7,53 (d, 1H); 7,62 (s, 1H).
2) Bei -70°C werden unter Argon 395,5 mg (3,324 mmol) Oxalylchlorid in 2 ml THF vorgelegt, 259,3 mg (3,324 mmol) Dimetylsulfoxid zugetropft und 5 min nachgerührt. Darauf tropft man eine Lösung der 420 mg (2,077 mmol) Alkohol aus der ersten Stufe in 5 ml THF bei max. -70°C hinzu und rührt 30 min nach. Nach Zutropfen von 3,147 g ( 31,16 mmol) Triethylamin bei -70°C werden zunächst 1 h bei dieser Temperatur, dann 16 h bei Raumtemperatur nachgerührt. Es wird mit Phosphatpuffer hydrolysiert, anschließend mit Dichlormethan extrahiert, die vereinigten Extrakte getrocknet und eingeengt. Säulenchromatographie an Kieselgel (mobile Phase: Cyclohexan : Ethylacetat = 8 : 2) liefert in der ersten Fraktion 180 mg (43,3% d.Th.) E-3-Trifluormethylzimtaldehyd.
   ¹H-NMR (CDCl₃): δ (ppm) 6,78 (dd, 1H); 7,53 (dd, 1H); 7,57 (d, 1H); 7,70 (d, 1H); 7,78 (d, 1H); 9,75 (d, 1H).

### Anwendungsbeispiele

### Beispiel A

### Hemmung der NF-κB-vermittelten Genexpression des Tumor Nekrose Faktor-α (TNFα) Gens in menschlichen Monozyten

Der Promotor des humanen TNFα Gens enthält drei NF-κB Bindesequenzen, die als k1, k2 und k3 bezeichnet werden. Die NF-κB Bindesequenzen finden sich im Promotor des TNFα Gens an den Nukleotid-Positionen k1 = -587 bis -577, k2 = -210 bis -202 und k3 = -98 bis -87 und diese DNA Sequenzen binden spezifisch NF-κB (A.E. Goldfield et al., Proc. Natl. Acad. Scri. USA 87, 9769-9773, 1990). Lipopolysaccharid oder Phorbolester (wie z.B. Phorbolmyristatacetat) induzieren die NF-κB Freisetzung (M.J. Lenardo et al., Cell 58, 227-229, 1989).

Daher wird zum Nachweis der Inhibition der NF-κB-vermittelten TNFα Genexpression durch die hier beschriebenen Substanzen folgender biologischer Test durchgeführt.

Mononukleare Zellen aus Spenderblut werden mit Vacutainer CPT(™) Röhrchen (Becton Dickinson and Company, Franklin Lakes, NJ. 07417-1885) isoliert, entsprechend den Vorschriften des Herstellers. Die Vacutainer CPT Röhrchen enthalten 1,0 ml phosphatgepufferte Saline mit 120 USP Einheiten Natriumheparin über 3,0 g eines Polyestergels, das 2,0 ml einer Ficoll-Lösung überschichtet. Nach der Zentrifugation des Spenderbluts werden die Monozyten aus einer Zone oberhalb des Polyestergels genommen und mit einer Zelldichte von 250 x 10⁵ Zellen pro well ind 96-well Mikrotiterplatten für die Zellkultur ausgesät.

Die Zellen werden 4 bis 6 Stunden in Medium RPMI-1640 (Gibco BRL, Life Technologies GmbH, Dieselstr. 5, 76344 Eggenstein) inkubiert. Anschließend wird der Kulturüberstand abgesaugt, es wird erneut Medium RPMI-1640 hinzugefügt und die zu testenden Substanzen werden in Konzentrationen üblicherweise zwischen 0 µM (Negativ-Kontrolle) und 20 µM zugesetzt. Direkt anschließend wird bakterielles Lipopolysaccharid (LPS), Fa. Sigma-Aldrich Chemie GmbH, Grünwalder Weg 30, 82039 Deisenhofen, Best.-Nr.: L 4391, in einer Konzentration von 125 ng/ml zur Stimulierung der NF-κB-vermittelten TNFα Genexpression zugegeben. Nach einer weiteren Inkubation von 18 Stunden bei 37°C in 5% CO₂ Atmosphäre wird aus den Mikrotiterplatten Kulturüberstand entnommen und darin der TNFα Gehalt quantitativ bestimmt mit kommerziell verfügbaren enzymgebundenen Immunosorbentassays (ELISA).

TNFα ELISA zur Konzentrationsbestimmung werden z.B. von Fa. Sigma-Aldrich Chemie GmbH, Grünwalder Weg 30, 82039 Deisenhofen unter der Bezeichnung Human TNFα ELISA Kit, Best.-Nr.: CKH-200A, vertrieben. Entsprechend der Gebrauchsanweisung des Herstellers wird die Konzentration des gebildeten TNFα im Kulturüberstand der Monozytenkultur nach LPS Stimulation mit und ohne Inhibitorsubstanz quantitativ bestimmt.

Die Ergebnisse der TNFα-Konzentrationsbestimmung werden in einem x-y-Achsendiagramm gegeneinander aufgetragen. Der Graph der y-Koordinaten (TNFα-Konzentration im Kulturüberstand) und der x-Koordinaten (Konzentration des eingesetzten Inhibitors) erlaubt es, die Hemmung der NF-κB-vermittelten TNFα Synthese in Abhängigkeit von der Konzentration der Inhibitorsubstanz abzulesen. Auf diese Weise kann diejenige Wirkstoffkonzentration des zugesetzten Inhibitors aus dem Diagramm abgelesen werden, die z.B. die TNFα Synthese um 50 % hemmt. Diese Wirkstoffkonzentration, die eine 50%-ige Hemmung hervorruft, wird effektive Hemmstoffkonzentration für 50 %-Hemmung (IC₅₀) genannt.

Dabei zeigten beispielsweise die folgenden Verbindungen, daß sie potente Inhibitoren der NF-κB-vermittelten TNFα Synthese darstellen:

| Wirkstoff aus Herstellbeispiel Nr. | IC₅₀ |
|---|---|
| I-1 | 0,5 |
| I-3-R | 0,1 |
| I-5 | 0,5 |

### Beispiel B

### Hemmung der NF-κB-vermittelten Genexpression des menschlichen Tissue-Factor Gens in Monozyten

Tissue Factor ist ein membranständiges Protein, das den primären Initiator der Blut-Koagulationskaskade darstellt und eine Schlüsselfunktion bei Herzkreislauferkrankungen wie unstabiler Angina pectoris, akuten Implikationen nach Plaque-Ruptur, Gefäßocclusionen verschiedener Ätiologie, arteriosklerotischen Prozessen und anderen Krankheiten wie septischem Schock oder Krebs einnimmt. Das Tissue Factor Gen wird insbesondere in Monozyten und Endothelzellen durch NF-κB-Aktivierung induziert. Der Promotor des humanen Tissue Factor Gens enthält eine NF-κB Bindestelle, die entscheidend zur Aktivierung des Promotors beiträgt (P. Oeth et al. Arteriosclerosis, Thrombosis, and Vascular Biology 17, 365-374, 1997).

Daher wurde zum weiteren Nachweis der Hemmung der NF-κB-vermittelten Genexpression durch die erfindungsgemäßen Inhibitoren folgender biologisch Test durchgeführt:

Das Promotor-Fragment des humanen Tissue Factor Gens, das die NF-κB Bindesequenz enthält, wurde mit Oligonukleotid-Primern der Sequenz 5'-TCC CTC GAG ATC TCC CAG AGG CAA ACT GCC AGA T-3' (5'-Primer der Position -925) und 5'-TCC TCG AGC CAT GGC TAC CAG TTG GGC GGC GAG ATC-3' (3'-Primer enthaltend das ATG-Startcodon der kodierenden Sequenz des Tissue-Factor Gens) durch die Polymerase-Kettenreaktion (PCR) kloniert und durch eine NcoI-/ XhoI-Klonierung mit dem Luciferase Startcodon im Plasmid pGL3-Basic Vector (Promega Corp. 2800 Woods Hollow Road, Madison, WI 53711-5399 USA) fusioniert. Dadurch wird die Expression der Luciferase im so entstandenen rekombinanten Plasmid durch den humanen Tissue Factor Promoter reguliert. Dieses Expressionskonstrukt wurde zur Analyse der DNA-Sequenzierung unterworfen und in die Monozytenzellinie RAW 264.7 (American Type Culture Collection 12301 Parklane Drive, Rockville, Maryland 20852, USA) transfiziert. Die Transfektion, Selektion und Klon-Analyse erfolgte nach Standardmethoden, wie sie beschrieben sind (siehe Transfection of Mammalian Cells in Culture, L.G. Davis et al. Basic Methods in Molecular Biology, Elsevier Sci. Publishing Co., New York 1986). Nach der Selektion von RAW-Klonen, die das Expressionskonstrukt stabil im Genom integriert hatten, wurde eine dieser Transfektanten, genannt RAW-A3, für den Test der Inhibitoren ausgewählt.

### Testdurchführung:

In 12-well Mikrotiterplatten werden in jede Vertiefung 106 RAW-A3 Zellen ausgesät. Schrittweise wird die Serum-Konzentration in drei Tagen im RPMI Medium von 10% fötalem Kälberserum auf 0,5 % und 0,1 % Serum-Anteil abgesenkt, um die serum-abhängige Tissue Factor Promotor Aktivierung auf ein Minimum zu senken. Nach 24 Stunden Kultur in Medium mit 0,1 % Serum wird der NF-κB Inhibitor zugesetzt und anschließend Serum bis zu einer Konzentration von 15 % im Medium zur Promotor-Induktion zugesetzt.

Nach weiteren 6 Stunden wird der Kulturüberstand abgesaugt und die Zellen werden zur Messung der Luciferase-Aktivität gemäß der Vorschrift des "Luciferase Assay System" (Technical Bulletin der Fa. Promega, 2800 Woods Hollow Road, Madison, WI 53711-5399 USA, Produkte E4030, E1483, E1501) verarbeitet. Das Zelllysat wird mit dem Luciferase Assay Substrat inkubiert und in einem Luminometer zur Messung des emittierten Lichts vermessen. Bei einer Auftragung in einem x-y-Achsendiagramm mit dem jeweils emittierten Licht (angegeben in relative light units) als y-Koordinate und den Inhibitorkonzentrationen als x-Koordinaten ergibt sich ein Graph für f(x), bei dem der halbmaximale y-Wert einer Inhibitorkonzentration x zuzuordnen ist, die der Hemmkonzentration IC₅₀ in diesem Test entspricht.

Zum Beispiel beträgt der IC₅₀-Wert der Verbindung gemäß Herstellbeispiel I-1 20 µM und I-5 2 µM. Die NF-κB-vermittelte Expression der Luciferase durch die erfindungsgemäßen Inhibitoren mit einer Konzentration im unteren mikromolaren oder im submikromolaren Bereich weist auf die hohe Wirksamkeit der Hemmung einer NF-κB-vermittelten Genexpression hin.

### Beispiel C

### Hemmung der NF-κB-vermittelten Genexpression des Tumor Nekrose Faktor-α (TNFα) Gens in menschlichen Monozyten in Abhängigkeit von drei verschiedenen TNFα Synthesestimuli

Die NF-κB-vermittelte Induktion der TNFα-Synthese ist unabhängig von den unterschiedlichen Stimuli, die durch LPS, opsonisiertem Zymosan oder Phorbolmyristatacetat (PMA) für die Aktivierung des TNFα Promotors eingesetzt werden (A. Baldwin, Annual Rev. Immunology 14, 649, 1996). Daher sollte der Einsatz der erfindungsgemäßen Inhibitoren auch zu einer vergleichbar starken Hemmung der TNFα Synthese führen unabhängig von der Art der Stimulierung der TNFα Synthese.

Die Tests für diesen Nachweis der stimulusunabhängigen Hemmung der TNFα Synthese erfolgten von der Art der Testdurchführung genauso wie es unter Beispiel A beschrieben ist mit dem Unterschied, daß neben LPS als Stimulus auch Zellkulturansätze mit 100 nM PMA oder mit 100 µg/ml opsonisiertem Zymosan stimuliert wurden. Zymosan und Phorbolmyristatacetat (PMA) können von der Firma Sigma, Grünwalder Weg 30, 82041 Deisenhofen, Deutschland unter den Bestell-Nm. Z4250 und P8139 bezogen werden. Zymosan wird in humanem Serum opsonisiert.

Die erfindungsgemäßen NF-κB-Inhibitoren hemmen unabhängig vom Stimulus mit IC₅₀ Werten im submikromolaren Bereich in vergleichbarer Weise die TNFα Synthese in humanen Monozyten wie es im Beispiel A gezeigt wurde. So ist für die Verbindung gemäß Herstellbeispiel I-1 der IC50-Wert nach PMA-Stimulus 0,10 µM und für Verbindung 1-5 0,09 µM.

### Beispiel D

### Hemmung der NF-κB vermittelten Genexpression des Adhäsionsproteins ELAM-1 an humanen Nabelschnurendothelzellen (HUVEC)

Die Rekrutierung von Leukozyten aus der Blutzirkulation in den extravaskulären Raum ist essentiell bei inflammatorischen Antworten und bei der Reparierung von Gewebeschäden. Der Prozess der Leukozyteneinwandeerung beinhaltet mehrere hintereinander geschaltete Schritte. Die initiale Interaktion zwischen Leukozyten und dem Endothel der Blutgefäße wird durch TNF und II-1 abhängige Expression des Adhäsionsproteins ELAM-1 am Endothel vermittelt. Sie vermittelt das sogenannte Rolling der Leukozyten entlang der Blutgefäßwand. Die transskriptionale Regulation der ELAM-1 Expression ist abhängig sowohl von der Nuclear Faktor-κB (NF-κB) Aktivierung und Bindung am ELAM-1 Promoter als auch von der "AP-1 binding site [MA. Read et., Biol. chem. Vol. 272, 2753-2761, (1997)].

Der Einfluß von Verbindung I-3-R auf die Expression von ELAM-1 wurde in zwei unterschiedlichen Testansätzen überprüft. Es wurde in einem fünktionellen Ansatz die Adhäsion von humanen Neutrophilen an TNF-α stimulierten HUVEC-Zellen gemessen. Die Expression von ELAM-1 an der Oberfläche der HUVECs wurde mit einem fluoreszenz-markierten ELAM-1 spezifischen monoklonalen Antikörper mittels FACS-(Cell Sorting) Analyse bestimmt.

### Versuchsdurchführung: ELAM- abhängige Neutrophilen-Adhäsion an Endothelzellen

Die Neutrophilen wurden aus menschlichem Blut (100 ml) isoliert. Dazu wurde in einem Zentrifugenbecher 3,5 ml Polyprep vorgelegt und mit 5 ml Blut vorsichtig überschichtet. Nach 30 Minuten Zentrifugieren bei 2 100 min⁻¹ wurde die Neutrophilenbande in der Mitte des Zentrifugenbechers abgesaugt. Nach 1:2 Verdünnungen in Endothelial Cell Basal Medium (EBM) Fa. Clonetics wurde wiederum 20 Minuten bei 1 000 min⁻¹ zentrifugiert und anschließend zu einer Zellkonzentration von 10⁶ Zellen/ml aufgenommen.

Die Nabelschnur-Endothezellen wurden in 96 Well-Mikrotiterplatten in EBM + 10 % FCS zur Konfluenz angezogen. Bei Versuchsbeginn wurde das Medium gegen EBM ohne FCS ausgetauscht und anschließend die Versuchssubstanzen eingesetzt Nach 20 Minuten wurden die HUVECs mit 10 nM TNF-α stimuliert. Nach 4 Stunden Inkubation wurde gegen 200 µl/Well der Neutrophilensuspension ausgetauscht. Die Neutrophilen wurden vorher 20 Minuten mit einer 25 µM BCECP-Fluoreszenzfarbstofflösung markiert. Nach 30 Minuten Inkubation wurde die BCECP-Neutrophilen-Lösung mit überschüssigen Neutrophilen abgezogen und gegen 200 µl/Well 0,5 %iger NaOH-Lösung ausgetauscht. Die Fluoreszenz der anhaftenden Neutrophilen wurde anschließend im Fluoreszenzphotometer gemessen.

Sowohl die Adhäsion der Neutrophilen an TNF-α stimulierten HUVECs, als auch die Expression von ELAM-1 an der Zelloberfläche konnte durch die Verbindung zu 50 % inhibiert werden. Die maximale Inhibition der Zelladhäsion wurde mit 50 nM für Verbindung I-3-R bestimmt. Die maximale 50 %ige Hemmung der ELAM-1-Expression steht in guter Übereinstimmung mit der gleichzeitig NF-κB und AP-1 abhängigen Regulation des ELAM-1-Promotors.

### Versuchsdurchführung Quantitative Messung der TNF-induzierten Expression von ELAM-1 in HUVECs

Nabelschnur-Endothelzellen (HUVEC) wurden wie oben beschrieben kultiviert und in Gegenwart oder Abwesenheit von 10 ng/ml TNF in Gegenwart oder Abwesenheit von Testsubstanzen für 4 Stunden inkubiert. Die Zellen wurden aus den Mikrotiterplatten durch Inkubation mit 5 mM EDTA in PBS herausgelöst, für 5 Minuten bei 1 000 min⁻¹ zentrifugiert, und in 100 µl PBS plus 1 % Rinderserum Albumin (BSA, Fa. Sigma-Aldrich GmbH, Best. Nr. A 7906) und einem Anti-ELAM 1 Antikörper (monoklonaler Antikörper, Fa. Becton and Dickinson, Erembodegem, Belgien, Best. Nr. 550023, 30 µg/ml) bei RT inkubiert. Nach 15 Minuten wurden die Zellen erneut zentrifugiert, der Überstand wurde verworfen und die Zellen wurden in PBS plus 1 % BSA gewaschen. Nach erneuter Zentrifugation wurden das erhaltene Zellpellet in PBS plus 1 % BSA und einem Ziege-anti-Maus Antikörper (Fa. Dianova, Raboisen 5, Hamburg; Bestell Nr. 115-096-062; 30 µg/ml) für die Dauer von 15 Minuten inkubiert. Nach erneutem Zentrifugieren und Waschen wurden die Zellen in I ml PBS plus 1 % BSA aufgenommen und in einem Flow-Cytometer (Fa. Becton and Dickinson) bei 488 nm vermessen. Gemessen wird mit diesem Verfahren die Intensität der Fluoreszenz in Abhängigkeit von gebundenem anti-ELAM-1-Antikörper pro Zelle. Für jeden Wert wurden 5 000 Zellen vermessen.

HUVECs, die mit TNF für 4 Stunden stimuliert wurden, zeigten nachh Inkubation mit anti-ELAM-1-Antikörpern deutlich stärkere Fluoreszenz-Signale als Zellen, die demgegenüber nicht mit TNF inkubiert wurden. Die Verbindung aus Herstellbeispiel I-3-R vermochte diese induzierte Expression von ELAM-1 in einem Konzentrationsbereich zwischen 0,05 µM und 5 µM signifikant zu inhibieren, die Expression von ELAM 1 wurde allerdings bei keiner der untersuchten Konzentrationen vollständig inhibiert.

### Beispiel E

### Hemmung der Interleukin-2-Synthese

Zur Testung der Hemmwirkung von Cyclopentabenzol-Derivaten auf die Interleukin-2-Synthese wurde eine Reportergenzellinie benutzt, die den Interleukin-2-Promotor gekoppelt an das Luciferasegen enthält. Der Promotor enthält die DNA-Sequenz von -480 bis +4. Der eingesetzte Vektor ist pGL3; die Ausgangszellinie, in die das gesamte Kontrukt stabil transfiziert wurde, ist SS-1. Das Kulturmedium für diese Zellinie war RPMI 1640 (Gibco, Rockille). Es enthielt zusätzlich: 100 µg/ml Streptomycin, 100 U/ml Penicillin, 2 mM L-Glutamin, 10 % Hitze-inaktiviertes FBS und 800 µg/ml G418 Sulfat.

### Testdurchführung

Die Reportergen-Testzellinie wurde in Phenolrot-freies RPMI mit den Zusätzen zu 1 x 10⁶ Zellen pro Well in 96-Well-Platten ausgesäht und mit Phorbol 12-myristat 13-Acetat (PMA; 5 ng/ml) und Ionomycin (10 M; 0,4 µg/ml) für 24 Stunden bei 37°C in einer Atmosphäre von 5 % CO₂, 95 % Luft inkubiert. Die Testsubstanzen wurden gleichzeitig mit PMA zugesetzt.

### Messung der Luciferaseaktivität:

Zur Generierung der Lumineszenz wurde LucLite™ Lösung (Packard, Meriden, CT) zu einer Konzentration von 100 µl/Well zugesetzt und sofort nach Zugabe die Lumineszenz im Luminometer (Luminoskan, Labsystems) gemessen.

Die Verbindung aus Herstellungsbeispiel I-3-R vermochte die Induktion der Luciferaseaktivität bei einer Konzentration von 5 bis 10 nM zu 50 % (IC₅₀) zu hemmen.

## Patentansprüche

1. Verwendung von Cyclopentabenzofuran-Derivaten der Formel (I) 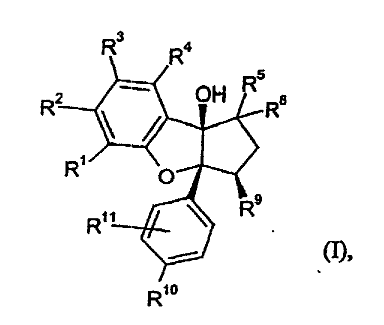 in welcher
R¹ und R³ jeweils für Wasserstoff, Fluor, Chlor, Brom, Methyl oder Ethyl stehen,
R² und R⁴ jeweils für Methoxy, Ethoxy, Trifluormethoxy, Difluormethoxy, Chlordifluormethoxy, 2-Chlor-1,1,2-trifluorethoxy, 1,1,2-Trifluorethoxy, 1,1,2,2-Tetrafluorethoxy, 2,2,2-Trichlor-1,1-difluormethoxy oder 1,1-Difluorethoxy stehen,
R⁵ für Hydroxy, Methylamino, Ethylamino, n-Propylamino, Isopropylamino, n-Butylamino, Isobutylamino, sec.-Butylamino, tert.-Butylamino oder für den Rest -NR¹²-CHR¹³-COOR¹⁴ steht,
R⁶ für Wasserstoff steht, oder
R³ und R⁶ gemeinsam für Sauerstoff (Oxo) stehen,
R⁹ für Phenyl, Methylendioxyphenyl, Furyl, Thienyl, Pyrrolyl, Oxazolyl, Isoxazolyl, Thiazolyl oder Pyridyl steht, die jeweils gegebenenfalls einfach oder zweifach substituiert sein können durch Substituenten der Gruppe Fluor, Chlor, Brom, Iod, Methyl, Ethyl, n-Propyl, Isopropyl, n-Butyl, Isobutyl, sec.-Butyl, tert.-Butyl, Trifluormethyl, Difluormethyl, Chlordifluormethyl, 2-Chlor-1,1,2-trifluorethyl, 1,1,2-Trifluorethyl, 1,1,2,2-Tetrafluorethyl, 1,1-Difluor-2,2,2-trichlorethyl, 2,2,2-Trifluorethyl, 1,1,2,3,3,3-Hexafluorpropyl, 2,2,3,3-Tetrafluorpropyl, 2,2,3,3,3-Pentafluorpropyl, Hydroxy, Methoxy, Ethoxy, n-Propoxy, Isopropoxy, n-Butoxy, Isobutoxy, sec.-Butoxy, tert.-Butoxy, Trifluormethoxy, Difluormethoxy, Chlordifluormethoxy, 2-Chlor-1,1,2-trifluorethoxy, 1,1,2-Trifluorethoxy, 1,1,2,2-Tetrafluorethoxy, 2,2,2-Trichlor-1,1-difluormethoxy, 1,1-Difluorethoyy, Methylthio, Ethylthio, Trifluormethylthio, 1,1-Difluorethylthio, 2,2,2-Trifluorethylthio, Phenyl, Phenoxy, Furyl, Thienyl, Pyrrolyl, Thiazolyl, Pyridyl, Furyloxy, Thienyloxy, Pyrrolyloxy, Thiazolyloxy, Pyridyloxy, Acetyl, Propionyl, Propylcarbonyl, Butylcarbonyl oder 2-Methylpropylcarbonyl,
R¹⁰ für Wasserstoff, Fluor, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy oder tert.-Butoxy steht,
R¹¹ für Wasserstoff, Fluor, Chlor, Brom, Methyl, Ethyl, n-Propyl, Isopropyl, n-Butyl, Isobutyl, sec.-Butyl oder tert.-Butyl steht,
R¹² für Wasserstoff steht,
R¹³ für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl, 3-Indolylmethyl oder 4-Imidazolylmethyl steht, und
R¹⁴ für Methyl, Ethyl, n-Propyl, Isopropyl, n-Butyl, Isobutyl, sec.-Butyl, tert.-Butyl oder Benzyl steht,
und deren Salze,
zur Herstellung eines
Arzneimittels zur Behandlung von Entzündungskrankheiten, immunologischen Erkrankungen, septischem Schock, Transplantatabstoßung, Strahlungsschäden, Reperfusionsverletzungen nach Ischämie, Schlaganfall oder Hirntrauma, neurodegenerativen Erkrankungen, Leberzirrhose, Asthma oder komplexen, chronisch-entzündlichen Erkrankungen wie Arteriosklerose und Multiple Sklerose.

2. Cyclopentabenzofuranderivate der Formel (I), definiert wie in Anspruch 1 und deren Salze, mit Ausnahme von 6,8-Dimethoxy-3a-(methoxyphenyl)-3-phenyl-1,2,3,3a-tetrahydro-8bH-cyclopenta[b][1]benzofuran-1,8b-diol und 8b-Hydroxy-6,8-dimethoxy-3a-(4-methoxyphenyl)-3-phenyl-23,3a,8btetrahydro-1H-cyclopenta[b][1]benzofuran-1-on.

3. Verfahren zur Herstellung von cis-Dihydrocyclopentabenzofurandiolen der Formel (I-a) 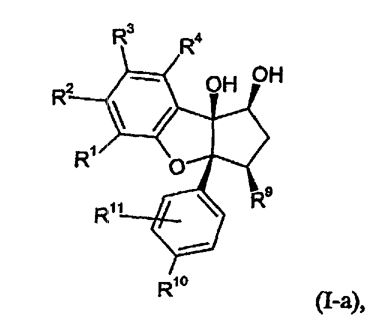 in welcher
R¹, R², R³, R⁴, R⁹, R¹⁰ und R¹¹ die in Anspruch 1 angegebenen Bedeutungen haben, mit der Ausnahme von 6,8-Dimethoxy-3a-(4-methoxyphenyl)-3-phenyl-1,2,3,3a-tetrahydro-8bH-cyclopenta[b][1]benzofuran-1,8b-diol,
**dadurch gekennzeichnet, daß** man Ketoaldehyde der Formel (II-a) 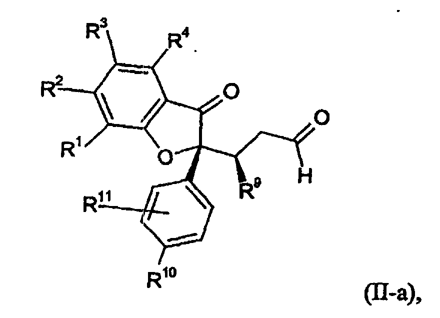 in welcher
R¹, R², R³, R⁴, R⁹, R¹⁰ und R¹¹ die in Anspruch 1 angegebenen Bedeutungen haben,
reduktiv cyclisiert.

4. Verfahren zur Herstellung von Tetrahydrocyclopentabenzofuranonen der Formel (I-b) 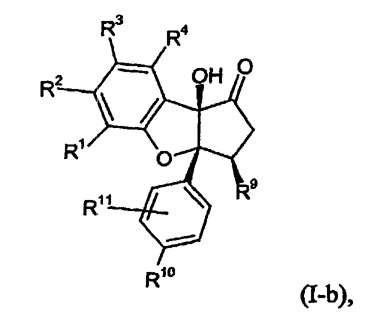 in welcher
R¹, R², R³, R⁴, R⁹, R¹⁰ und R¹¹ die in Anspruch 1 angegebenen Bedeutungen haben, mit der Ausnahme von 8b-Hydroxy-6,8-dimethoxy-3a-(4-methoxyphenyl)-3-phenyl-2,3,3a,8b-tetrahydro-1H-cyclopenta[b][1]benzofuran-1-on,
**dadurch gekennzeichnet, daß** man Cyclopentabenzofurandiole der Formel (I-a) 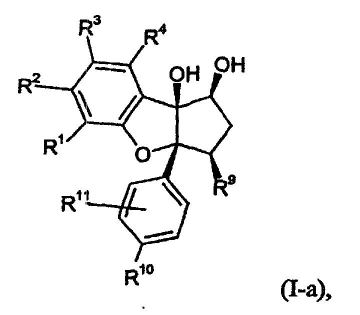 in welcher
R¹, R², R³, R⁴, R⁹, R¹⁰ und R¹¹ die in Anspruch 1 angegebenen Bedeutungen haben, mit der Ausnahme von 6,8-Dimethoxy-3a-(4-methoxyphenyl)-3-phenyl-1,2,3,3a-tetrahydro-8bH-cyclopenta[b][1]benzofuran-1,8bdiol, oxidiert.

5. Verfahren zur Herstellung von trans-Dihydrocyclopentabenzofurandiole der Formel (I-c) 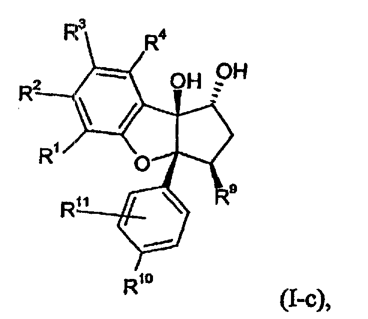 in welcher
R¹, R², R³, R⁴, R⁹, R¹⁰ und R¹¹ die in Anspruch 1 angegebenen Bedeutungen haben, mit der Ausnahme von 6,8-Dimethoxy-3a-(4-methoxyphenyl)-3-phenyl-1,2,3,3a-tetrahydro-8bH-cyclopenta[b][1]benzofuran-1,8bdiol,
**dadurch gekennzeichnet, daß** man Tetrahydrocyclopentabenzofuranone der Formel (I-b) 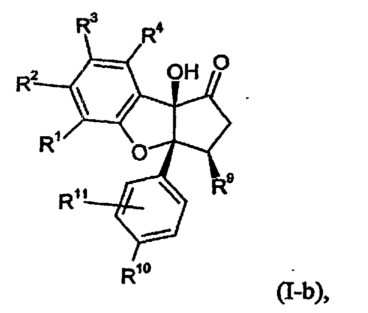 in welcher
R¹, R², R³, R⁴, R⁹, R¹⁰ und R¹¹ die in Anspruch 1 angegebenen Bedeutungen haben, mit der Ausnahme von 8b-Hydroxy-6,8-dimethoxy-3a-(4-methoxyphenyl)-3-phenyl-2,3,3a,8b-tetrahydro-1Hcyclopenta[b][1]benzofuran-1-on,
mit Alkali- oder Tetraalkylammonium-acyloxyboranaten reduziert.

6. Verfahren zur Herstellung von Cyclopentabenzofuran-Derivaten der Formel (I-d) 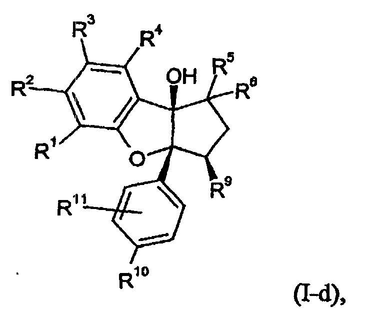 in welcher
R¹ bis R¹¹ die in Anspruch 1 angegebenen Bedeutungen haben, mit der Einschränkung, daß mindestens einer der Reste R¹, R³ und R¹¹ für Halogen oder Alkyl steht,
**dadurch gekennzeichnet, daß** man diesen Rest oder diese Reste durch elektrophile aromatische Substitution von Verbindungen der oben angegebenen Formel (I), in welchen der oder die zu substituierenden Reste für Wasserstoff steht, einführt.

7. Verfahren zur Herstellung von Cyclopentabenzofuran-Derivaten der Formel (I-e) 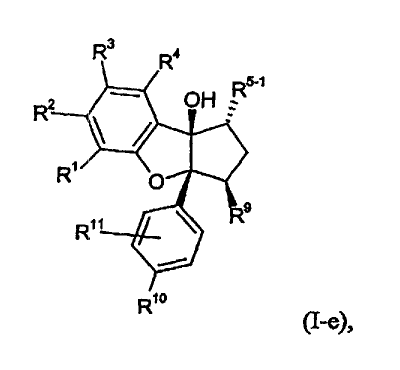 in welcher
R¹ bis R⁴ und R⁹ bis R¹¹ die in Anspruch 1 angegebenen Bedeutungen haben und
R⁵⁻¹ für Alkylamino oder für den Rest -NR¹²-CHR¹³-COOR¹⁴ steht, worin
R¹², R¹³ und R¹⁴ die in Anspruch 1 angegebenen Bedeutungen haben,
**dadurch gekennzeichnet, daß** man Tetrahydrocyclopentabenzofuranone der Formel (I-b) 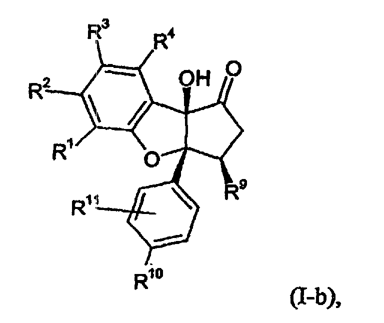 in welcher
R¹ bis R⁴ und R⁹ bis R¹¹ die in Anspruch 1 angegebenen Bedeutungen haben,
mit primären Aminen oder Aminosäurederivaten der Formel (III)
H-R⁵⁻¹ (III),
in welcher
R⁵⁻¹ für Alkylamino oder für den Rest -NR¹²-CHR¹³-COOR¹⁴ steht, worin
R¹², R¹³ und R¹⁴ die Anspruch 1 angegebenen Bedeutungen haben,
in Gegenwart eines Reduktionsmittels umsetzt.

8. Arzneimittel enthaltend ein oder mehrere Cyclopentafuran-Derivate nach Anspruch 2.

## Claims

1. Use of cyclopentabenzofuran derivatives of the formula (I) 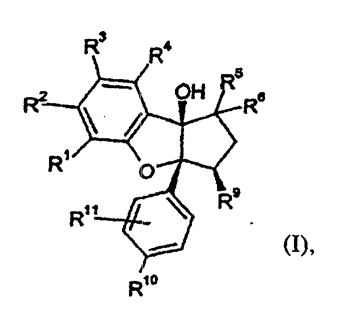 in which
R¹ and R³ in each case represent hydrogen, fluorine, chlorine, bromine, methyl or ethyl,
R² and R⁴ in each case represent methoxy, ethoxy, trifluoromethoxy, difluoromethoxy, chlorodifluoromethoxy, 2-chloro-1,1,2-trifluoroethoxy, 1,1,2-trifluoroethoxy, 1,1,2,2-tetrafluoroethoxy, 2,2,2-trichloro-1,1-difluoromethoxy or 1,1-difluoroethoxy,
R⁵ represents hydroxyl, methylamino, ethylamino, n-propylamino, isopropylamino, n-butylamino, isobutylamino, sec-butylamino, tert-butylamino or the radical -NR¹²-CHR¹³-COOR¹⁴,
R⁶ represents hydrogen, or
R⁵ and R⁶ together represent oxygen (oxo),
R⁹ represents phenyl, methylenedioxyphenyl, furyl, thienyl, pyrrolyl, oxazolyl, isoxazolyl, thiazolyl or pyridyl, which in each case can optionally be monosubstituted or disubstituted by substituents of the group fluorine, chlorine, bromine, iodine, methyl, ethyl, n-propyl, isopropyl, n-butyl, isobutyl, sec-butyl, tert-butyl, trifluoromethyl, difluoromethyl, chlorodifluoromethyl, 2-chloro-1,1,2-trifluoroethyl, 1,1,2-trifluoroethyl, 1,1,2,2-tetrafluoroethyl, 1,1-difluoro-2,2,2-trichloroethyl, 2,2,2-trifluoroethyl, 1,1,2,3,3,3-hexafluoropropyl, 2,2,3,3-tetrafluoropropyl, 2,2,3,3,3-pentafluoropropyl, hydroxyl, methoxy, ethoxy, n-propoxy, isopropoxy, n-butoxy, isobutoxy, sec-butoxy, tert-butoxy, trifluoromethoxy, difluoromethoxy, chlorodifluoromethoxy, 2-chloro-1,1,2-trifluoroethoxy, 1,1,2-trifluoroethoxy, 1,1,2,2-tetrafluoroethoxy, 2,2,2-trichloro-1,1-difluoromethoxy, 1,1-difluoroethoxy, methylthio, ethylthio, trifluoromethylthio, 1,1-difluoroethylthio, 2,2,2-trifluoroethylthio, phenyl, phenoxy, furyl, thienyl, pyrrolyl, thiazolyl, pyridyl, furyloxy, thienyloxy, pyrrolyloxy, thiazolyoxy, pyridyloxy, acetyl, propionyl, propylcarbonyl, butylcarbonyl or 2-methylpropylcarbonyl,
R¹⁰ represents hydrogen, fluorine, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy or tert-butoxy,
R¹¹ represents hydrogen, fluorine, chlorine, bromine, methyl, ethyl, n-propyl, isopropyl, n-butyl, isobutyl, sec-butyl or tert-butyl,
R¹² represents hydrogen,
R¹³ represents hydrogen, methyl, iso-propyl, iso-butyl, sec-butyl, hydroxymethyl, 1-hydroxyethyl, mercaptomethyl, 2-methylthioethyl, 3-aminopropyl, 4-aminobutyl, carboxymethyl, 2-carboxyethyl, carbamoylmethyl, 2-carbamoylethyl, 3-guanidinopropyl, phenyl, benzyl, 4-hydroxybenzyl, 3-indolylmethyl or 4-imidazolylmethyl, and
R¹⁴ represents methyl, ethyl, n-propyl, isopropyl, n-butyl, isobutyl, sec-butyl, tert-butyl or benzyl,
and their salts, for the preparation of a medicament for the treatment of inflammatory diseases, immunological disorders, septic shock, transplant rejection, radiation damage reperfusion injuries after ischemia, stroke or cerebral trauma, neurodegenerative diseases, cirrhosis of the liver, asthma or complex, chronic inflammatory disorders such as arteriosclerosis and multiple sclerosis is prepared.

2. Cyclopentabenzofuran derivatives of the formula (I), defined as in Claim 1 and their salts, with the exception of 6,8-dimethoxy-3a-(methoxyphenyl)-3-phenyl-1,2,3,3a-tetrahydro-8bH-cyclopenta[b][1]benzofuran-1,8b-diol and 8b-hydroxy-6,8-dimethoxy-3a-(4-methoxyphenyl)-3-phenyl-(2,3,3a,8b-tetrahydro-1H-cyclopenta[b][1]benzofuran-1-one.

3. Process for the preparation of cis-dihydrocyclopentabenzofurandiols of the formula (I-a) 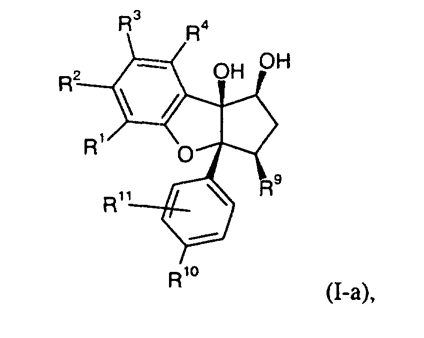 in which
R¹, R², R³, R⁴, R⁹, R¹⁰ and R¹¹ have the meanings indicated in Claim 1, with the exception of 6,8-dimethoxy-3a-(4-methoxyphenyl)-3-phenyl-1,2,3,3a-tetrahydro-8bH-cyclopenta[b][1]benzofuran-1,8b-diol,
**characterized in that** ketoaldehydes of the formula (II-a) 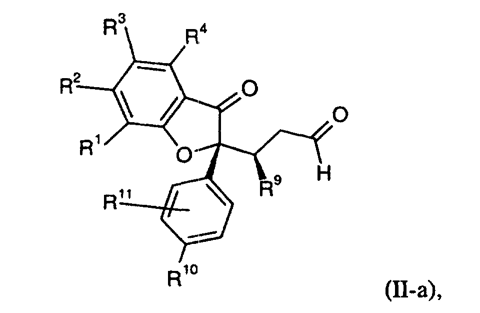 in which
R¹, R², R³, R⁴, R⁹, R¹⁰ and R¹¹ have the meanings indicated in Claim1,
are subjected to reductive cyclization.

4. Process for the preparation of tetrahydrocyclopentabenzofuranones of the formula (I-b) 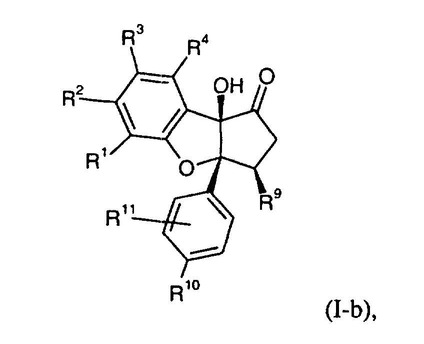 in which
R¹, R², R³, R⁴, R⁹, R¹⁰ and R¹¹ have the meanings indicated in Claim 1, with the exception of 8b-hydroxy-6,8-dimethoxy-3a-(4-methoxyphenyl)-3-phenyl-2,3,3a,8b-tetrahydro-1H-cyclopenta[b][1]benzofuran-1-one,
**characterized in that** dihydrocyclopentabenzofurandiols of the formula (I-a) 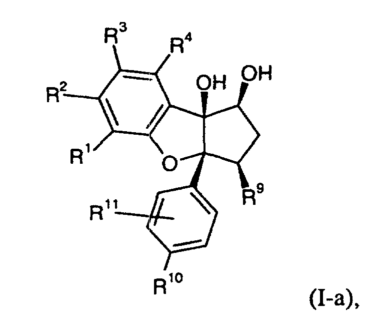 in which
R¹, R², R³, R⁴, R⁹, R¹⁰ and R¹¹ have the meanings indicated in Claim 1, with the exception of 6,8-dimethoxy-3a-(4-methoxyphenyl)-3-phenyl-1,2,3,3a-tetrahydro-8bH-cyclopenta[b] [1]benzofuran-1,8b-diol, are oxidized.

5. Process for the preparation of trans-dihydrocyclopentabenzofurandiols of the formula (I-c) 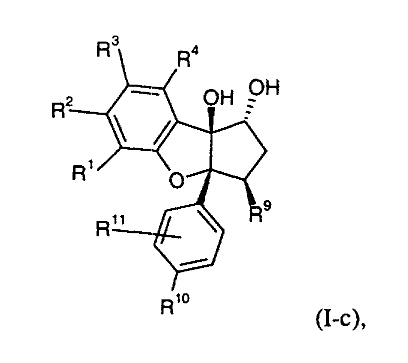 in which
R¹, R², R³, R⁴, R⁹, R¹⁰ and R¹¹ have the meanings indicated in Claim 1, with the exception of 6,8-dimethoxy-3a-(4-methoxyphenyl)-3-phenyl-1,2,3,3a-tetrahydro-8bH-cyclopenta[b][1]benzofuran-1,8b-diol,
**characterized in that** tetrahydrocyclopentabenzofuranones of the formula (I-b) 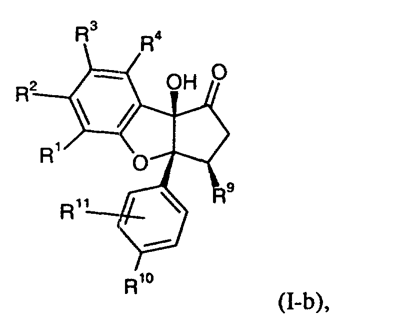 in which
R¹, R², R³, R⁴, R⁹, R¹⁰ and R¹¹ have the meanings indicated in Claim 1, with the exception of 8b-hydroxy-6,8-dimethoxy-3a-(4-methoxyphenyl)-3-phenyl-,2,3,3a,8b-tetrahydro-1H-cyclopenta[b][1]benzofuran-1-one, are reduced with alkali metal or tetraalkylammonium acyloxyborohydrides.

6. Process for the preparation of cyclopentabenzofuran derivatives of the formula (I-d) 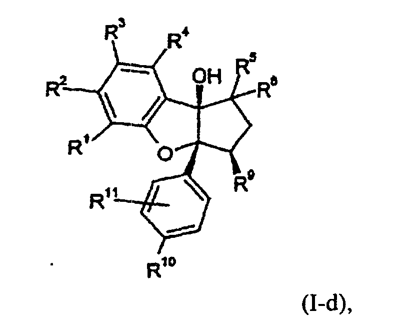 in which
R¹ to R¹¹ have the meanings indicated above, with the restriction that at least one of the radicals R¹, R³ and R¹¹ represents halogen or alkyl,
**characterized in that** this radical or these radicals are introduced by electrophilic aromatic substitution of compounds of the formula (I) indicated above in which the radical or radicals to be substituted represent(s) hydrogen.

7. Process for the preparation of cyclopentabenzofuran derivatives of the formula (I-e) 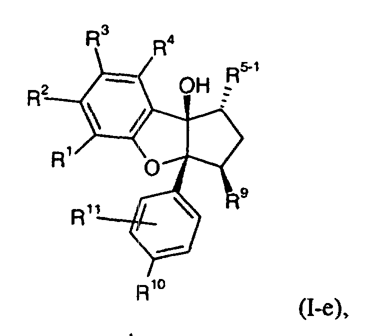 in which
R¹ to R⁴ and R⁹ to R¹¹ have the meanings indicated in Claim 1 and
R⁵⁻¹ represents alkylamino or the radical -NR¹²-CHR¹³-COOR¹⁴, in which
R¹², R¹³ and R¹⁴ have the meanings indicated in Claim 1,
**characterized in that** tetrahydrocyclopentabenzofuranones of the formula (I-b) 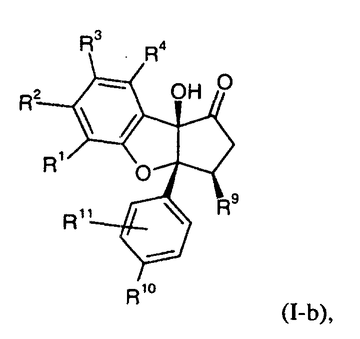 in which
R¹ to R⁴ and R⁹ to R¹¹ have the meanings indicated in Claim 1,
are reacted with primary amines or amino acid derivatives of the formula (III)
H-R⁵⁻¹ (III),
in which
R⁵⁻¹ represents alkylamino or the radical -NR¹²-CHR¹³-COOR¹⁴, in which
R¹², R¹³ and R¹⁴ have the meanings indicated in Claim 1,
in the presence of a reducing agent.

8. Pharmaceuticals comprising one or more cyclopentabenzofuran derivatives according to Claim 2.

## Revendications

1. Utilisation de dérivés de cyclopentabenzofuranne de formule (I) 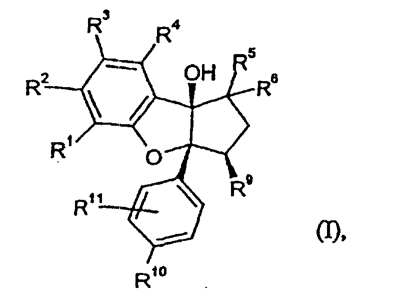 dans laquelle
R¹ et R³ représentent chacun l'hydrogène, le fluor, le chlore, le brome, un reste méthyle ou éthyle,
R² et R⁴ représentent chacun un reste méthoxy, éthoxy, trifluorométhoxy, difluorométhoxy, chlorodifluorométhoxy, 2-chlore-1,1,2-trifluoréthoxy, 1,1,2-trifluoréthoxy, 1,1,2,2-tétrafluoréthoxy, 2,2,2-trichloro-1,1-difluorométhoxy ou 1,1-difluoréthoxy,
R⁵ est un reste hydroxy, méthylamino, éthylamino, n-propylamino, isopropylamino, n-butylamino, isobutylamino, sec.-butylamino, tertio-butylamino ou le reste -NR¹²-CHR¹³-COOR¹⁴,
R⁶ représente l'hydrogène, ou bien
R⁵ et R⁶ représentent ensemble l'oxygène (groupe oxo),
R⁹ est un reste phényle, méthylènedioxyphényle, furyle, thiényle, pyrrolyle, oxazolyle, isoxazolyle, thiazolyle ou pyridyle, chacun de ces restes pouvant porter éventuellement un ou deux substituants appartenant au groupe des subtituants fluoro, chloro, bromo, iodo, méthyle, éthyle, n-propyle, isopropyle, n-butyle, isobutyle, sec.-butyle, tertio-butyle, trifluorométhyle, difluorométhyle, chlorodifluorométhyle, 2-chloro-1,1,2-trifluoréthyle, 1,1,2-trifluoréthyle, 1,1,2,2-tétrafluoréthyle, 1,1-difluoro-2,2,2-trichloréthyle, 2,2,2-trifluoréthyle, 1,1,2,3,3,3-hexafluoropropyle, 2,2,3,3-tétrafluoropropyle, 2,2,3,3,3-pentafluoropropyle, hydroxy, méthoxy, éthoxy, n-propoxy, isopropoxy, n-butoxy, isobutoxy, sec.-butoxy, tertio-butoxy, trifluorométhoxy, difluorométhoxy, chlorodifluorométhoxy, 2-chlore-1,1,2-trifluoréthoxy, 1,1,2-trifluoréthoxy, 1,1,2,2-tétrafluoréthoxy, 2,2,2-trichloro-1,1-difluorométhoxy, 1,1-difluoréthoxy, méthylthio, éthylthio, trifluorométhylthio, 1,1-difluoréthylthio, 2,2,2-triflucréthylthio, phényle, phénoxy, furyle, thiényle, pyrrolyle, thiazolyle, pyridyle, furyloxy, thiényloxy, pyrrolyloxy, thiazolyloxy, pyridyloxy, acétyle, propionyle, propylcarbonyle, butylcarbonyle ou 2-méthylpropylcarbonyle,
R¹⁰ représente l'hydrogène, le fluor, un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy ou tertio-butoxy,
R¹¹ représente l'hydrogène, le fluor, le chlore, le brome, un reste méthyle, éthyle, n-propyle, isopropyle, n-butyle, isobutyle, sec.-butyle ou tertio-butyle,
R¹² représente l'hydrogène,
R¹³ représente l'hydrogène, un reste méthyle, isopropyle, isobutyle, sec.-butyle, hydroxymétyle, 1-hydroxyéthyle, mercaptométhyle, 2-méthylthioéthyle, 3-aminopropyle, 4-aminobutyle, carboxyméthyle, 2-carboxyéthyle, carbamoylméthyle, 2-carbamoyléthyle, 3-guanidinopropyle, phényle, benzyle, 4-hydroxybenzyle, 3-indolylméthyle ou 4-imidazolylméthyle, et
R¹⁴ est un reste méthyle, éthyle, n-propyle, isopropyle, n-butyle, isobutyle, sec.-butyle, tertio-butyle ou benzyle,
et de leurs sels, pour la préparation d'un médicament destiné au traitement de maladies inflammatoires, de maladies immunologiques, du choc septique, du rejet de greffes, de lésions d'irradiation, de lésions de reperfusions après une ischémie, d'apoplexie cérébrale ou de traumatisme cérébral, de maladies neurodégénératives, de la cirrhose du foie, de l'asthme ou de maladies inflammatoires chroniques complexes tels que l'artériosclérose et la sclérose en plaques.

2. Dérivés de cyclopentabenzofuranne de formule (I), tels que définis dans la revendication 1, et leurs sels, à l'exception du 6,8-diméthoxy-3a-(méthoxyphényl)-3-phényl-1,2,3,3a-tétrahydro-8bH-cyclpenta[b][1]benzofuranne-1,8b-diol et de la 8b-hydroxy-6,8-diméthoxy-3a-(4-méthoxyphényl)-3-phényl-2,3,3a,8b-tétrahydro-1H-cyclopenta[b][1]-benzofuranne-1-one.

3. Procédé de production de cis-dihydrocyclopentabenzofuranne-diols de formule (I-a) 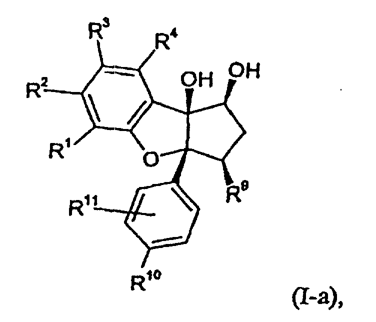 dans laquelle
R¹, R², R³, R⁴, R⁹, R¹⁰ et R¹¹ ont les définitions indiquées dans la revendication 1, à l'exception du 6,8-diméthoxy-3a-(4-méthoxyphényl)-3-phényl-1,2,3,3a-tétrahydro-8bH-cyclopenta[b] [1]benzofuranne-1,8b-diol,
**caractérisé en ce qu'**on cyclise par voie de réduction des céto-aldéhydes de formule (II-a) 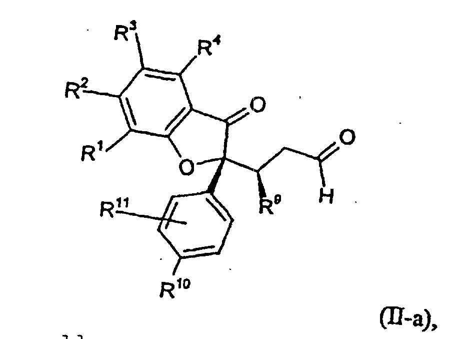 dans laquelle
R¹, R², R³, R⁴, R⁹, R¹⁰ et R¹¹ ont les définitions indiquées dans la revendication 1.

4. Procédé de production de tétrahydrocyclopentabenzofurannones de formule (I-b) 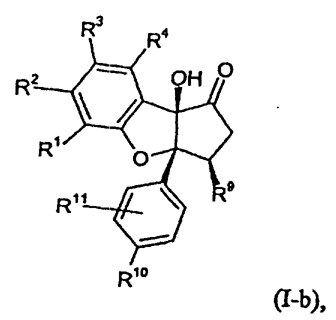 dans laquelle
R¹, R², R³, R⁴, R⁹, R¹⁰ et R¹¹ ont les définitions indiquées dans la revendication 1, à l'exception de la 8b-hydroxy-6,8-diméthoxy-3a-(4-méthoxyphényl)-3-phényl-2,3,3a,8b-tétrahydro-lH-cyclopenta[b][1]benzofuranne-1-one,
**caractérisé en ce qu'**on oxyde des cyclopentabenzofuranne-diols de formule (I-a) 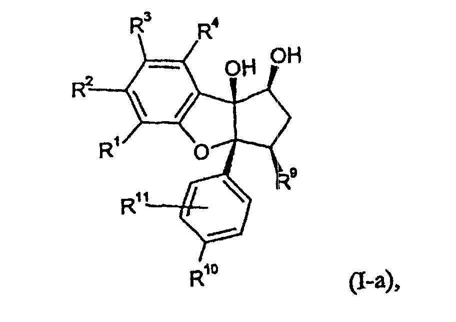 dans laquelle
R¹, R², R³, R⁴, R⁹, R¹⁰ et R¹¹ ont les définitions indiquées dans la revendication 1, à l'exception du 6,8-diméthoxy-3a-(4-méthoxyphényl)-3-phényl-1,2,3,3a-tétrahydro-8bH-cyclopenta[b][1]benzofuranne-1,8b-diol.

5. Procédé de production de trans-dihydrocyclopentabenzofuranne-diols de formule (I-c) 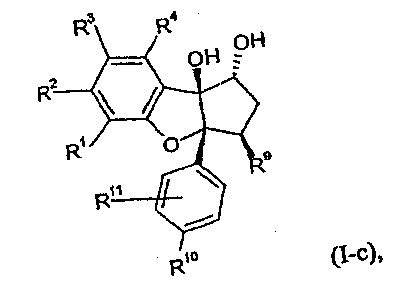 dans laquelle
R¹, R², R³, R⁴, R⁹, R¹⁰ et R¹¹ ont les définitions indiquées dans la revendication 1, à l'exception du 6,8-diméthoxy-3a-(4-méthoxyphényl)-3-phényl-1,2,3,3a-tétrahydro-8bH-cyclopenta[b][1]benzofuranne-1,8b-diol,
**caractérisé en ce qu'**on réduit avec des acyloxyboranates de métaux alcalins ou de tétra-alkylammonium des tétrahydrocyclopentabenzofurannones de formule (I-b) 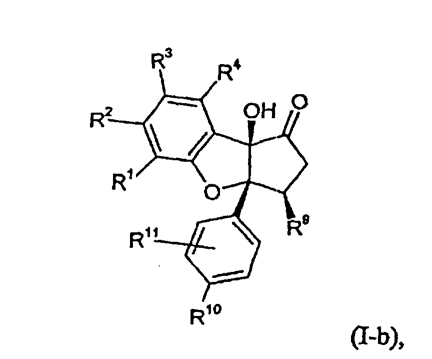 dans laquelle
R¹, R², R³, R⁴, R⁹, R¹⁰ et R¹¹ ont les définitions indiquées dans la revendication 1, à l'exception de la 8b-hydroxy-6,8-diméthoxy-3a-(4-méthoxyphényl)-3-phényl-2,3,3a,8b-tétrahydro-lH-cyclopenta[b][1]benzofuranne-1-one.

6. Procédé de production de dérivés de cyclopentabenzo-furanne de formule (I-d) 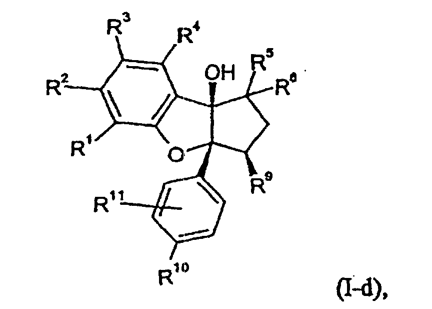 dans laquelle
R¹ à R¹¹ ont les définitions indiquées dans la revendication 1, sous réserve qu'au moins l'un des restes R¹, R³ et R¹¹ représente un halogène ou un reste alkyle,
**caractérisé en ce qu'**on introduit ce reste ou ces restes par substitution aromatique électrophile de composés de formule (I) indiquée ci-dessus, dans lesquels le ou les restes à substituer représentent l'hydrogène.

7. Procédé de production de dérivés de cyclopentabenzofuranne de formule (I-e) 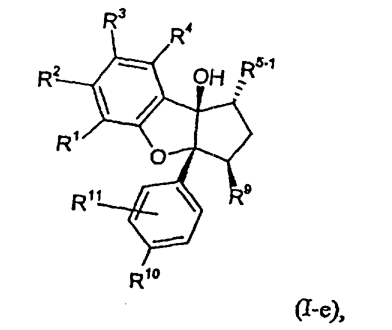 dans laquelle
R¹ à R⁴ et R⁹ à R¹¹ ont les définitions indiquées dans la revendication 1
et
R⁵⁻¹ est un reste alkylamino ou le reste -NR¹²-CHR¹³-COOR¹⁴, où
R¹², R¹³ et R¹⁴ ont les définitions indiquées dans la revendication 1,
**caractérisé en ce qu'**on fait réagir des tétrahydrocyclopentabenzofurannones de formule (I-b) 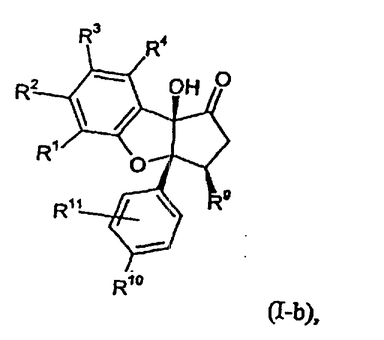 dans laquelle
R¹ à R⁴ et R⁹ à R¹¹ ont les définitions indiquées dans la revendication 1,
avec des aminés primaires ou des dérivés d'amino-acides de formule (III)
H-R⁵⁻¹ (III)
dans laquelle
R⁵⁻¹ est un reste alkylamino ou le reste -NR¹²-CHR¹³-COOR¹⁴, dans lequel
R¹², R¹³ et R¹⁴ ont les définitions indiquées dans la revendication 1,
en présence d'un agent réducteur.

8. Médicament contenant un ou plusieurs dérivés de cyclopentabenzofuranne suivant la revendication 2.
